# EUROPÄISCHE PATENTSCHRIFT

(11) **EP 0 835 659 B1**
(45) Veröffentlichungstag und Bekanntmachung des Hinweises auf die Patenterteilung: **15.12.2004**
(21) Anmeldenummer: 97117677.1
(22) Anmeldetag: 13.10.1997
(51) Int. Cl.: A61K 31/47, A61K 31/425, A61P 25/28

(54) **Verwendung von nicht-peptidischen Bradykinin-Antagonisten zur Herstellung von Arzneimitteln zur Behandlung und Prävention der Alzheimerischen Krankheit**
Use of non-peptidic bradykinin antagonists in the preparation of medicaments for the treatment and prevention of Alzheimer's disease
Utilisation d'antagonistes non peptidiques de la bradykinine dans la préparation de médicaments pour le traitement et la prévention de la maladie d'Alzheimer

(30) Priorität: 14.10.1996 DE 19642290; 08.07.1997 DE 19729140
(43) Veröffentlichungstag der Anmeldung: 15.04.1998
(73) Patentinhaber: Aventis Pharma Deutschland GmbH, 65929 Frankfurt am Main (DE)
(72) Erfinder: Heitsch, Holger, Dr., 55252 Mainz-Kastel (DE); Wirth, Klaus, Dr., 65830 Kriftel (DE); Wiemer, Gabriele, Prof. Dr., 61476 Kronberg (DE)

(56) Entgegenhaltungen:
- EP-A- 0 622 361
- WO-A-97/32585
- US-A- 5 385 915
- GRIESBACHER ET AL.: "FR173657, a new, potent and selective nonpeptide bradykinin antagonist : in vivo studies" NAUNYN-SCHMIEDEBERG'S ARCH. PHARMACOL., Bd. 354, Nr. 4sup1, September 1996, Seite r6 XP002052578
- RACCHI ET AL.: "Bradykinin induced amyloid precursor protein secretion in fibroblasts from Alzheimer's disease, Down's syndrome and control donors" SOC. NEUROSCI. ABST., Bd. 22, Nr. 1-3, November 1996, Seite 1944 XP002052579
- ASANO ET AL.: "The identification of an orally active, nonpeptide bradykinin B2 receptor antagonist, FR173657 " BR. J. PHARMACOL., Bd. 120, Nr. 4, Februar 1997, Seiten 617-624, XP002052580
- NITSCH ET AL.: "Regulation of APP processing for the therapeutical reduction of brain amyloid burden" ANN. NEW YORK ACAD. SCI., THE NEUROBIOLOGY OF ALZHEIMER'S DISEASE, Bd. 777, 1996, Seiten 175-182, XP002052581
- HUANG ET AL.: "Increased inositol 1,4,5-triphosphate accumulation correlates with an up-regulation of bradykinin receptors in Alzheimer's disease" J. NEUROCHEM., Bd. 64, Nr. 2, 1995, Seiten 761-766, XP002052582
- PETERSON ET AL.: "Altered response of fibroblasts from aged and Alzheimer donors to drugs that elevate cytosolic free calcium" NEUROBIOL. AGING, Bd. 9, Nr. 3, 1988, Seiten 261-266, XP000562449

## Beschreibung

Bradykinin und verwandte Peptide sind potente, Entzündungen und Schmerz erzeugende, vasoaktive, körpereigene Substanzen. Aus EP-A 622 361 US 5,212,182, US 5,216,165, US 5,438,064, WO 96 04251 WO 9613485 sowie den noch nicht offengelegten deutschen Patentanmeldungen P 19610784.9 und P 19620508.5 sind substituierte, anellierte Heterobicyclen und ihre Verwendung als Bradykinin-Rezeptorantagonisten und ihre Verwendung als Mittel zur Bekämpfung von Zuständen, die durch Bradykinin vermittelt, ausgelöst oder unterstützt werden, bekannt.

Überraschenderweise wurde nun gefunden, daß nicht-peptidische Bradykinin-Antagonisten dieses Strukturtyps darüber hinaus geeignete Mittel zur Behandlung und Prävention der Alzheimerschen Krankheit sind. Dies betrifft sowohl die Intention, ein Fortschreiten der Krankheit zu verhindern als auch bereits aufgetretene Symptome zu behandeln. Darüber hinaus sind die o. a. Bradykinin-Antagonisten bei präventiver Gabe auch dazu geeignet, das Entstehen der Alzheimerschen Krankheit zu verhindern, wenn es durch geeignete diagnostische Maßnahmen in Zukunft gelingen sollte, einen späteren Ausbruch der Krankheit vorherzusagen.

Als Verbindungen eignen sich nicht-peptidische Bradykinin-Antagonisten die die Wirkungen des Alzheimer-Proteins Amyloid (β/A4) an isolierten Endothelzellen hemmen.

Geeignete nicht-peptidische Bradykinin-Antagonisten sind unter anderem die Verbindungen der Formel (I) 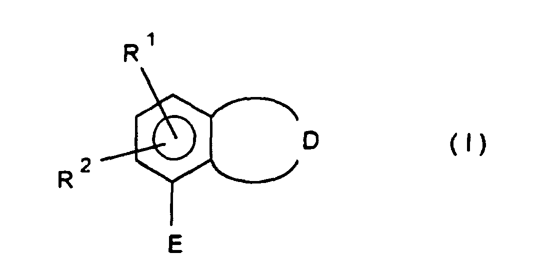 in welcher die Symbole folgende Bedeutung haben:
- D: 1. ein Rest der Formel (II) oder 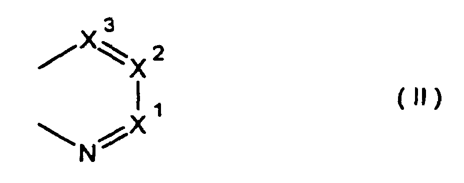
2. ein Rest der Formeln (III) bis (VI): 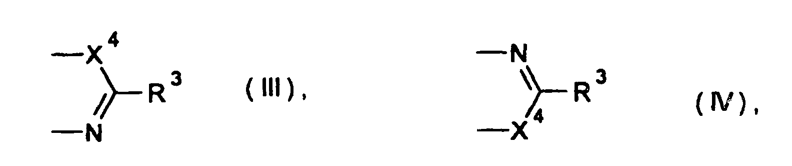 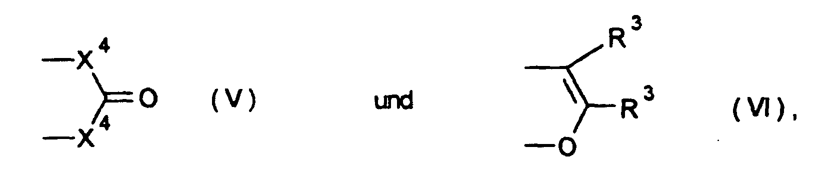
- E: 1. ein Rest der Formel (VII) oder 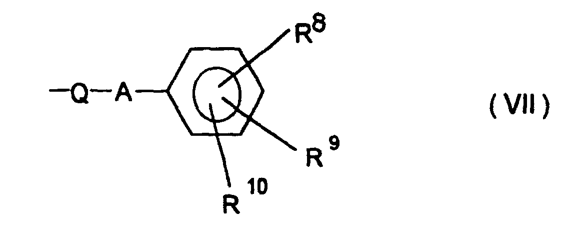
2. Wasserstoff, Halogen, (C₁-C₄)-Alkyl, (C₁-C₄)-Alkylthio oder (C₁-C₄)-Alkoxy, wobei in den letzten 3 Resten 1 oder mehrere Wasserstoffatome durch Fluor ersetzt sein können;
- X¹: Stickstoff oder C-R⁴ ;
- X²: Stickstoff oder C-R⁵ ;
- X³: Stickstoff oder C-R⁶ ;
- X⁴: unabhängig voneinander Sauerstoff, Schwefel, Stickstoff oder N-R⁷ ;
- R¹,R²: sind gleich oder verschieden und stehen für Wasserstoff, Halogen, (C₁-C₄)-Alkyl oder (C₁-C₄)-Alkoxy;
- R³: unabhängig voneinander Wasserstoff, Halogen, (C₁-C₆)-Alkyl, (C₆-C₁₂)-Aryl, (C₁-C₃)-Alkyl-(C₆-C₁₂)-Aryl, (C₃-C₅)-Alkenyl, (C₁-C₄)-Alkoxy oder CO₂R¹¹;
- R⁴: Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, wobei der Alkylrest jedes vorgenannten Substituenten gegebenenfalls substituiert ist mit Hydroxy, (C₁-C₄)-Alkoxy, Amino und (C₁-C₄)-Alkylamino, oder (C₆-C₁₂)-Aryl, gegebenenfalls substituiert mit (C₁-C₄)-Alkyl oder CO₂R¹¹;
- R⁵: 1. Wasserstoff, (C₁-C₄)-Alkyl oder
2. 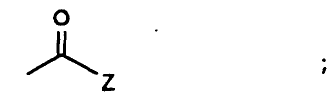
- R⁶: 1. Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, wobei der Alkylrest jedes vorgenannten Substituenten gegebenenfalls substituiert ist mit Hydroxy, (C₁-C₄)-Alkoxy, Amino und (C₁-C₄)-Alkylamino, oder (C₆-C₁₂)-Aryl, gegebenenfalls substituiert mit (C₁-C₄)-Alkyl oder CO₂R¹¹; oder
2. ein Rest der Formel (VIII): 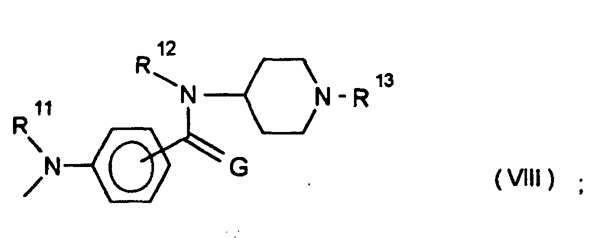
- R⁷: ist gleich oder verschieden (C₁-C₄)-Alkyl, (C₆-C₁₂)-Aryl oder (C₁-C₃)-Alkyl-(C₆-C₁₂)-Aryl;
- R⁸,R⁹: sind gleich oder verschieden und stehen für Wasserstoff, Halogen, (C₁-C₃)-Alkyl und (C₁-C₃)-Alkoxy;
- A: (C₁-C₃)-Alkandiyl;
- Q: O oder NR¹¹;
- R¹⁰: ein Rest der Formel (IX) 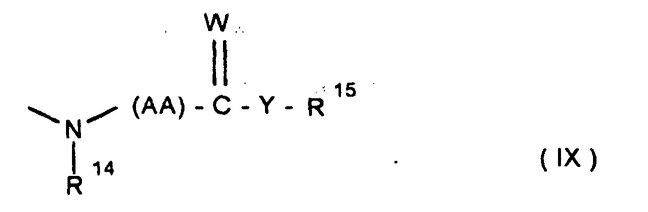
- R¹¹,R¹⁴: unabhängig voneinander Wasserstoff oder (C₁-C₄)-Alkyl;
- W: O oder S;
- G: O oder zwei Wasserstoffatome
- R¹²: steht bei G=O für Wasserstoff und bei G = zwei Wasserstoffatome für Wasserstoff oder R¹⁶CO;
- R¹³: ist (C₁-C₄)-Alkyl, (C₃-C₇)-Cycloalkyl, -(CH₂)ₘ-(C₃-C₇)-Cycloalkyl, -(CH₂)ₘ-CON(R¹¹)₂, oder 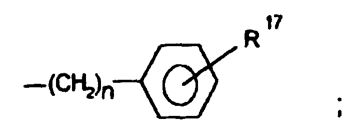
- m, n: sind gleich oder verschieden eine Zahl 0-6
- AA: steht für eine Aminosäure wie Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, o-Methyltyrosin, β-(2-Thienyl)-alanin, Glycin, Cyclohexylalanin, Leucin, Isoleucin, Valin, Norleucin, Phenylglycin, Serin, Cystein, Aminopropionsäure oder Aminobuttersäure;
- Y: 1. (C₂-C₅)-Alkendiyl,
2. (C₁-C₈)-Alkandiyl,
3. (C₃-C₁₀)-Cycloalkandiyl oder
4. -(CH)ₚ-Tₒ-(CH₂)_{q}-,
wobei 1. bis 4. gegebenenfalls mit einen oder mehreren gleichen oder verschiedenen Resten wie O-R¹⁸, NO₂, CN, CO₂R¹¹, SO₃R¹⁸, NR²⁰R²¹, SO₂NR²⁰R²¹, CONR²⁰R²¹ substituiert sein kann;
- T: O, NR²¹ oder S;
- o: ist eine Zahl 0 oder 1;
- p,q: sind gleich oder verschieden und bedeuten eine Zahl von 0 bis 6;
- R¹⁵: 1. Wasserstoff,
2. (C₁-C₅)-Alkyl,
3. (C₆-C₁₀)-Aryl oder
4. (C₁-C₉)-Heteroaryl,
wobei 3. und 4. gegebenenfalls mit einer oder mehreren gleichen oder verschiedenen Gruppen substituiert sein können, wie Halogen, CN, NO₂, (C₁-C₆)-Alkyl, (C₆-C₁₀)-Aryl, (C₂-C₅)-Alkenyl, (C₁-C₅)-Alkoxy, wobei die letzten vier Reste gegebenenfalls teilweise oder vollständig mit Halogen substituiert sein können, (C₁-C₅)-Alkylthio, NR²⁰R²¹, CO₂R¹⁹, SO₃R¹⁸, SO₂NR²⁰R²¹, SO₂R¹⁸, O-R¹⁸, NR²⁰CO-R¹⁵;
- R¹⁶: ist unabhängig voneinander Wasserstoff, (C₁-C₄)-Alkyl, (C₆-C₁₂)-Aryl, (C₁-C₄)-Alkyl-(C₆-C₁₂)-Aryl oder Perfluoro-(C₁-C₄)-Alkyl;
- R¹⁷: ist Wasserstoff, Halogen, (C₁-C₄)-Alkyl, (C₁-C₄)-Alkoxy, Perfluoro-(C₁-C₄)-Alkyl, NO₂, OH, NH₂, CON(R¹⁶)₂ oder NR¹⁶CON(R¹⁶)₂;
- R¹⁸,R¹⁹,R²⁰: gleich oder verschieden sind und Wasserstoff, (C₁-C₅)-Alkyl, (C₃-C₅)-Alkenyl, (C₆-C₁₂)-Aryl-(C₁-C₃)-Alkyl, (C₃-C₁₀)-Cycloalkyl, (C₃-C₁₀)-Cycloalkyl-(C₁-C₃)-Alkyl, C(O)-O-(C₁-C₅)-Alkyl oder C(O)-NH-(C₁-C₅)-Alkyl bedeuten;
- R²¹: unabhängig voneinander Wasserstoff, C(O)-O-(C₁-C₅)-Alkyl oder C(O)-O-(C₁-C₃)-Alkyl-(C₆-C₁₀)-Aryl;
- Z: -N(R¹⁴)(R²²);
- R²²: steht für 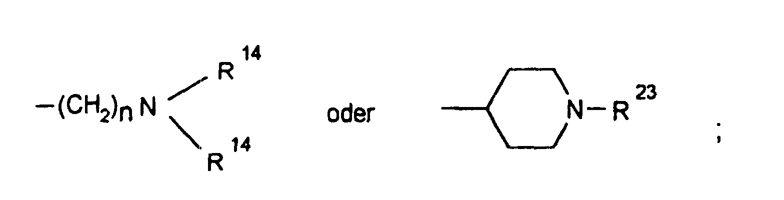
- R²³: (C₁-C₄)-Alkyl, 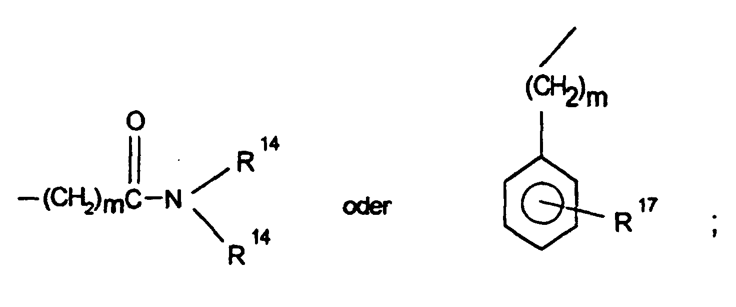
sowie deren physiologisch verträglichen Salze.

Alkyl und Alkenyl können geradkettig oder verzweigt sein. Entsprechendes gilt für davon abgeleitete Reste wie Alkoxy.

Alkenyl steht für einfach oder mehrfach ungesättigte Reste wie 1,4-Butadienyl, 8,11-Heptadienyl, 8,11,14-Heptatrienyl und Butenyl. Entsprechendes gilt für Cycloalkenyl.
Cycloalkyl steht für mono- oder bicyclische Reste wie Cyclopropyl, Cyclopentyl, Cyclohexyl, Bicyclononyl. Entsprechendes gilt für Cycloalkenyl.

(C₆-C₁₂)-Aryl ist beispielsweise Phenyl, Naphthyl oder Biphenylyl, vorzugsweise Phenyl. Entsprechendes gilt auch für davon abgeleitete Reste wie Aralkyl.

Halogen (Hal) steht für Fluor, Chlor, Brom oder Jod, vorzugsweise für Chlor oder Fluor.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl, Benzopyranonyl, Coumarinyl, Pyranonyl, Furandionyl.

Unter physiologisch verträglichen Salzen von Verbindungen der Formel (I) versteht man sowohl deren organische als auch anorganische Salze, wie sie in Remington*'*s Pharmaceutical Sciences (A.R. Gennard Editor, Mack Publishing Co., Easton PA, 17. Auflage, Seite 1418 (1985)) beschrieben sind. Aufgrund der physiologischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie Essigsäure, Zitronensäure, Benzoesäure, Maleinsäure, Fumarsäure, Weinsäure und p-Toluolsulfonsäure bevorzugt.

Der selektive nicht-peptidische Bradykinin-Antagonist FR 173657, entsprechend Beispiel 3, ist bekannt (s. Griesbacher et al.; "FR173657, A New, Potent and Selective Nonpeptide Bradykinin Antagonists: In Vivo Studies"; Naunyn-Schmiedeberg's Archives of Pharmacology, Springer, Berlin, DE; Vol. 354; Nr. 4 Suppl. 1; Seite R06).

Geeignete nicht-peptidische Bradykinin-Antagonisten und deren Synthese sind zum Beispiel beschrieben in den Patentanmeldungen EP-A 622 361, US 5,212,182, US 5,216,165, US 5,438,064, WO 9604251, WO 9613485 sowie in den noch nicht offengelegten deutschen Patentanmeldungen P 19610784.9 und P 19620508.5.

In der noch nicht offengelegten deutschen Patentanmeldung P 19 610 784.9 werden heterocyclische Fluoralkylderivate und Fluoralkoxyderivate der Formel (IA) 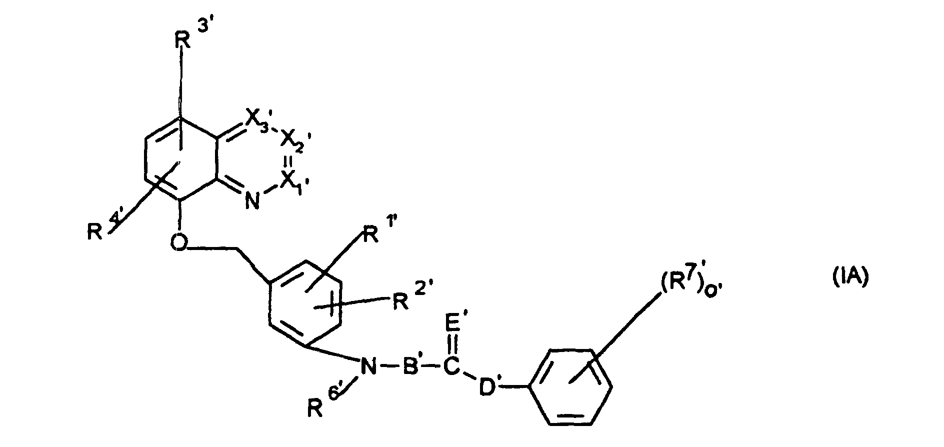 beschrieben, in welcher die Symbole folgende Bedeutung haben:
a)
   - X₁'-X₃': sind gleich oder verschieden N oder CR⁵;
b)
   - R^{1'} und R^{2'}: sind gleich oder verschieden
   1. H
   2. Halogen;
c)
   - R^{3'} und R^{4'}: gleich oder verschieden
   1. H
   2. Halogen
   3. (C₁-C₅)-Alkyl
   4. (C₂-C₅)-Alkenyl:
d)
   - R^{5'}: 1. H
   2. Halogen
   3. (C₁-C₆)-Alkyl
   4. O-R^{6'}
   5. S-R^{6'}
   6. NHR^{6'}
   7. (C₆-C₁₂)-Aryl
   8. (C₆-C₁₂)-Aryl-(C₁-C₃)-Alkyl
   9. -C(O)-OR^{6'}
   10. -C(O)-H;
   wobei 3., 7., 8. gegebenenfalls mit einer oder mehreren Gruppen wie z. B. OR^{6'}, SR^{6'}, NO₂, CN, NHR^{6'}, Halogen substituiert sein können;
e)
   - R^{6'} und R^{8'}: gleich oder verschieden
   1. H
   2. (C₁-C₅)-Alkyl
   3. (C₃-C₅)-Alkenyl
   4. (C₆-C₁₂)-Aryl-(C₁-C₃)-Alkyl;
f)
   - R^{7'}: 1. (C₁-C₅)-Alkyl, wobei Wasserstoff teilweise oder vollständig durch Fluor oder Chlor ersetzt ist
   2. (C₁-C₅)-Alkoxy, wobei Wasserstoff teilweise oder vollständig durch Fluor oder Chlor ersetzt ist;
g)
   - B': eine Aminocarbonsäure, z. B. Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, O-Methyltyrosin, β-(2-Thienyl)alanin, Glycin, Cyclohexylalanin, Leucin, Isoleucin, Valin, Norleucin oder Phenylglycin, Serin oder Cystein, Aminopropionsäure, Aminobuttersäure;
h)
   - D': 1. (C₂-C₅)-Alkendiyl
   2. (C₁-C₅)-Alkandiyl
   3. -(CH₂)_{n'}-Y'_{p'} -(CH₂)_{m'}-;
i)
   - E': 1. O
   2. S;
j)
   - Y': 1. O
   2. S
   3. NR^{8'};
k)
   - n*'* und m': gleich oder verschieden eine Zahl 0 - 3;
I)
   - o': eine Zahl 1 - 3;
m)
   - p': eine Zahl 0 oder 1 bedeuten
sowie deren physiologisch verträglichen Salze.

Die Verbindungen der Formel (IA) werden durch ein Verfahren hergestellt, das dadurch gekennzeichnet ist, daß man
a) eine Verbindung der Formel (XII), 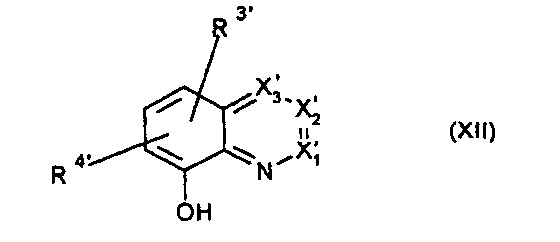 worin X'₁-X'₃ und R^{3'} und R^{4'} wie oben in Formel (IA) definiert sind, mit Cs₂CO₃ oder K₂CO₃ in einem inerten Lösungsmittel, bevorzugt DMF oder N-Methylpyrrolidin, deprotoniert und bei Raumtemperatur mit einer Verbindung der Formel (XIII) 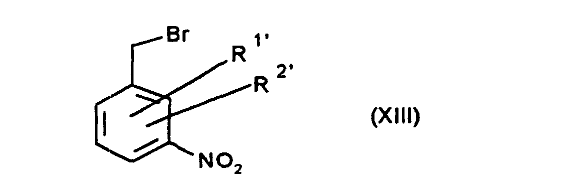 worin R^{1'} und R^{2'} wie oben in Formel (IA) definiert sind, umsetzt;
b) die so erhaltene Verbindung der Formel (XIV) 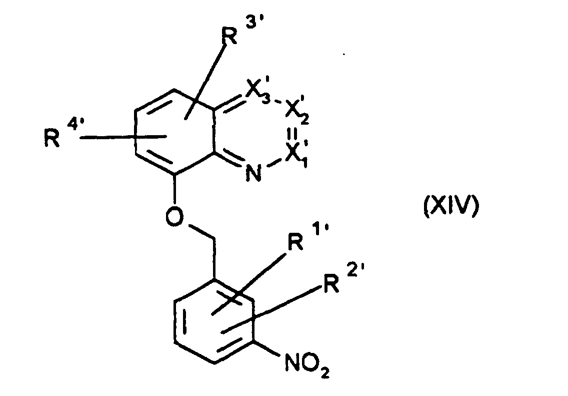 worin R^{1'}, R^{2'}, R^{3'}, R^{4'}, X'₁, X'₂ und X'₃ wie oben in Formel (IA) definiert sind, mit Hilfe von Übergangsmetallhalogeniden, bevorzugt SnCl₂, FeCl₃ zu einer Verbindung der Formel (XV) reduziert, 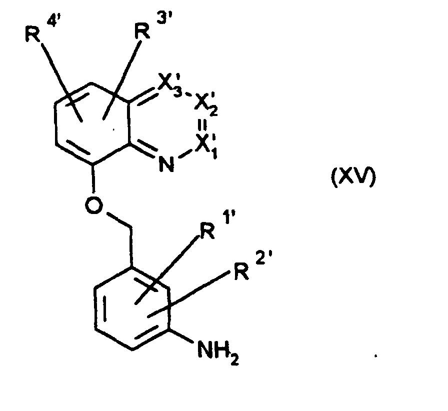 worin R^{1'}, R^{2'}, R^{3'}, R^{4'}, X'₁, X'₂ und X'₃ wie oben in Formel (IA) definiert sind;
c) eine Verbindung der Formel (XV) mit aktivierten, geeignet geschützten Aminocarbonsäurederivaten von B*'* (B*'*-Prot*'*) , bevorzugt den Säurechloriden der Phthaloyl-geschützten Aminocarbonsäurederivate von B*'*, in inerten Lösungsmitteln wie z. B. NMP, gegebenenfalls durch Zugabe von DMAP, umsetzt und so eine Verbindung der Formel (XVI) erhält, 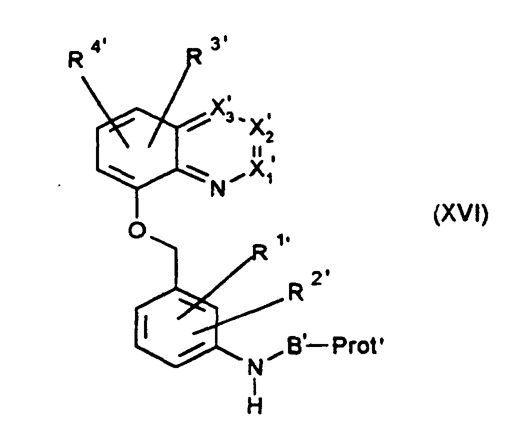 worin B', R^{1'}, R^{2'}, R^{3'}, R^{4'}, X'₁, X'₂ und X'₃ wie oben in Formel (IA) definiert sind, und
   Prot*'* für eine Aminoschutzgruppe steht, wie in T.W. Greene "Protective Groups in organic Synthesis", Verlag John Wiley, 2. Auflage 1991 beschrieben, z.B. Phthaloyl, Benzyl oder Paramethoxybenzyl;
d) eine Verbindung der Formel (XVI) nach erfolgter Einwirkung von Alkalihydriden, Alkalicarbonaten oder Alkoholaten in inerten Lösungsmitteln, bevorzugt DMF oder NMP, gefolgt von einer Behandlung mit R^{6'}X, wobei R^{6'} wie oben in Formel (IA) definiert ist und X eine Abgangsgruppe, z. B. Halogen, Mesylat oder Tosylat, bedeutet, umsetzt, wobei man eine Verbindung der Formel (XVII) erhält, 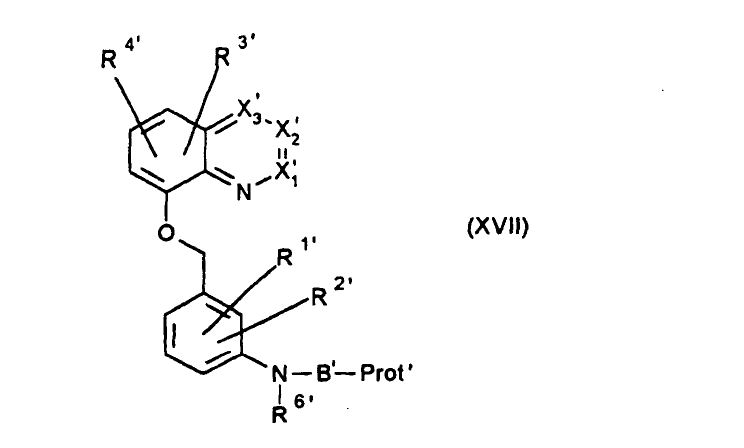 worin B', R^{1'}, R^{2'}, R^{3'}, R^{4'}, R^{6'}, X'₁, X'₂ und X'₃ wie oben in Formel (IA) und Prot wie oben in Formel (XVI) definiert sind;
e) zum Entfernen der Schutzgruppe (Prot) von der Verbindung der Formel (XVII), im Falle der Phthaloylgruppe bevorzugt mit Hydrazin in Alkoholen als Lösungsmittel bei Temperaturen zwischen Raumtemperatur und dem Siedepunkt, bevorzugt bei Raumtemperatur, umsetzt wobei man eine Verbindung der Formel (XVIII) erhält, 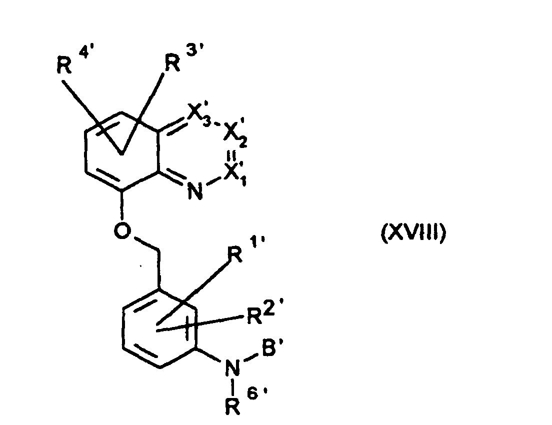 worin B', R^{1'}, R^{2'}, R^{3'}, R^{4'}, R^{6'}, X'₁, X'₂ und X'₃ wie oben in Formel (IA) und Prot wie oben in Formel (XVI) definiert sind;
f₁) eine Verbindung der Formel (XVIII) mit aktivierten Carbonsäurederivaten der Formel (XIX), 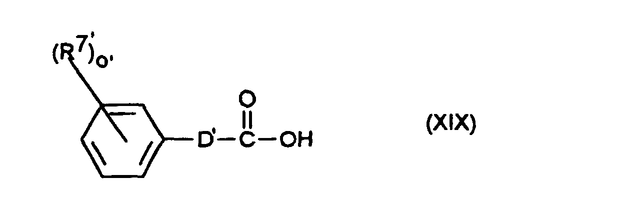 worin R^{7'}, o*'* und D*'* wie oben in Formel (IA) definiert sind, bevorzugt deren Säurechloriden oder Carbonsäuren der Formel (XIX), aktiviert durch Reagenzien, wie sie in der Peptidsynthese verwendet werden, umsetzt, oder
f₂) eine Verbindung der Formel (XVIII) mit einem Amin oder einem Alkohol der Formel (XX) 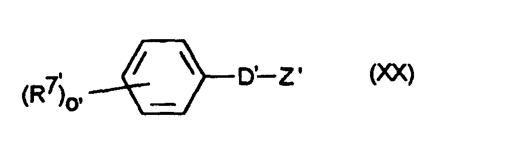 worin R^{7'}, o' und D' wie oben in Formel (IA) definiert sind und Z' OH oder NH₂ bedeutet, wobei man zunächst jedoch die Verbindung der Formel (XVIII) oder (XX) mit einer doppelt aktivierten Carbonylverbindung zur Bildung der Harnstoff- bzw. Urethangruppe, z.B. mit Carbodiimiden, Phosgen oder Chlorkohlensäureestern, bevorzugt Phosgen und Carbonyldiimidazol, reagieren läßt, umsetzt, bevorzugt bei Temperaturen zwischen 0°C und Raumtemperatur in inerten Lösungsmitteln, bevorzugt Dichlormethan oder Dimethoxyethan, oder
f₃) eine Verbindung der Formel (XVIII) mit einem entsprechenden Isocyanat oder Isothiocyanat, bevorzugt bei Temperaturen zwischen 0°C und Raumtemperatur in inerten Lösungsmitteln, bevorzugt Dichlormethan oder Dimethoxyethan, umsetzt, und
g) die erhaltene Verbindung der Formel (IA) gegebenenfalls nach bekannten Methoden in ihre physiologisch verträglichen Salze überführt.

Die Konversion zur Bromethylverbindung erfolgt durch Umsetzung des entsprechenden Methyl-Derivates mit N-Bromsuccinimid, Dibromhydantoin oder Brom in inerten Lösungsmitteln, bevorzugt Brombenzol oder Cyclohexan bei Temperaturen von 60 °C bis zum Siedepunkt.

Als Kupplungsreagenz können alle möglichen in der Peptidsynthese verwendeten Aktivierungsreagenzien, siehe z. B. Houben-Weyl, Methoden der organischen Chemie, Band 15/2, Georg Thieme Verlag, Stuttgart 1974, verwendet werden, insbesondere aber Carbodiimide wie z. B. N,N'-Dicyclohexylcarbodiimid, N,N'-Diisopropyl-carbodiimid oder N-Ethyl-N'-(3-dimethylaminopropyl)carbodiimid. Die Kupplung kann dabei direkt durch Addition von Carbonsäurederivat mit dem Aktivierungsreagenz und gegebenenfalls einem Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R.Geiger, Chem.Ber. 103, 2054 (1970)) durchgeführt werden oder aber die Voraktivierung des Carbonsäurederivats als symmetrisches Anhydrid oder HOBt- bzw. HOObt-Ester kann separat erfolgen und die Lösung der aktivierten Spezies in einem geeigneten Lösungsmittel zum Amin gegeben werden.

Die Kupplung bzw. Aktivierung der Aminosäurederivate mit einem der oben genannten Aktivierungsreagenzien kann in Dimethylformamid, N-Methylpyrrolidon oder Methylenchlorid oder einer Mischung aus den genannten Lösungsmitteln durchgeführt werden.

Anstelle der Phthaloylgruppe können auch Schutzgruppen verwendet werden, die beide Protonen der Aminogruppe schützen, z.B. 2 Benzyl-Gruppen.

In der noch nicht offengelegten Patentanmeldung P 19620508.5 werden schwefelenthaltende heterocyclische Verbindungen der Formel (IB) beschrieben 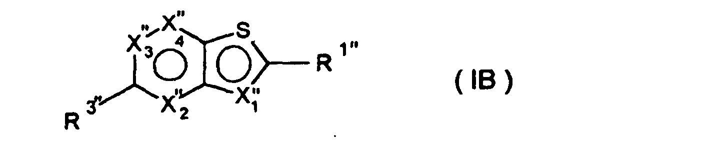 worin die Symbole folgende Bedeutungen haben:
a) einer der Reste X"₁, X"₂ oder X"₃
   steht für C-O-R^{2"} und die jeweils anderen X"₁, X"₂, X"₃ und X"₄ sind dann gleich oder verschieden
   1. N
   2. CR^{1"};
b) R^{1"} und R^{3"} gleich oder verschieden
   1. H
   2. Halogen
   3. (C₁-C₆)-Alkyl
   4. O-R^{6"}
   5. S-R^{6"}
   6. NHR^{6"}
   7. (C₆-C₁₂)-Aryl
   8. (C₆-C₁₂)-Aryl-(C₁-C₃)-Alkyl
   9. C(O)-OR^{6"}
   10. C(O)-H
   11. (C₂-C₅)-Alkenyl
   12. NO₂
   13. SO₃R^{7"}
   14. CN
   15. C(O)-NHR^{8"}
   wobei 3., 7., 8., 11. gegebenenfalls mit einer oder mehreren Gruppen wie C(O)-(O)_{o"}-(C₁-C₅)-Alkyl, OR^{6"}, SR^{7"}, NO₂, CN, NHR^{8"}, Halogen substituiert sein können;
c) R^{2"} eine Verbindung der Formel (XXII) 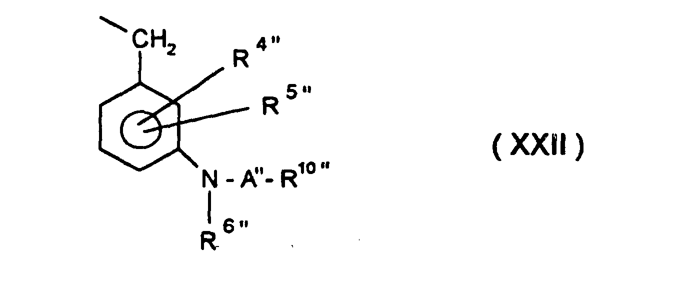 bedeutet;
d) R^{4"} und R^{5"} gleich oder verschieden
   1. H
   2. Halogen
   3. OR^{6"}
   4. SR^{6"}
   5. CN
   6. (C₁-C₅)-Alkyl bedeuten;
e) R^{6"}, R^{7"} und R^{8"} gleich oder verschieden
   1. H
   2. (C₁-C₅)-Alkyl
   3. (C₃-C₅)-Alkenyl
   4. (C₆-C₁₂)-Aryl-(C₁-C₃)-Alkyl;
   5. (C₃-C₁₀)-Cycloalkyl,
   6. (C₃-C₁₀)-Cycloalkyl-(C₁-C₃)-Alkyl;
   7. C(O)-(O)_{o"}-(C₁-C₅)-Alkyl,
   8. C(O)-(NH)_{o"}-(C₁-C₅)-Alkyl;
f) A" eine Aminocarbonsäure, z.B. Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, O-Methyltyrosin, β-(2-Thienyl)alanin, Glycin, Cyclohexylalanin, Leucin, Isoleucin, Valin, Norleucin, Phenylglycin, Serin, Cystein, Aminopropionsäure oder Aminobuttersäure;
g) R^{9"}
   1. H
   2. C(O)-(O)ₒ-(C₁-C₅)-Alkyl
   3. C(O)-(O)ₒ-(C₁-C₃)-Alkyl-(C₆-C₁₀)Aryl;
h) R^{10"}
   1. -C(O)-D"-E"
   2. -C(S)-D"-E"
   3. -SO₂-D"-E"
   4. Wasserstoff;
i) D"
   1. (C₂-C₅)-Alkendiyl
   2. (C₁-C₈)-Alkandiyl
   3. -(CH₂)_{n"}-Y"_{o"}-(CH₂)_{m"}-
   4. (C₃-C₁₀)-Cycloalkandiyl
   5. (C₃-C₁₀)-Cycloalkyl-(C₁-C₃)-alkandiyl
   6. (C₃-C₁₀)-Cycloalkendiyl
   7. (C₃-C₁₀)-Cycloalkenyl-(C₁-C₃)-alkandiyl
   wobei 1.-7. gegebenenfalls mit einer oder mehreren Gruppen wie z.B. OR⁶, NO₂, CN, CO₂R^{7"}, NR^{8"}R^{9"}, SO₂R^{6"}, SO₂NR^{8"}R^{9"}, SO₃R^{7"} oder C(O)-NR^{8"}R^{9"} substituiert sein kann;
j) E"
   1. H
   2. (C₆-C₁₀)-Aryl,
   3. (C₁-C₉)-Heteroaryl,
   wobei 2. und 3. gegebenenfalls mit einer oder mehreren Gruppen substituiert sein können wie z.B. NR^{8"}R^{9"}, CN, CO₂R^{6"}, SO₃R^{7"}, NO₂, SO₂NR^{8"}R^{9"}, SO₂R^{6"}, O-(C₁-C₅)-Alkyl, S-(C₁-C₅)-Alkyl, (C₁-C₅)-Alkyl, (C₂-C₅)-Alkenyl, wobei O-(C₁-C₅)-Alkyl und (C₁-C₅)-Alkyl gegebenenfalls teilweise oder vollständig mit Halogen substituiert sein können;
k) Y"
   1. O
   2. S
   3. NR^{8"};
I) n" und m" gleich oder verschieden eine Zahl 0-6;
m) o" gleich oder verschieden eine Zahl 0 oder 1;
sowie deren physiologisch verträglichen Salze.

Die Verbindungen der Formel (IB) werden durch ein Verfahren hergestellt, das dadurch gekennzeichnet ist, daß man
a₁)
   1. eine Verbindung der Formel (XXIII) 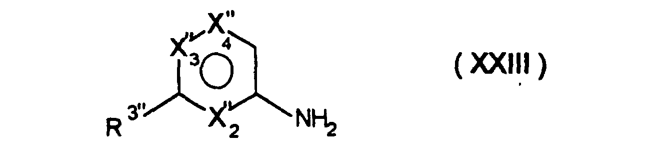 worin R^{3"}, X"₂, X"₃ und X"₄ wie oben in Formel IB definiert sind, zuerst mit aktivierten Carbonsäurederivaten, bevorzugt deren Säurechloriden unter Verwendung einer Hilfsbase, bevorzugt Triethylamin oder Diisopropylethylamin, bei Temperaturen zwischen 0-20°C acyliert,
   2. die so erhaltene Verbindung der Formel (XXIV) 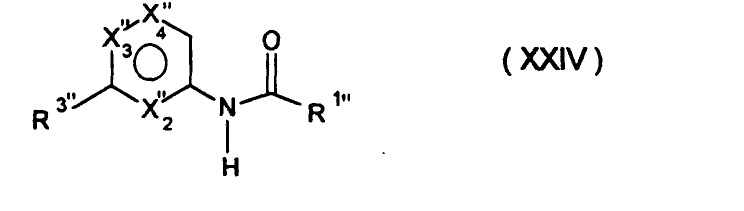 mit Laweson*'*s Reagenz oder bevorzugt P₂S₁₀ in Butylacetat oder anderen inerten hochsiedenden Lösungsmitteln zum Sieden erhitzt und so eine Verbindung der Formel (XXV) erhält, 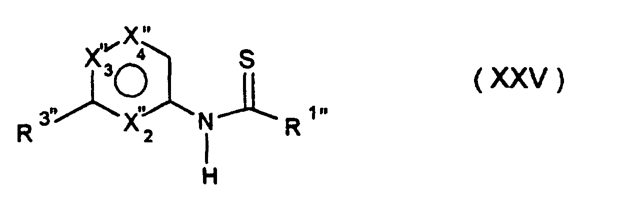 worin R^{1"}, R^{3''}, X"₂, X"₃ und X"₄ in Formeln (XXIV) und (XXV) wie oben in Formel IB definiert sind, mit der Maßgabe, daß falls X"₂ oder X"₃ = C-O-R^{2"} ist, dann steht R^{2"} für H oder (C₁-C₅)-Alkyl, bevorzugt für Methyl oder Ethyl,
   3. die so erhaltene Verbindung (XXV) durch radikalische Cyclisierung mit radikalerzeugenden Reagenzien, bevorzugt K₃Fe(CN)₆ oder Br₂ in inerten Lösungsmitteln, bevorzugt H₂O, bei Temperaturen zwischen 80-110°C umsetzt und dabei eine Verbindung der Formel (XXVI) erhält 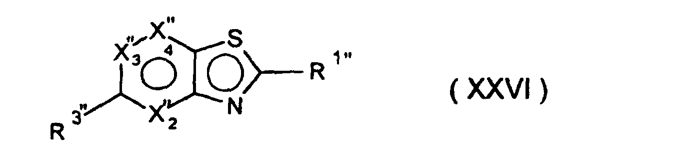 worin die Reste R^{1"}, R^{3"}, X"₂, X"₃ und X"₄ wie oben in Formel IB definiert sind, mit der Maßgabe, daß falls X"₂ oder X"₃ = C-O-R^{2''} ist, dann steht R^{2''} H oder (C₁-C₅)-Alkyl, bevorzugt für Methyl oder Ethyl,
   4. eine Verbindung der Formel (XXVI), worin X"₂ oder X"₃ C-O-R^{2''} bedeutet und wie unter 3. definiert ist, durch etherspaltende Reagenzien, bevorzugt BBr₃, HJ/roter Phosphor, HBr, HBr/CH₃CO₂H in inerten Lösungsmitteln oder ohne Lösungsmittel bei Temperaturen zwischen 0°C und dem Siedepunkt, zu Verbindungen der Formel (XXVI) umsetzt, worin X^{2"} oder X^{3"} COR^{2"} bedeutet und R^{2"} = Wasserstoff ist;
   oder
a₂)
   1. eine Verbindung der Formel (XXVII) 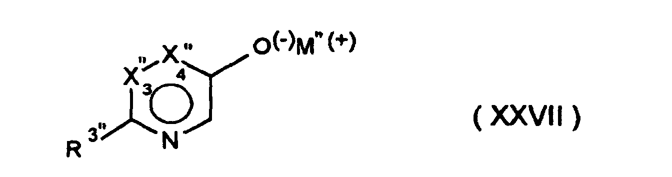 worin R^{3''}, X₃, X₄ wie oben in Formel IB definiert sind und M" für Kalium, Natrium oder Cäsium steht,
      durch sukzessive Behandlung mit CO₂ und anschließend NH₃ unter erhöhtem Druck und Temperaturen, bevorzugt 100 atm und 200°C, in eine Verbindung der Formel (XXVIII) umwandelt, 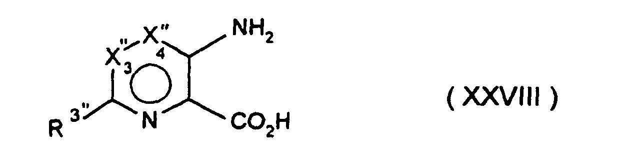 worin R^{3"}, X"₃, X"₄ wie oben in Formel IB definiert sind,
   2. eine Verbindung der Formel (XXVIII) durch Diazotierung und anschließender Behandlung mit HS-CHR^{1"}-CO₂H in eine Verbindung der Formel (XXIX) umwandelt, 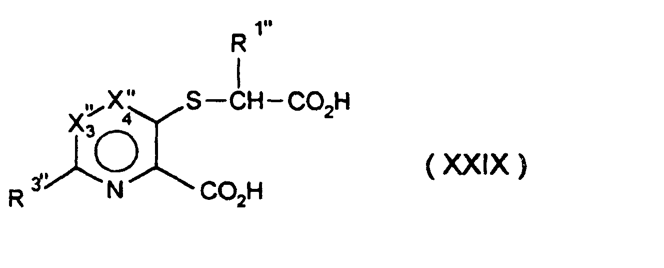 worin R^{1"}, R^{3"}, X"₃, X"₄ wie oben in Formel IB definiert sind,
   3. eine Verbindung der Formel (XXIX) durch Cyclisierung unter gleichzeitiger Decarboxylierung und Wasserabspaltung in inerten Lösungsmitteln, bevorzugt H₂O, oder ohne Lösungsmittel, bevorzugt bei Temperaturen ∼ 100°C in eine Verbindung der Formel (XXX) überführt, 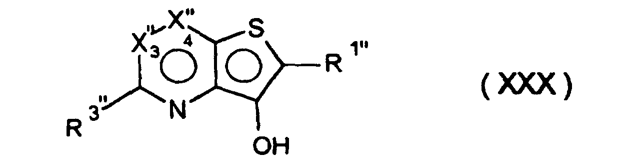 worin R^{1"}, R^{3"}, X"₃, X"₄ wie oben in Formel (IB) definiert sind;
b) eine Verbindung der Formeln (XXVI) oder (XXX) 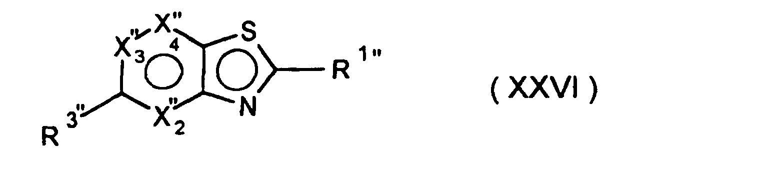 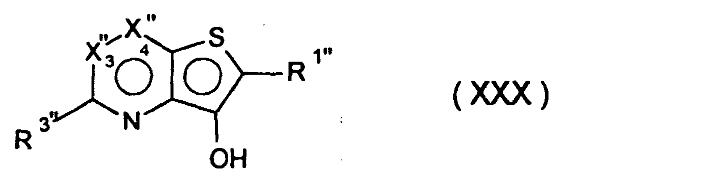 worin X"₂, X"₃, X"₄, R^{1"} und R^{3"} wie oben in Formel (IB) definiert sind, im Falle von Verbindungen der Formel (XXVI) X"₂ oder X"₃ = C-O-H bedeutet, mit Cs₂CO₃ oder K₂CO₃ in einem inerten Lösungsmittel, bevorzugt DMF oder N-Methylpyrrolidin, deprotoniert und bei Raumtemperatur mit einer Verbindung der Formel (XXXI) 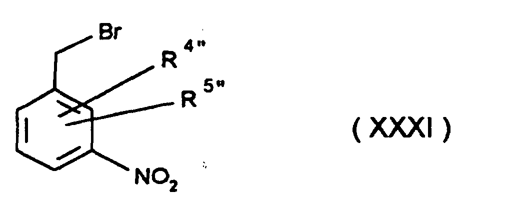 worin R^{4''} und R^{5''} wie oben in Formel (XXII) definiert sind, umsetzt;
c) die so erhaltenen Verbindungen der Formeln (XXXII) oder (XXXII*'*) 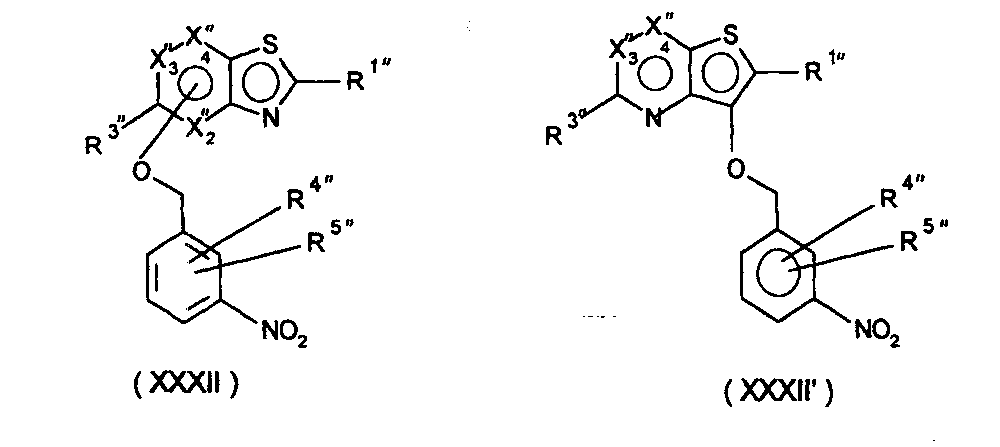 worin R^{1"}, R^{3"}, R^{4"}, R^{5"}, X"₂, X"₃, X"₄ wie oben in Formel (IB) und (XXII) definiert sind, mit Hilfe von Übergangsmetallhalogeniden, bevorzugt SnCl₂, FeCl₃ zu einer Verbindung der Formeln (XXXIII) oder (XXXIII*'*) reduziert, 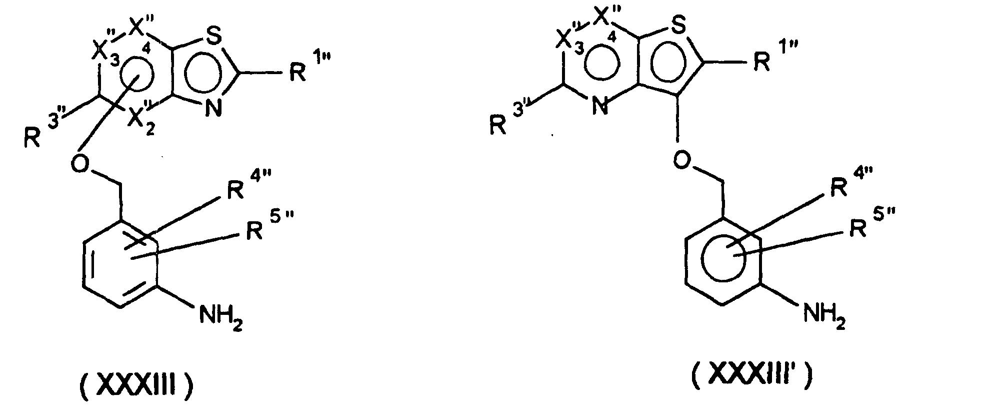 worin R^{1"}, R^{3"}, R^{4"}, R^{5"}, X"₂, X"₃ und X"₄ wie oben in Formeln (IB) und (XXII) definiert sind;
d) eine Verbindung der Formeln (XXXIII) oder (XXXIII*'*) mit aktivierten, geeignet geschützten Aminocarbonsäurederivaten von A" (A"-Prot"), bevorzugt den Säurechloriden der Phthaloyl-geschützten Aminocarbonsäurederivate von A", in inerten Lösungsmitteln wie z.B. NMP, gegebenenfalls durch Zugabe von DMAP, umsetzt und so eine Verbindung der Formeln (XXXIV) oder (XXXIV*'*) erhält, 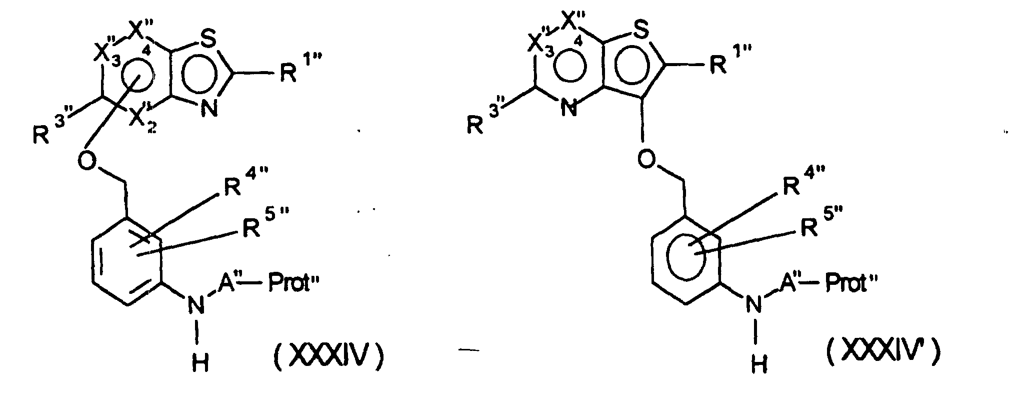 worin A", R^{1"}, R^{3"}, R^{4"}, R^{5"}, X"₂, X"₃ und X"₄ wie oben in Formeln (IB) und (XXII) definiert sind, und
   Prot" für eine Aminoschutzgruppe steht, wie in T.W. Greene "Protective Groups in Organic Synthesis", Verlag John Wiley, 2. Auflage 1991 beschrieben, wobei beide Protonen der Aminoschutzgruppe geschützt sind, z.B. Benzyl, Paramethoxybenzyl oder Phthaloyl;
e) eine Verbindung der Formeln (XXXIV) oder (XXXIV*'*) nach erfolgter Einwirkung von Alkalihydriden, Alkalicarbonaten oder Alkoholaten in inerten Lösungsmitteln, bevorzugt DMF oder NMP, gefolgt von einer Behandlung mit R^{6"}X", wobei R^{6"} wie oben in Formel (IB) definiert ist und X" eine Abgangsgruppe, z. B. Halogen, Mesylat oder Tosylat, bedeutet, umsetzt, wobei man eine Verbindung der Formeln (XXXV) oder (XXXV*'*) erhält, 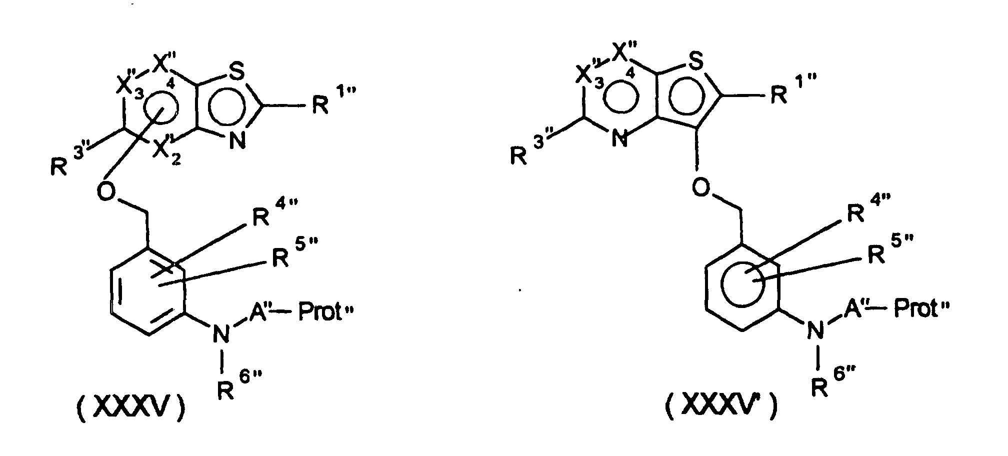 worin A", R^{1"}, R^{3"}, R^{4"}, R^{5"}, R^{6"}, X"₂, X"₃ und X"₄ wie oben in Formel (IB) und (XXII) und Prot" wie oben in Formel (XXXIV) definiert sind;
f) zum Entfernen der Schutzgruppe (Prot) von der Verbindung der Formeln (XXXV) oder (XXXV*'*), im Falle der Phthaloylgruppe bevorzugt mit Hydrazin in Alkoholen als Lösungsmittel, bei Temperaturen zwischen Raumtemperatur und dem Siedepunkt, bevorzugt bei Raumtemperatur, umsetzt, wobei man eine Verbindung der Formeln (XXXVI) oder (XXXVI*'*) erhält, 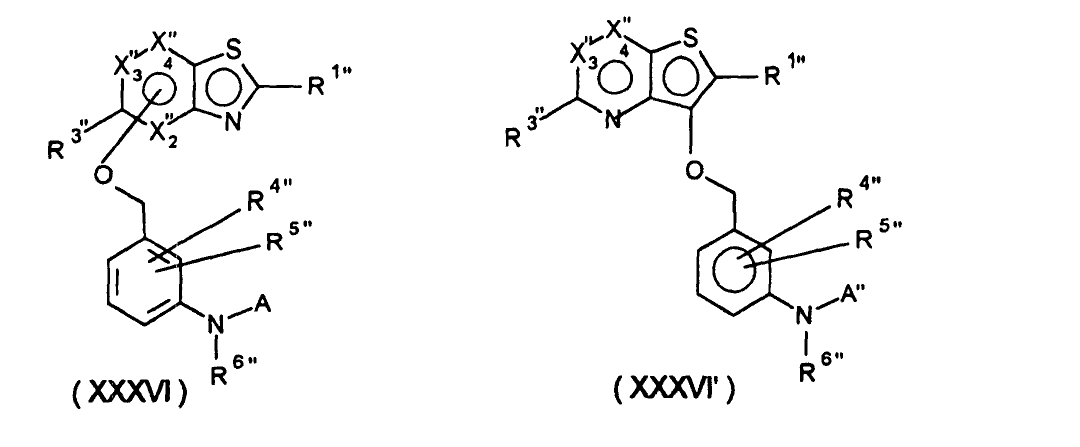 worin A", R^{1"}, R^{3"}, R^{4"}, R^{5"}, R^{6"}, X"₂, X"₃ und X"₄ wie oben in Formeln (IB) und (XXII) und Prot" wie oben in Formel (XXXIV) definiert sind;
g₁) eine Verbindung der Formeln (XXXVI) oder (XXXVI*'*) mit aktivierten Säurederivaten der Formeln (XXXVII), (XXXVIII) oder (XXXIX),

   E" - D" - C(O) - OH (XXXVII)

   E" - D" - C(S) - OH (XXXVIII)

   E" - D" - SO₂ - OH (XXXIX)

   worin D und E wie oben in Formel (XXII) definiert sind, bevorzugt deren Säurechloriden, Anhydriden oder Carbonsäuren der Formeln (XXXVII), (XXXVIII) oder (XXXIX), aktiviert durch Reagenzien, wie sie in der Peptidsynthese verwendet werden, umsetzt, oder
g₂) eine Verbindung der Formeln (XXXVI) oder (XXXVI*'*) mit einem Amin oder einem Alkohol der Formel (XL)

   E"-D"-Z" (XL)

   worin E" und D" wie oben definiert sind und Z" OH oder NH₂ bedeuten, wobei man zunächst jedoch die Verbindungen der Formeln (XXXV), (XXXV*'*) oder (XL) mit einer doppelt aktivierten Carbonylverbindung zur Bildung der Harnstoff- bzw. Urethangruppe, z.B. mit Carbodiimiden, Phosgen oder Chlorkohlensäureestern, bevorzugt Phosgen und Carbonyldiimidazol, reagieren läßt, umsetzt, bevorzugt bei Temperaturen zwischen 0°C und Raumtemperatur in inerten Lösungsmitteln, bevorzugt Dichlormethan oder Dimethoxyethan, oder
g₃) eine Verbindung der Formeln (XXXVI) oder (XXXVI*'*) mit einem entsprechenden Isocyanat oder Isothiocyanat, bevorzugt bei Temperaturen zwischen 0°C und Raumtemperatur in inerten Lösungsmitteln, bevorzugt Dichlormethan oder Dimethoxyethan, umsetzt, und
h) die erhaltene Verbindung der Formel (IB) gegebenenfalls nach bekannten Methoden in ihre physiologisch verträglichen Salze überführt.

Der Austausch von Chlor gegen Alkoxy oder den entsprechenden S-Alkylen erfolgt durch Reaktion mit den entsprechenden Alkoholaten oder Thiolaten, bevorzugt deren Alkali- oder Erdalkalisalzen in inerten Lösungsmitteln bevorzugt DMF, NMP oder dem entsprechenden Alkohol bei Temperaturen zwischen 0°C und 60°C, bevorzugt zwischen 0°C und Raumtemperatur.

Der Ersatz von Chlor gegen Cyano erfolgt durch Einwirken von Cyaniden, bevorzugt den Kupfercyaniden, in inerten hochsiedenden Lösungsmitteln, wie z.B. DMF oder NMP bei deren Siedepunkten.

Für die Konversion zur Bromethylverbindung, die Kupplungsreagenzien und die Kupplung als solche sowie für die Phthaloylgruppe gilt das bei den Verbindungen der Formel la Ausgeführte.

Besonders geeignet sind Verbindungen der Formel I, in welcher bedeuten:
- D: 1. einen Rest der Formel (Xa) 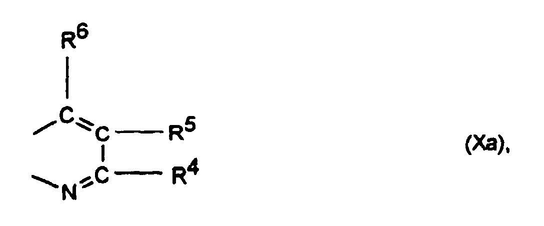
2. einen Rest der Formel (Xb) 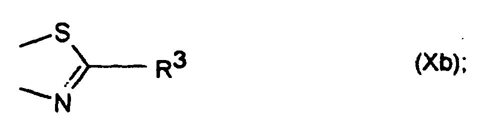
- E: 1. einen Rest der Formel (XI) 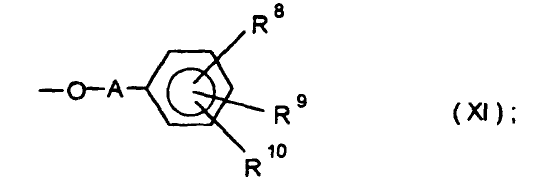
2. Wasserstoff, (C₁-C₄)-Alkyl und (C₁-C₄)-Alkoxy;
- R¹,R²: sind gleich oder verschieden und stehen für Wasserstoff, Halogen und (C₁-C₄)-Alkyl;
- R⁴: ist Wasserstoff, (C₁-C₄)-Alkyl, Phenyl oder Methoxy;
- R⁵: 1. Wasserstoff, (C₁-C₄)-Alkyl oder
2. 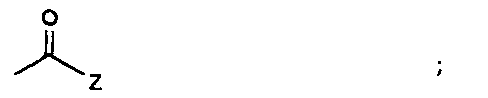
- R⁶: 1. Wasserstoff, (C₁-C₄)-Alkyl oder
2. ein Rest der Formel (VIII): 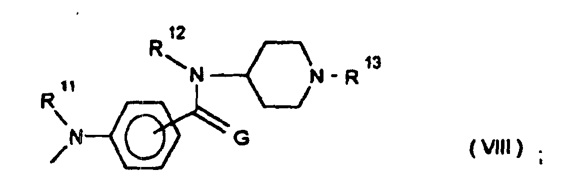
- R⁸,R⁹: sind gleich oder verschieden und stehen für Wasserstoff, Halogen, (C₁-C₃)-Alkyl und (C₁-C₃)-Alkoxy;
- A: ist -CH₂- oder -CH₂-CH₂- ;
- R¹⁰: steht für einen Rest der Formel (IX): 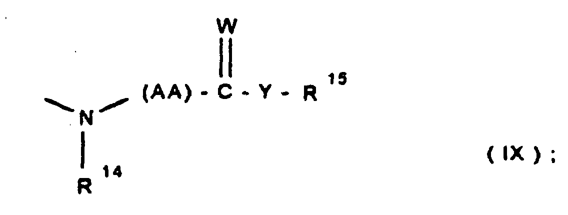
- R¹¹,R¹⁴: sind gleich oder verschieden Wasserstoff, Methyl oder Ethyl;
- W: steht für O oder S;
- G: steht für O oder zwei Wasserstoffatome
- R¹²: steht bei G gleich O für Wasserstoff oder bei G gleich zwei Wasserstoffatome für Wasserstoff oder R¹⁶CO;
- R¹³: ist (C₁-C₄)-Alkyl, Cyclopentyl, Cyclohexyl, -(CH₂)ₘCON(R¹¹)₂, 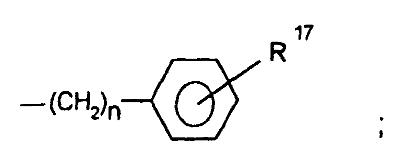
- m,n: sind gleich oder verschieden eine Zahl 0-2;
- AA: steht für die Aminocarbonsäure Glycin oder Alanin;
- Y: 1. (C₂-C₅)-Alkendiyl,
2. (C₂-C₄)-Alkandiyl,
3. (C₃-C₆)-Cycloalkandiyl oder
4. -(CH₂)ₚ-Tₒ-(CH₂)_{q}- ;
- T: steht für 0 oder S;
- o: ist eine Zahl 0 oder 1;
- p,q: sind gleich oder verschieden und bedeuten eine Zahl von 0-2;
- R¹⁵: 1. Wasserstoff,
2. (C₁-C₅)-Alkyl,
3. Phenyl oder
4. (C₅-C₉)-Heteroaryl,
wobei 3. und 4. gegebenenfalls mit einer, zwei oder drei gleichen oder verschiedenen Gruppen substituiert sein können, wie Halogen, NO₂, (C₁-C₃)-Alkyl, (C₁-C₃)-Alkoxy, in denen gegebenenfalls die H-Atome des Alkylrestes jedes vorgenannten Substituenten teilweise oder vollständig durch Halogen ersetzt sind, (C₁-C₃)-Alkylthio, NR²⁰R²¹, NR²⁰CO-(C₁-C₅)-Alkyl und NR²⁰CO-Pyridyl
- R¹⁶: ist Wasserstoff, (C₁-C₄)-Alkyl oder Phenyl;
- R¹⁷: ist Wasserstoff, Halogen, (C₁-C₄)-Alkyl, NO₂ oder NH₂;
- R²⁰: ist Wasserstoff, (C₁-C₄)-Alkyl oder Benzyl;
- R²¹: ist Wasserstoff oder C(O)-O-(C₁-C₅)-Alkyl;
- Z: ist -N(R¹⁴)(R²²)
- R²²: 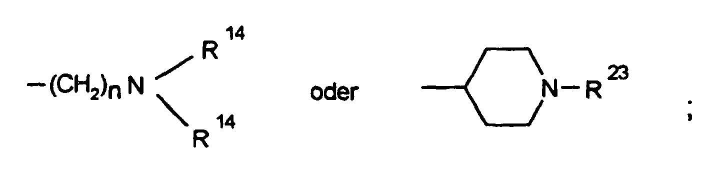
- R²³: (C₁-C₄)-Alkyl, 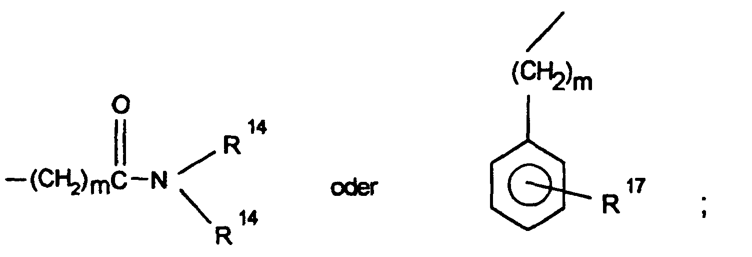
sowie deren physiologisch verträglichen Salze.

Besonders geeeignet sind Verbindungen der Formel (I), in welcher die Symbole die folgende Bedeutung haben:
- D: 1. ein Rest der Formel (Xa) oder 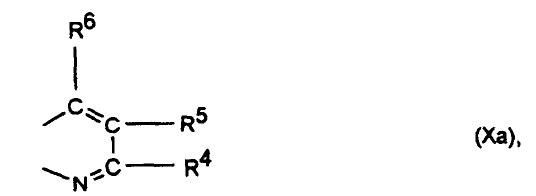
2. ein Rest der Formel (Xb) 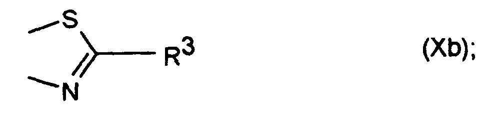
- E: 1. ein Rest der Formel (XI) 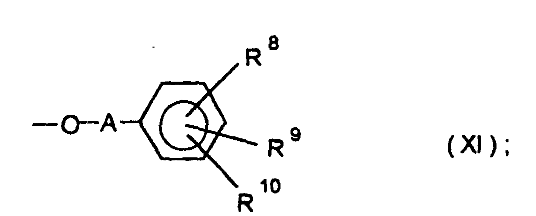
- R¹R²: sind gleich oder verschieden und stehen für Wasserstoff, Halogen und (C₁-C₄)-Alkyl;
- R⁴: Wasserstoff oder (C₁-C₄)-Alkyl;
- R⁵: Wasserstoff;
- R⁶: Wasserstoff;
- R⁸R⁹: sind gleich oder verschieden und stehen für Wasserstoff, Chlor, Methyl und Methoxy
- A: -CH₂- oder -CH₂-CH₂- ;
- R¹⁰: ein Rest der Formel (IX): 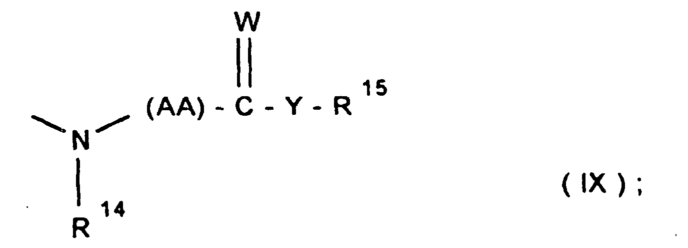
- R¹⁴: Wasserstoff, Methyl oder Ethyl;
- W: ist O;
- AA: steht für die Aminocarbonsäure Glycin;
- Y: 1. (C₂-C₅)-Alkendiyl,
2. (C₂-C₄)-Alkandiyl,
3. (C₃-C₆)-Cycloalkandiyl oder
4. -(CH₂)ₚ-Tₒ-(CH₂)_{q}- ;
- T: steht für 0 oder S;
- o: ist eine Zahl 0 oder 1;
- p,q: sind gleich oder verschieden und bedeuten eine Zahl von 0-2;
- R¹⁵: 1. Wasserstoff
2. (C₁-C₃)-Alkyl,
3. Phenyl oder
4. (C₅-C₉)-Heteroaryl,
wobei 3. und 4. gegebenenfalls mit einer, zwei oder drei gleichen oder verschiedenen Gruppen substituiert sein können, wie Halogen, NO₂, (C₁-C₃)-Alkyl, (C₁-C₃)-Alkoxy, in denen gegebenenfalls die H-Atome des Alkylrestes jedes vorgenannten Substituenten teilweise oder vollständig durch Halogen ersetzt sind, NR²⁰R²¹, NR²⁰CO-(C₁-C₃)-Alkyl und NR²⁰CO-Pyridyl;
- R²⁰: ist Wasserstoff, (C₁-C₄)-Alkyl oder Benzyl;
- R²¹: ist Wasserstoff oder C(O)-O-(C₁-C₅)-Alkyl;
sowie deren physiologisch verträglichen Salze.

Ganz besonders geeignet sind die Verbindungen
N-[1-[4-(1,1-Dimethylethyl)phenyl]methyl-4-piperidinyl]-8-methoxy4-[[4-[[[1-(phenylmethyl)-4-piperidinyl]amino]carbonyl]phenyl-amino]-3-chinolincarboxylamid;
N-[1-[(3-chlorphenyl]methyl-4-piperidinyl]-8-methoxy-4-[[4-[[[1-(phenylmethyl)-4-piperidinyl]amino]carbonyl]phenyl-amino]-3-chinolincarboxylamid;
8-Methoxy-N-[1-(phenyl]methyl-4-piperidinyl-[4-[[4-[[[1-(phenylmethyl)-4-piperidinyl]amino]carbonyl]phenyl-amino]-3-chinolincarboxylamid;
N-[2-(Dimethylamino)ethyl]-8-methoxy-4-[[4-[[[1-(phenylmethyl)-4-piperidinyl]amino]carbonyl]phenyl-amino]-3-chinolincarboxylamid-trifluoracetat;
N-[2-(Dimethylamino)ethyl]-N-ethyl-8-methoxy-4-[[4-[[[1-(phenylmethyl)-4-piperidinyl]amino]carbonyl]phenyl-amino]-3-chinolincarboxylamid;
4-[[4-[[[(3-Cyclopentyl-1-oxopropyl)-[1-[6-(diethylamino)-6-oxo-hexyl-4-piperidinylamino]-methylphenyl]-amino]-8-methoxy-N-[1-(phenylmethyl)-4-piperidinyl]-3-chinolincarboxylamid;
4-[[4-[[(1-Butyl-4-piperidinylamino]methyl]-phenylamino]-8-methoxy-N-[1-(phenylmethyl)-4-piperidinyl]-3-chinolincarboxylamid;
N-(1-Butyl-4-piperidinyl)-8-methoxy-[[4-[[[1-(phenylmethyl)-4-piperidinyl]amino]-carbonyl]-phenyl]-amino]-3-chinolincarboxylamid;
N-[1-[6-(Diethylamino)-6-oxohexyl-4-piperidinyl-8-methoxy-4-[[4-[[[1-(phenylmethyl)-4-piperidinyl]-amino]-carbonyl]-phenyl]-amino]-3-chinolincarboxylamid;
4-[[4-[[(1-Butyl-4-piperidinyl)-(1-oxobutyl)-amino]-methyl]-phenyl]-amino-[8-methoxy-N-[1-(phenylmethyl)-4-piperidinyl]-3-chinolincarboxylamid;
N-[1-[4-[(Diethylamino)-carbonyl]-phenyl-4-piperidinyl-8-methoxy-4-[[4-[[[1-(phenylmethyl)-4-piperidinyl]-amino]-carbonyl]-phenyl]-amino-3-chinolincarboxylamid;
N-[1-(2-Phenylethyl)-4-piperidinyl]-8-methoxy-4-[[4-[[[1-(phenylmethyl)-4-piperidinyl]-amino]-carbonyl]-phenyl]-amino]-3-chinolincarboxylamid;
4-[[4-[[(1-Butyl-4-piperidinyl)-amino]-carbonyl]-phenyl]-amino]-8-methoxy-N-[1-(phenylmethyl)-4-piperidinyl]-3-chinolincarboxylamid;
8-Methoxy-N-(1-methyl-4-piperidinyl)-4-[[4-[[[1-(phenylmethyl)-4-piperidinyl]-amino]-carbonyl]-phenyl]-amino]-3-chinolincarboxylamid;
N-[1-[(3-Methoxyphenyl)-methyl]-4-piperidinyl-8-methoxy-4-[[4-[[[1-(phenylmethyl)-4-piperidinyl]-amino]-carbonyl]-phenyl]-amino-3-chinolincarboxylamid;
8-Methoxy-4-[[4-[[[1-(phenylmethyl)-4-piperidinyl]-amino]-carbonyl]-phenyl]-amino]-1-[[3-(trifluoromethyl)-phenyl]-methyl]-4-piperidinyl]-3-chinolincarboxylamid; oder
7-Chloro-N-[1-(phenylmethyl)-4-piperidinyl]-4-[[4-[[[1-(phenylmethyl)-4-piperidinyl]-amino]-carbonyl]-phenyl]-amino]-3-chinolincarboxylamid;
sowie deren physiologisch verträglichen Salze.

Ganz besonders geeignet sind die folgenden, in den Tabellen 1 bis 10 aufgelisteten Verbindungen der Beispiele 1 bis 118.

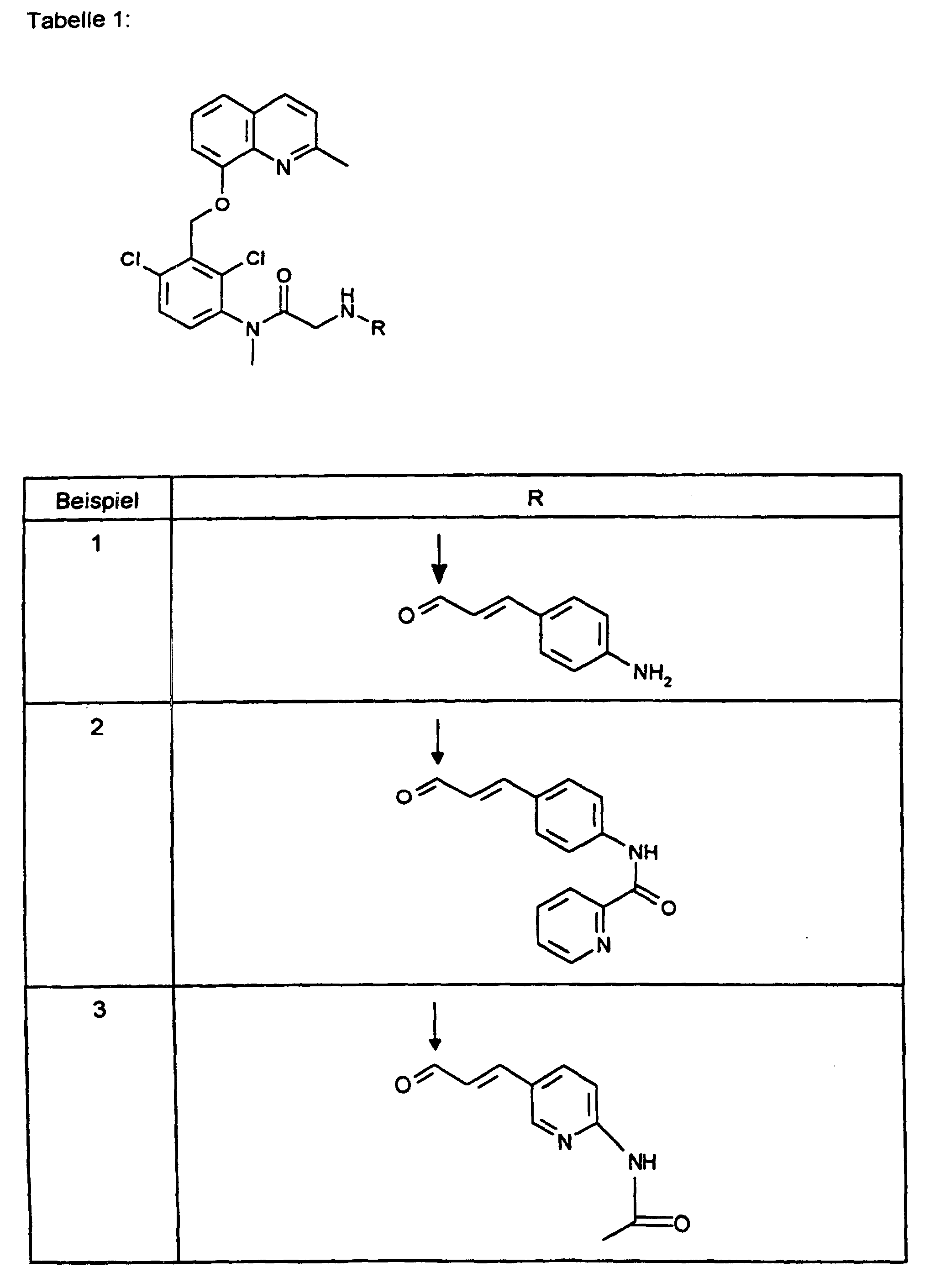

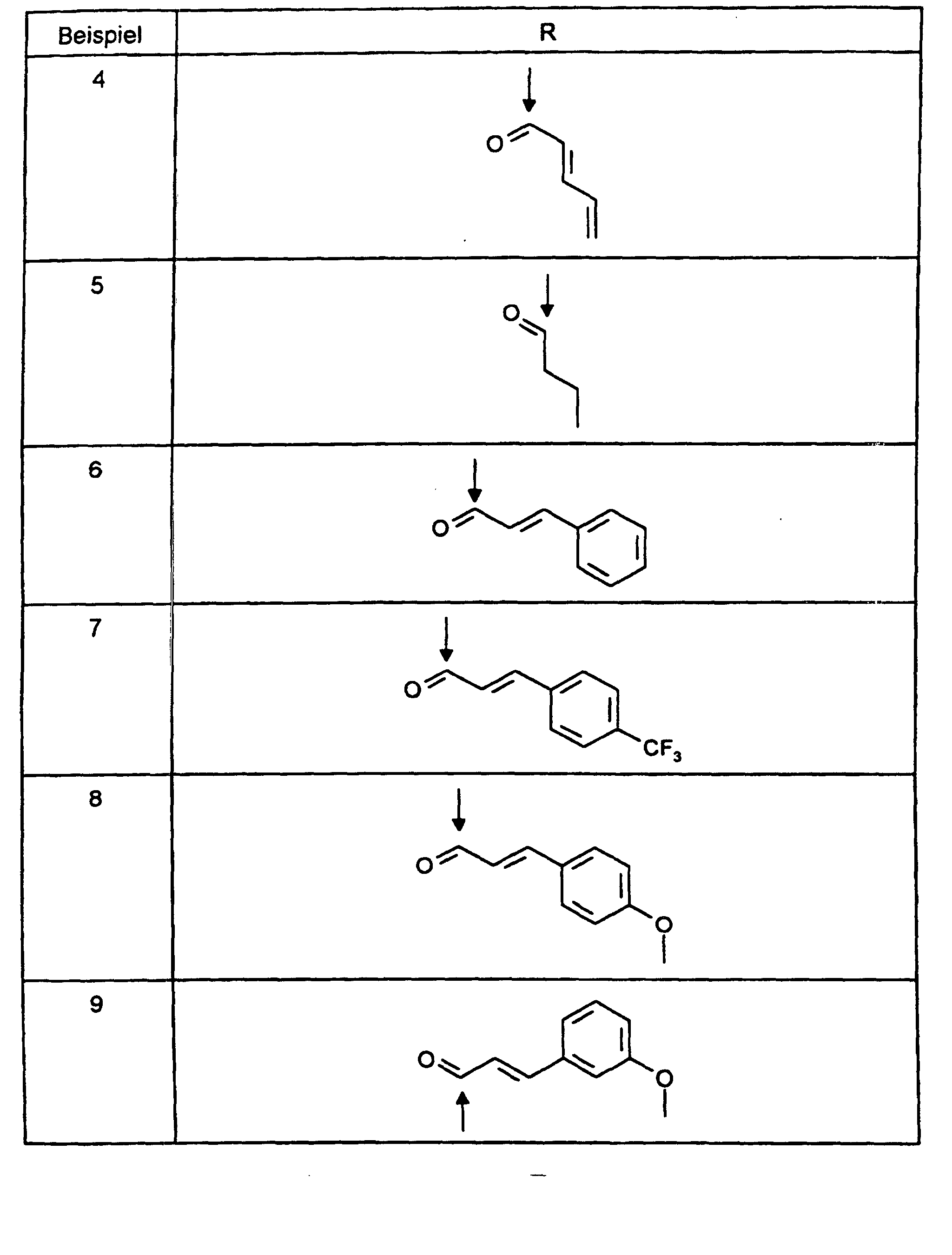

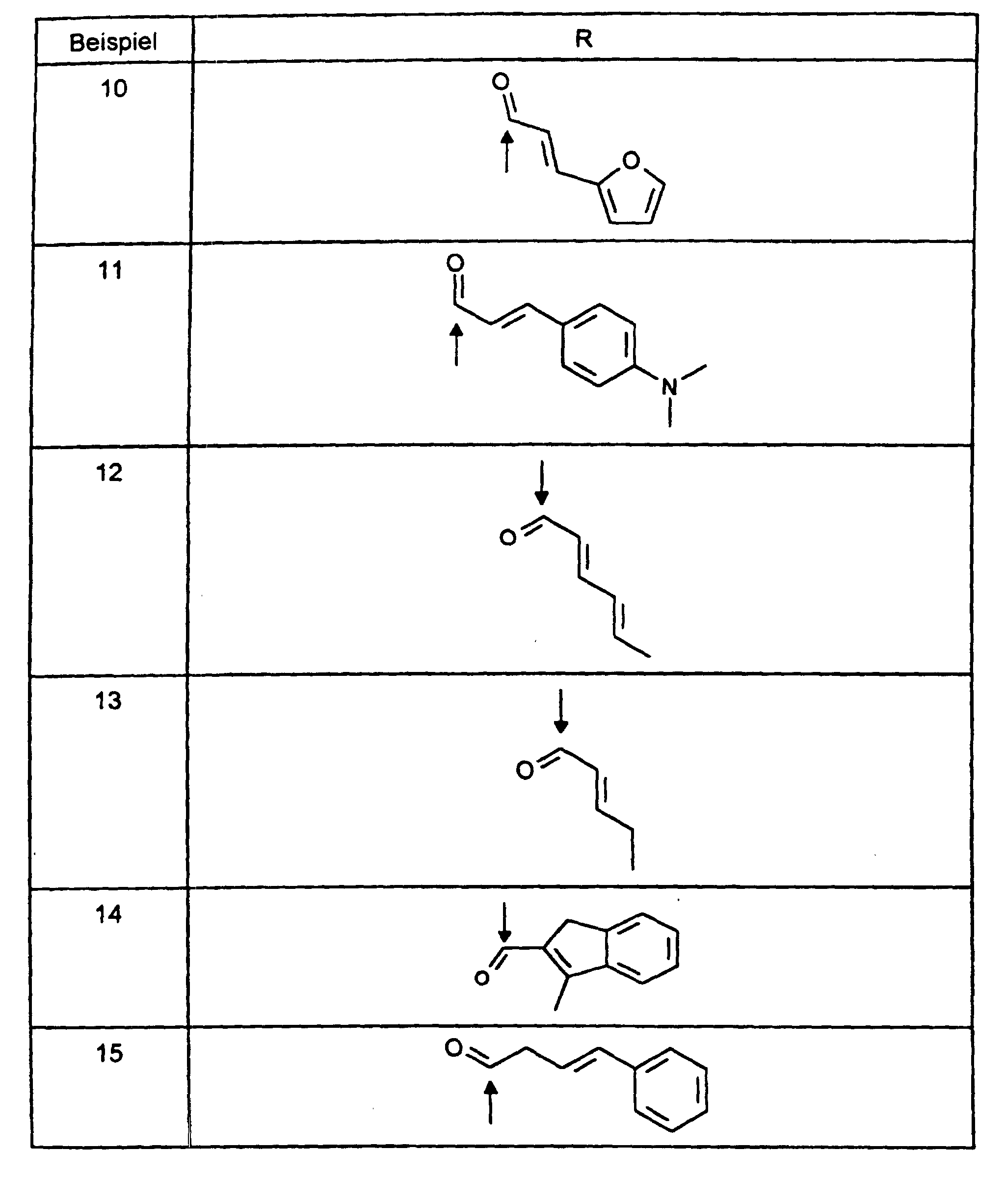

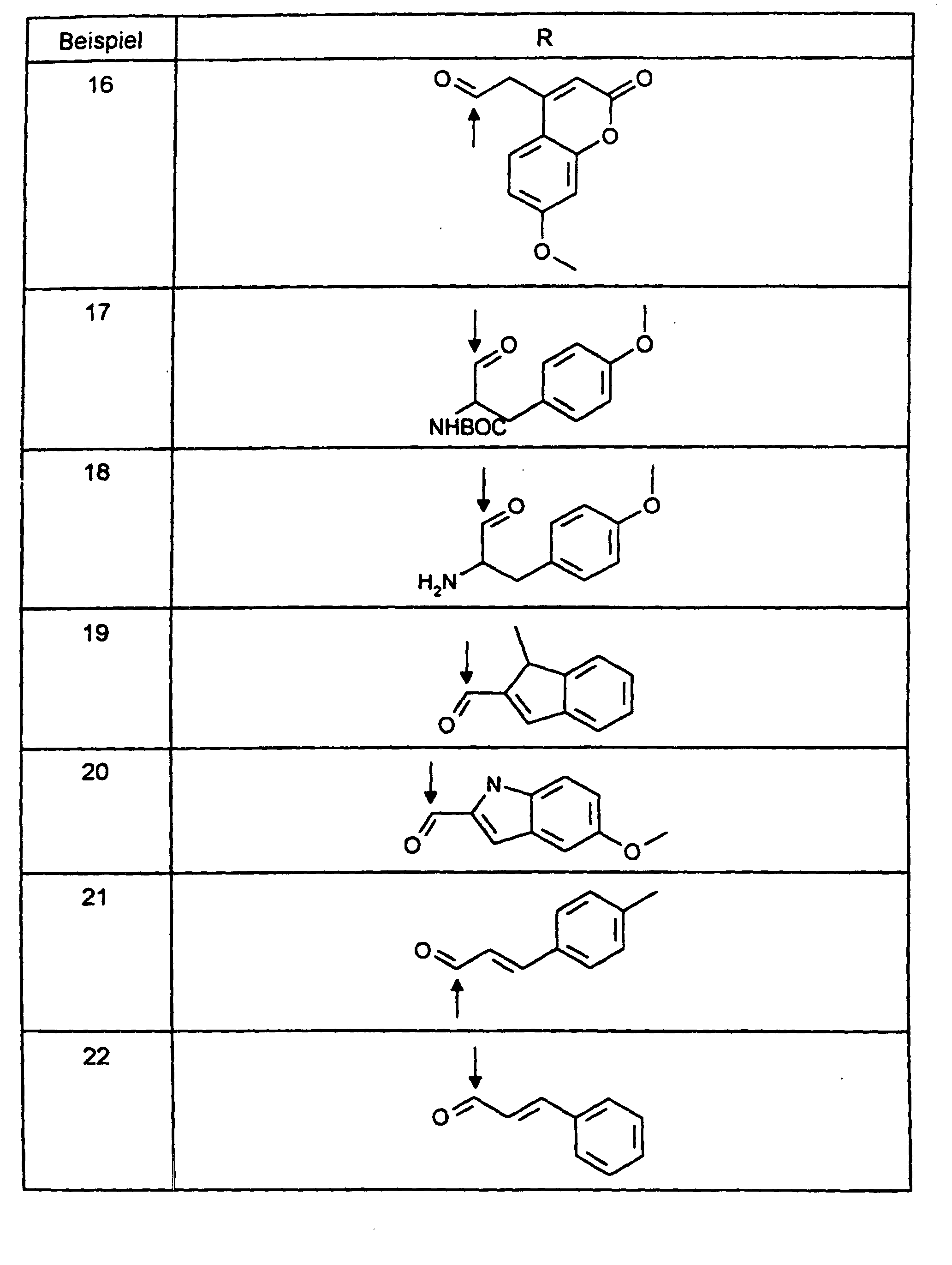

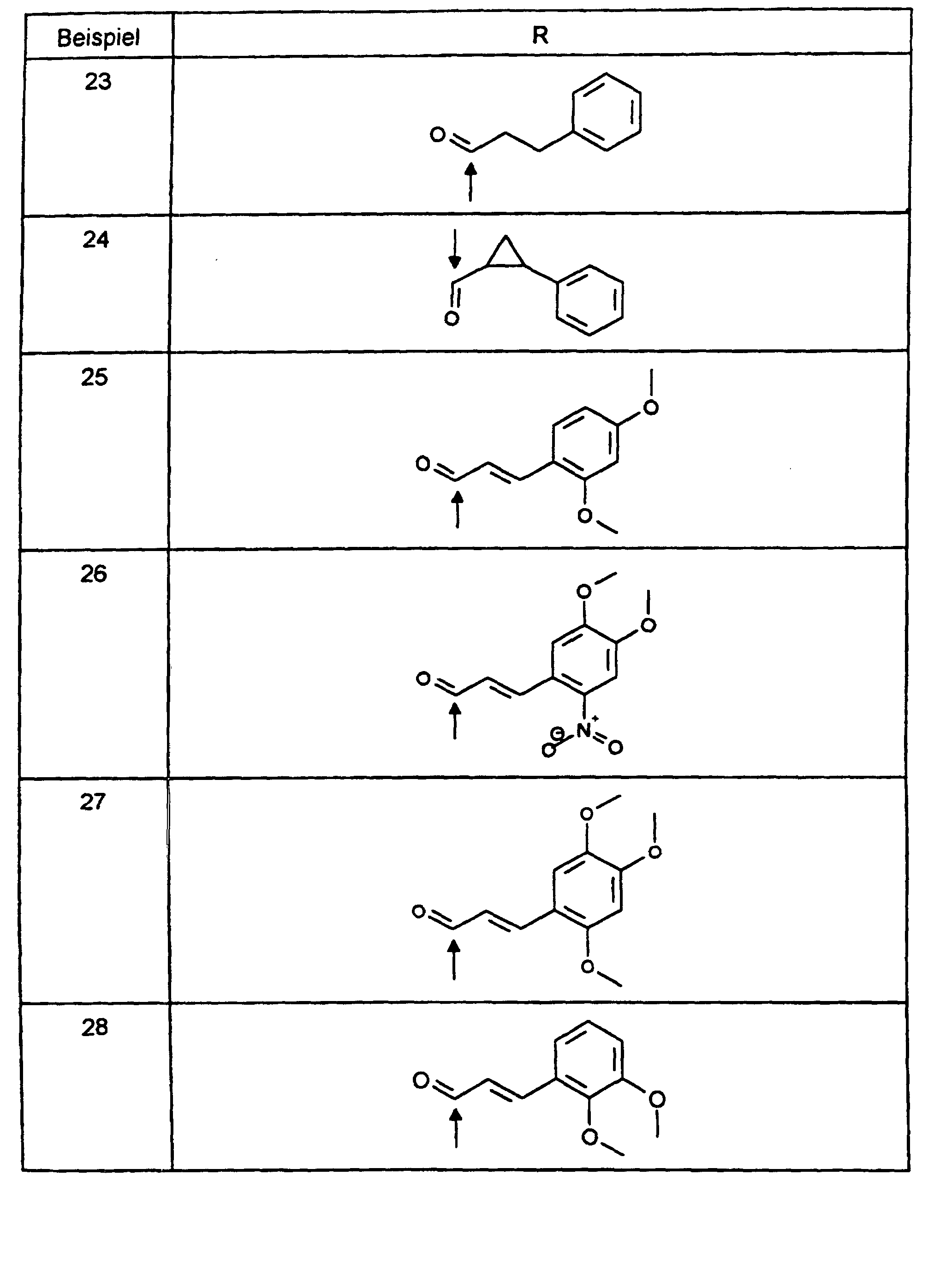

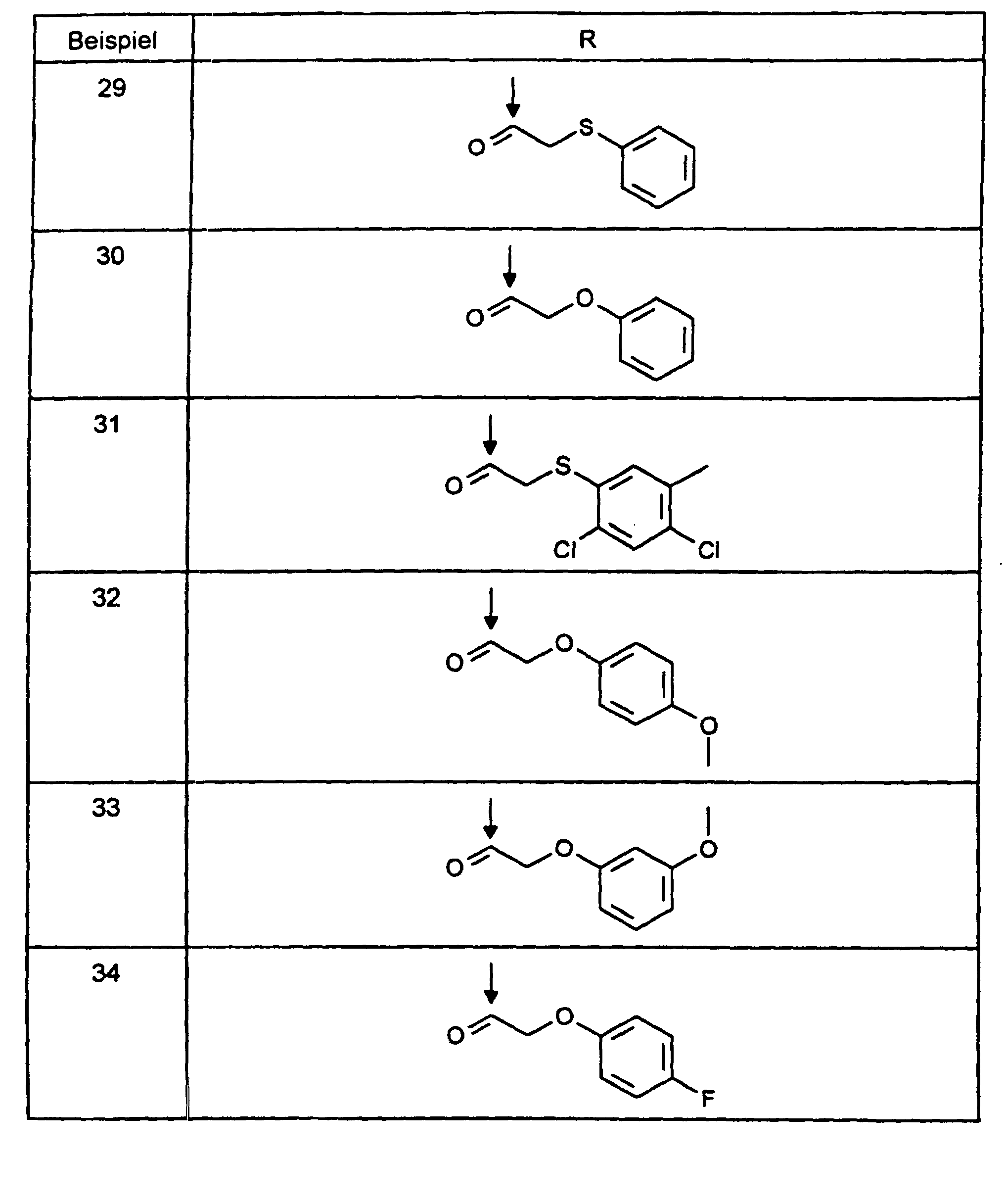

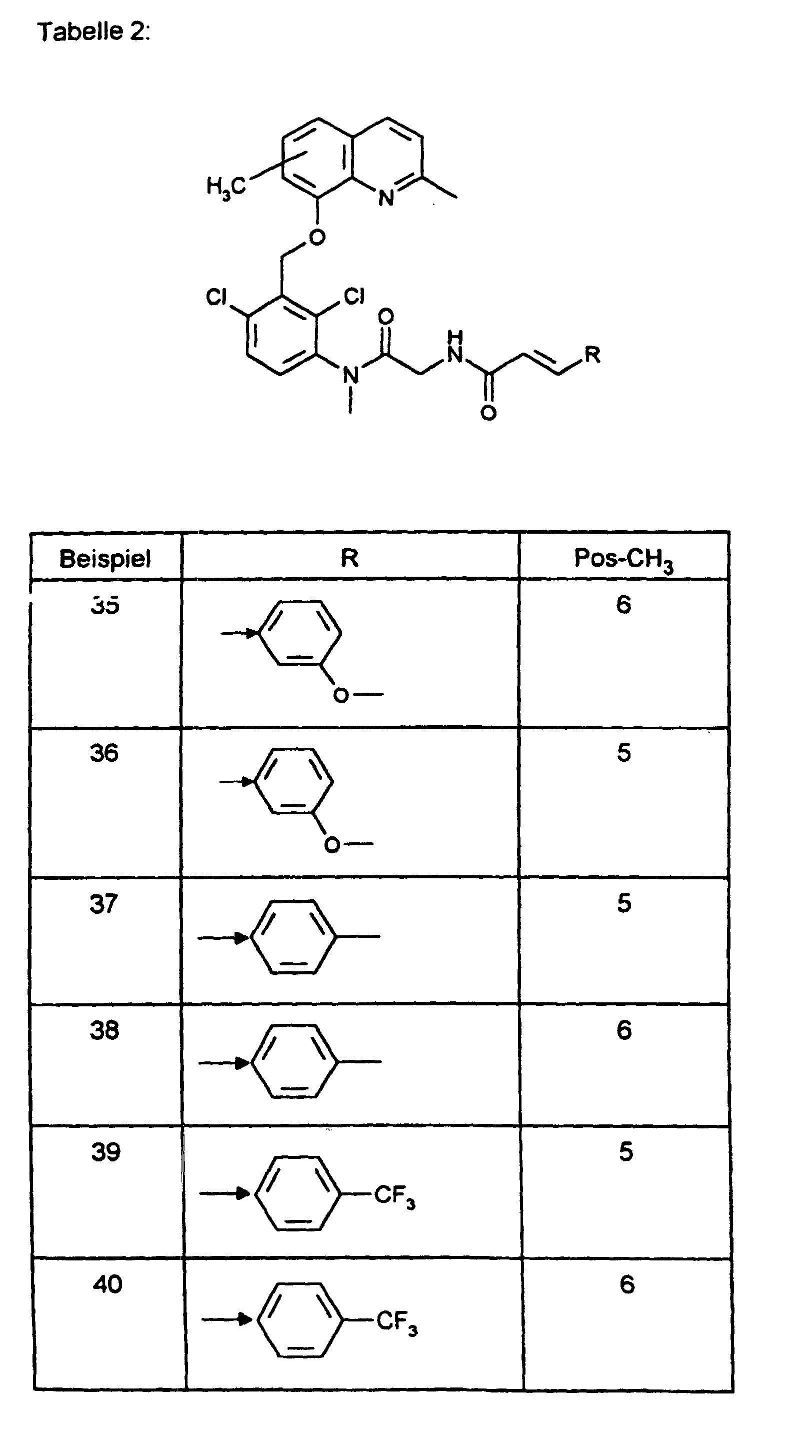

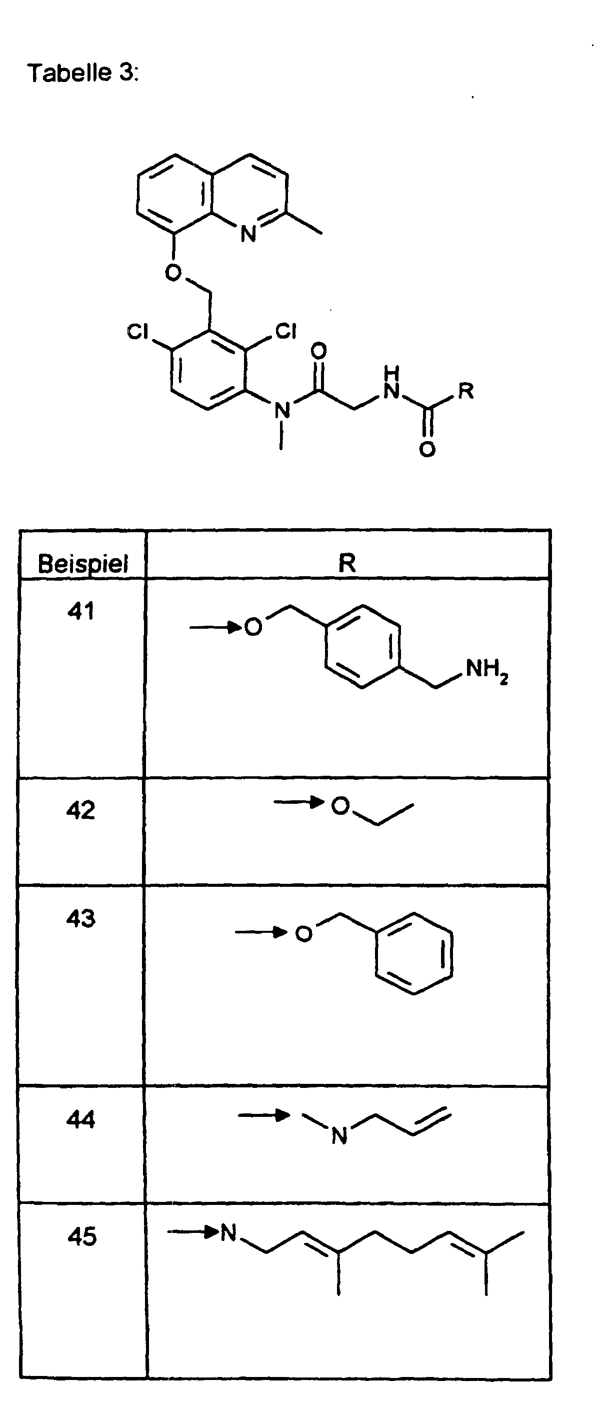

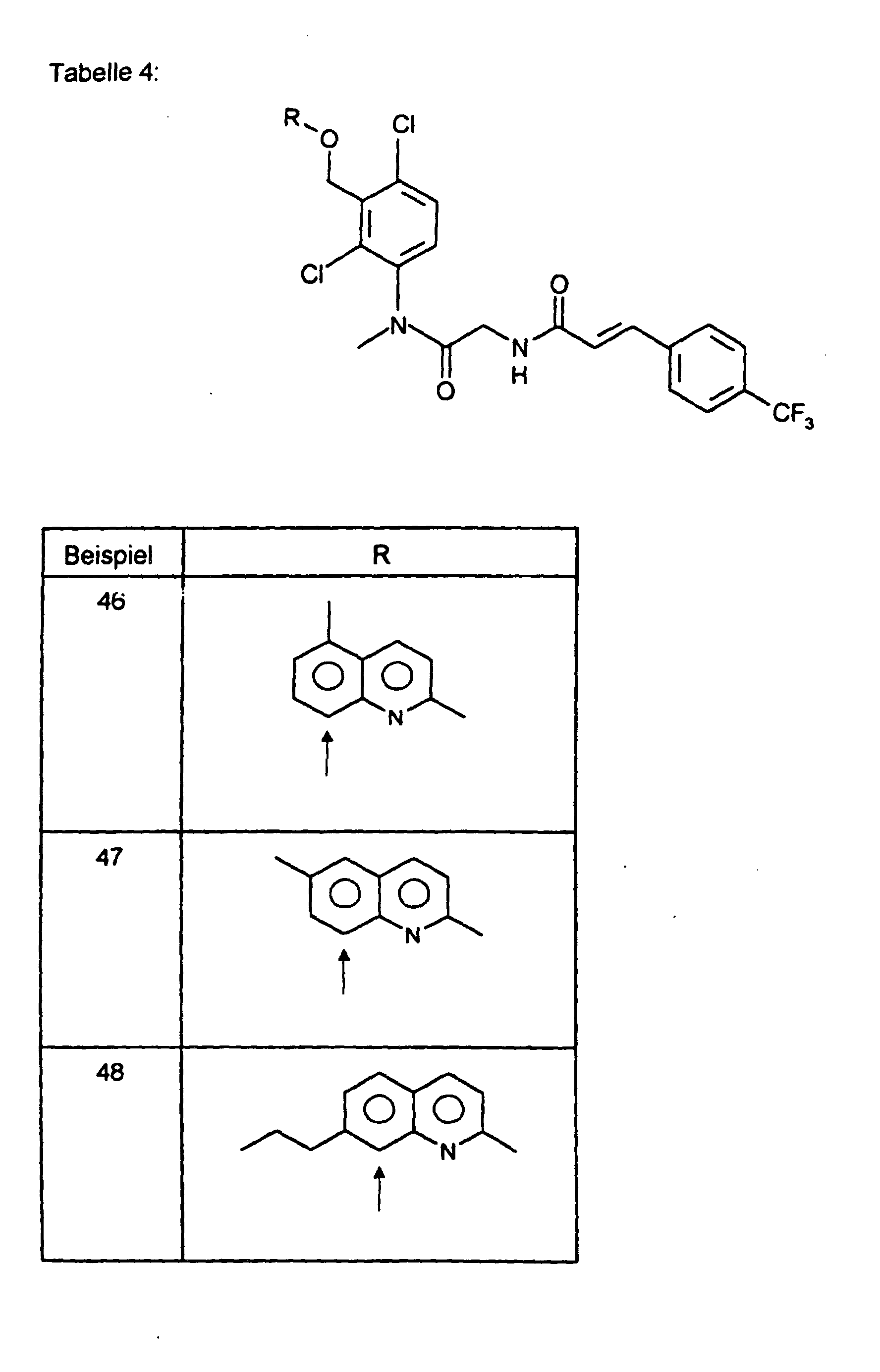

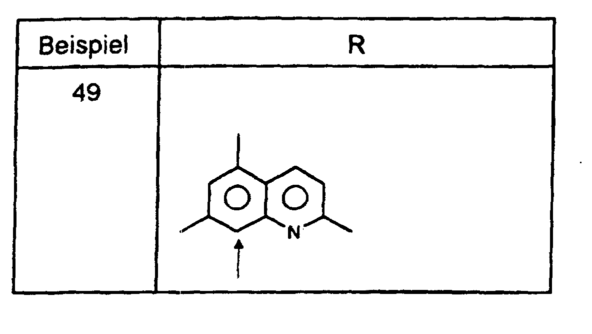

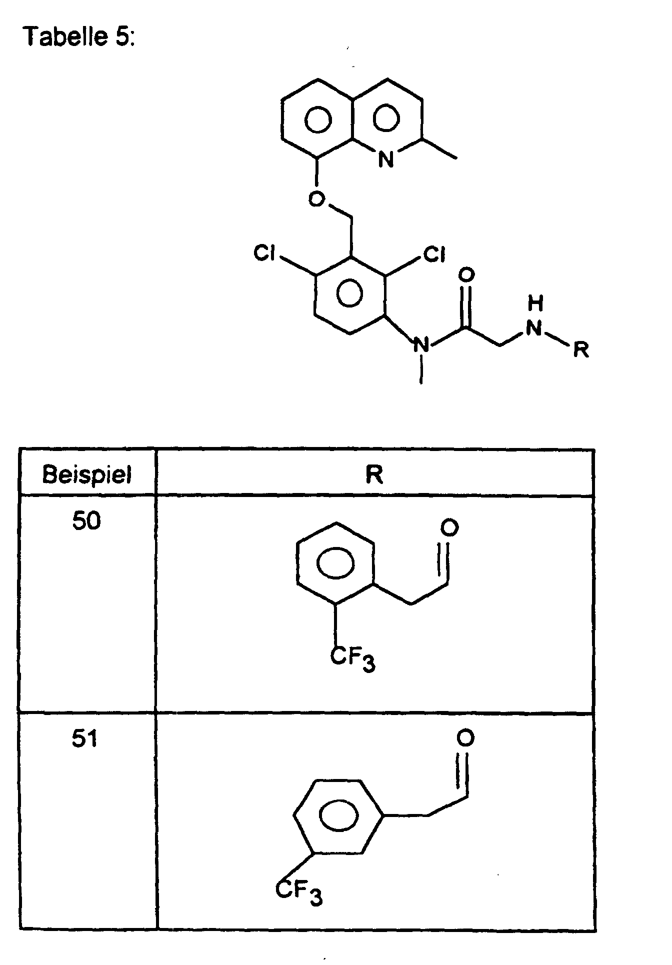

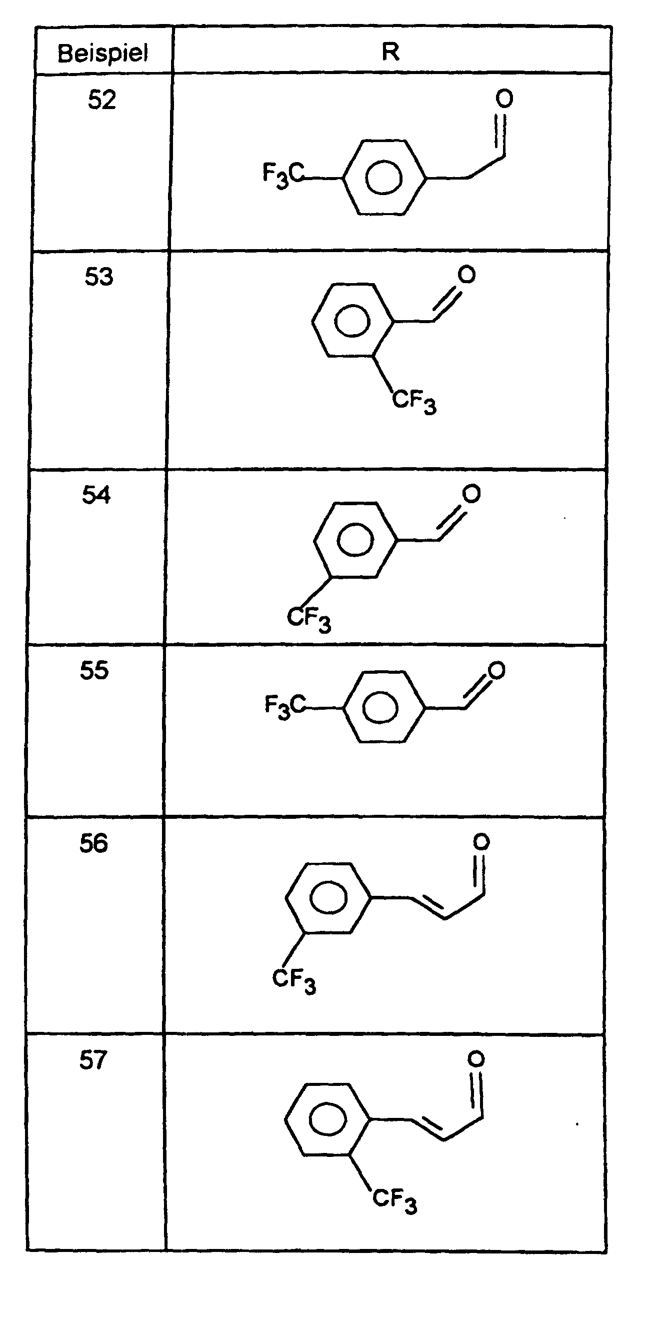

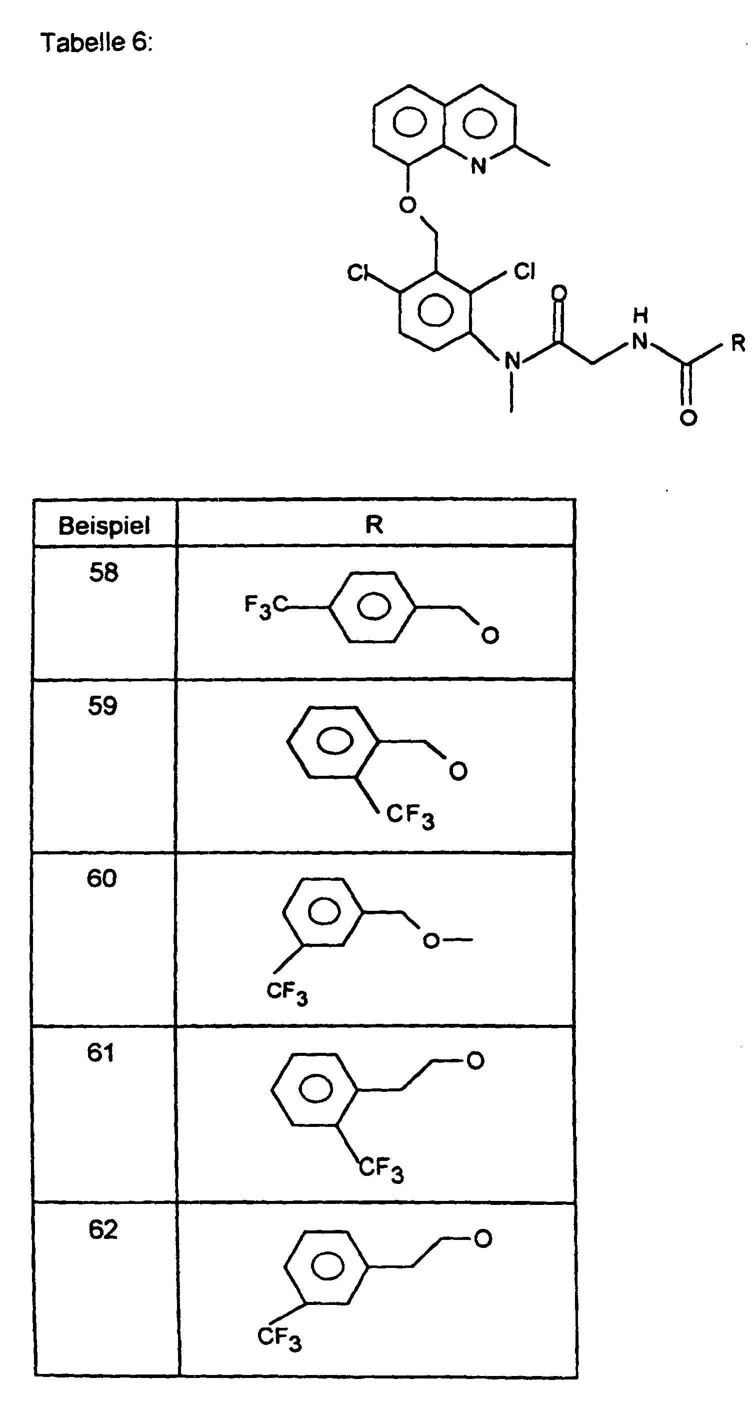

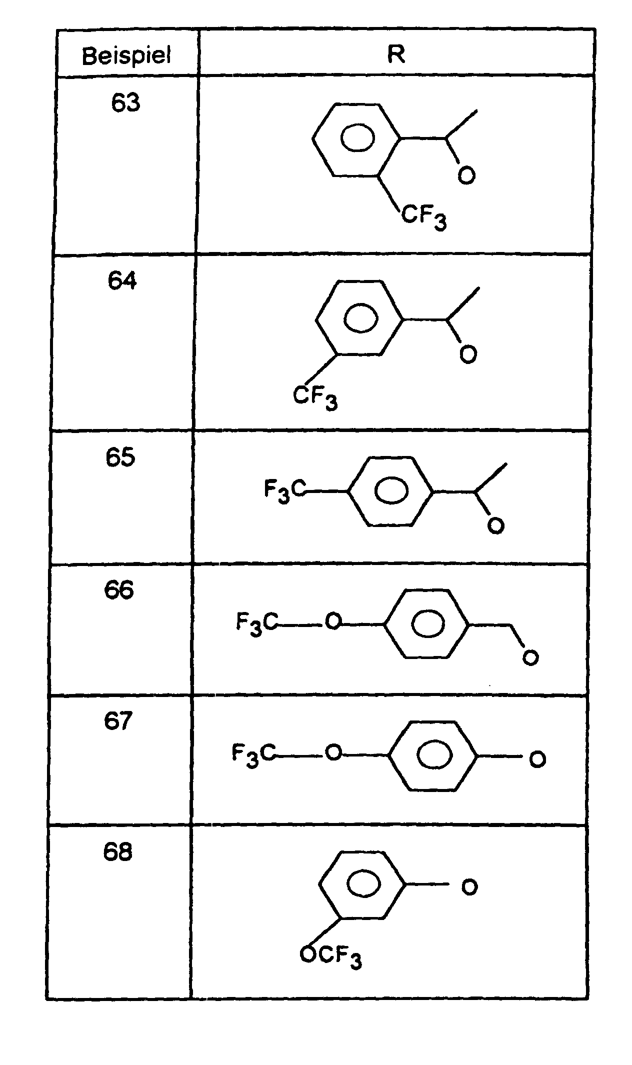

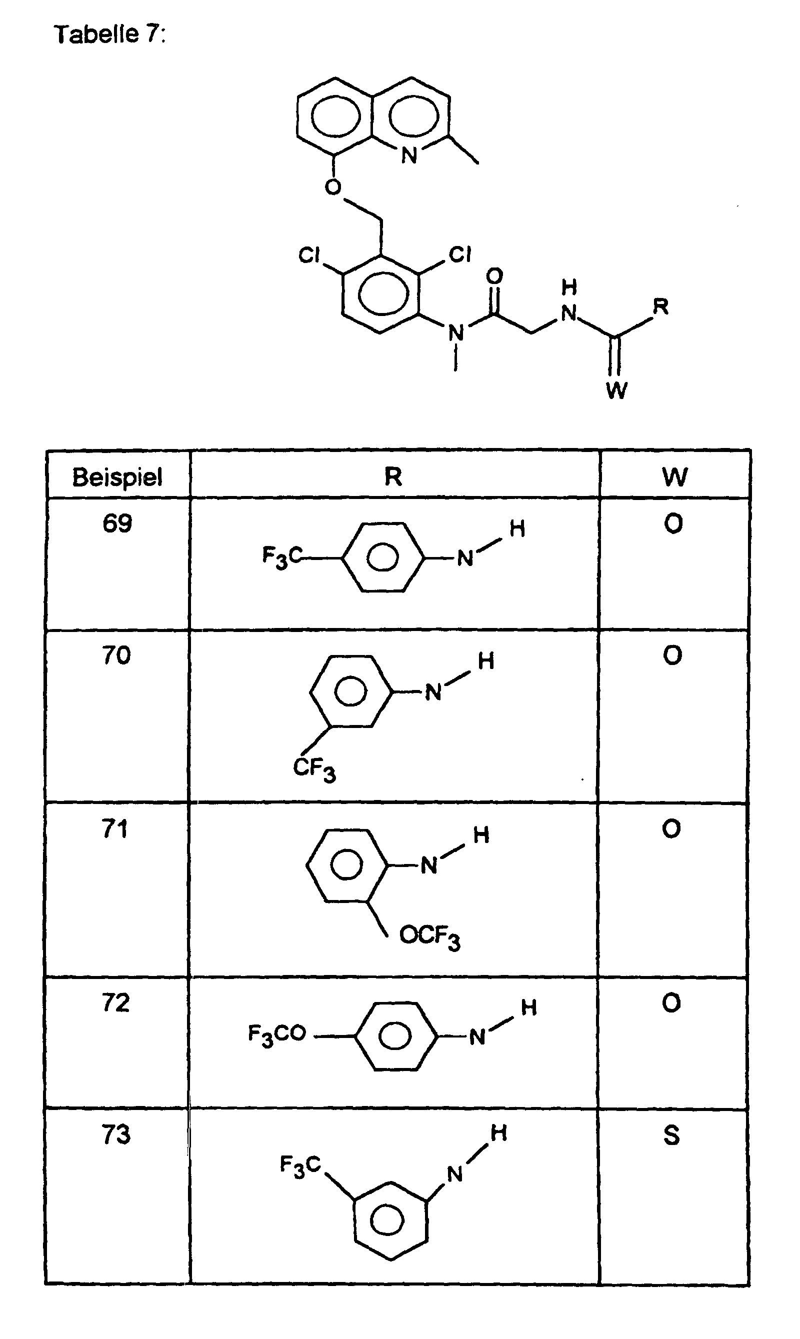

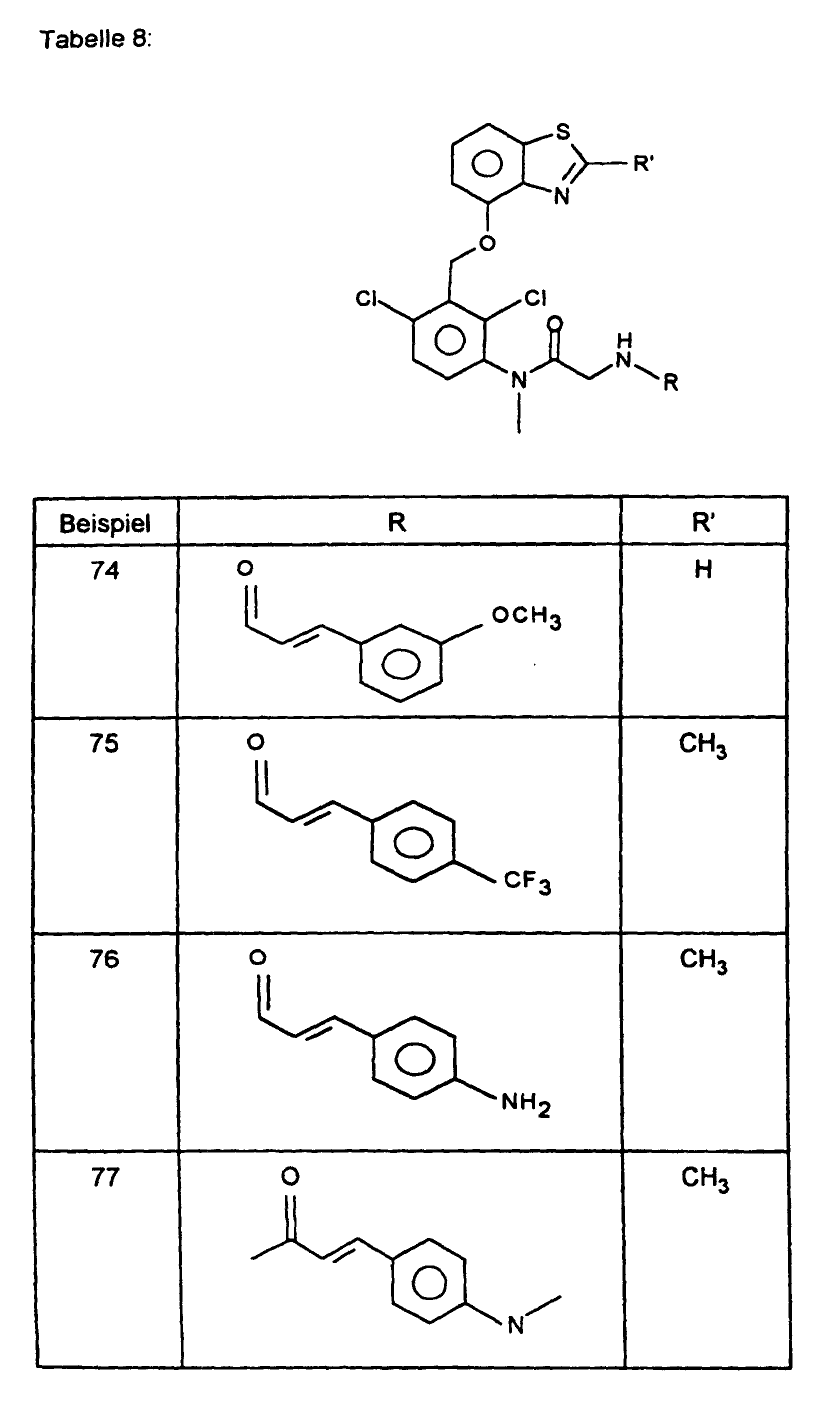

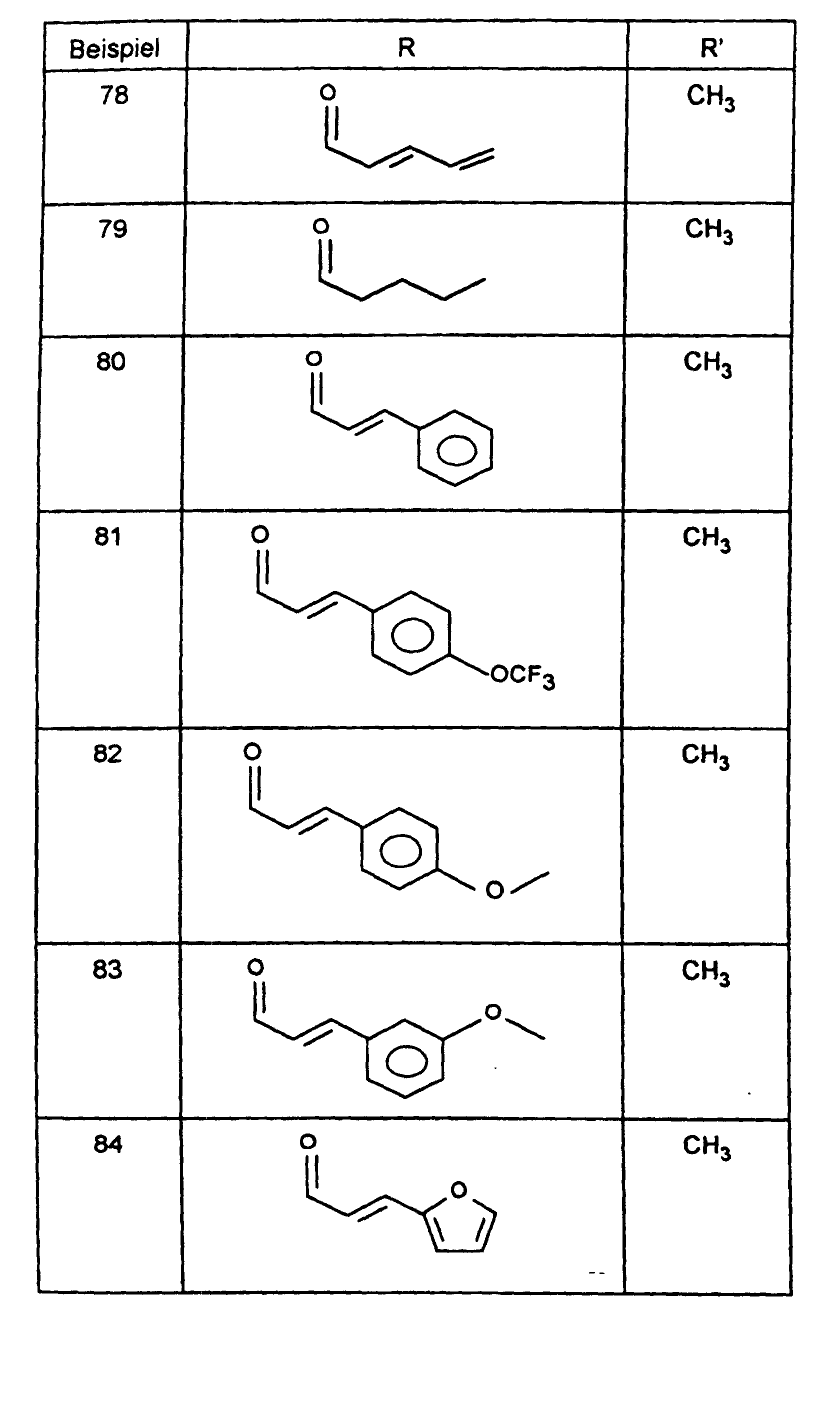

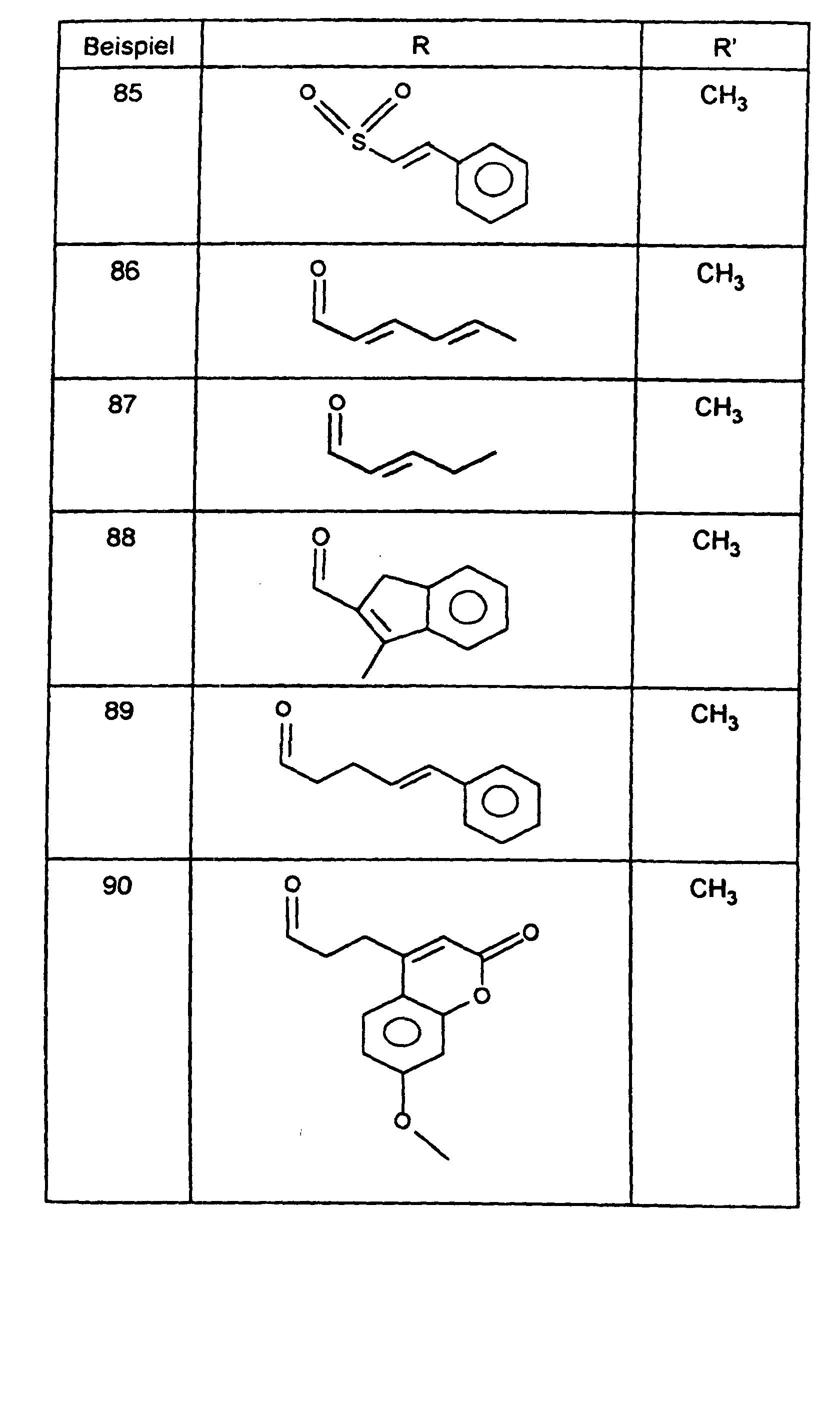

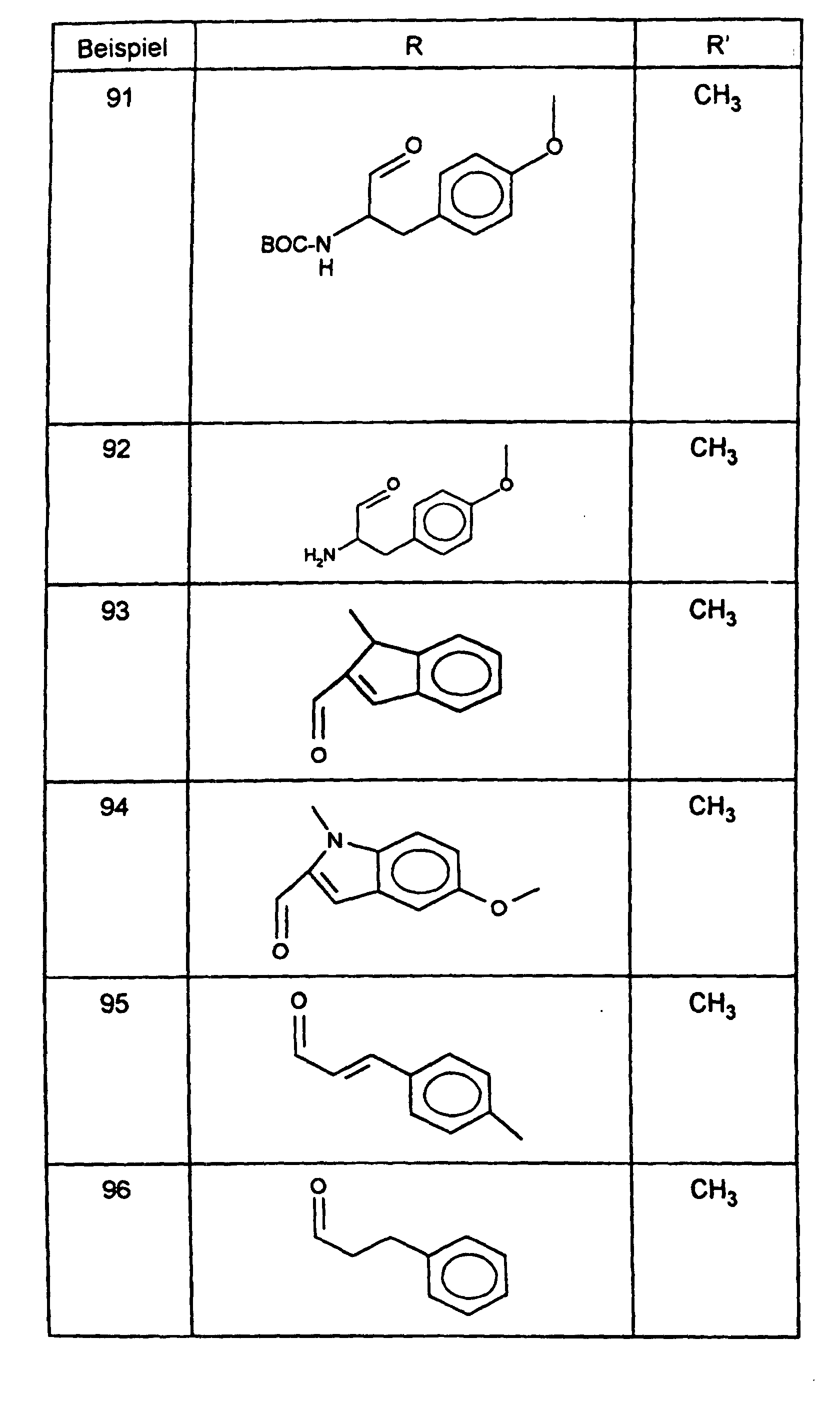

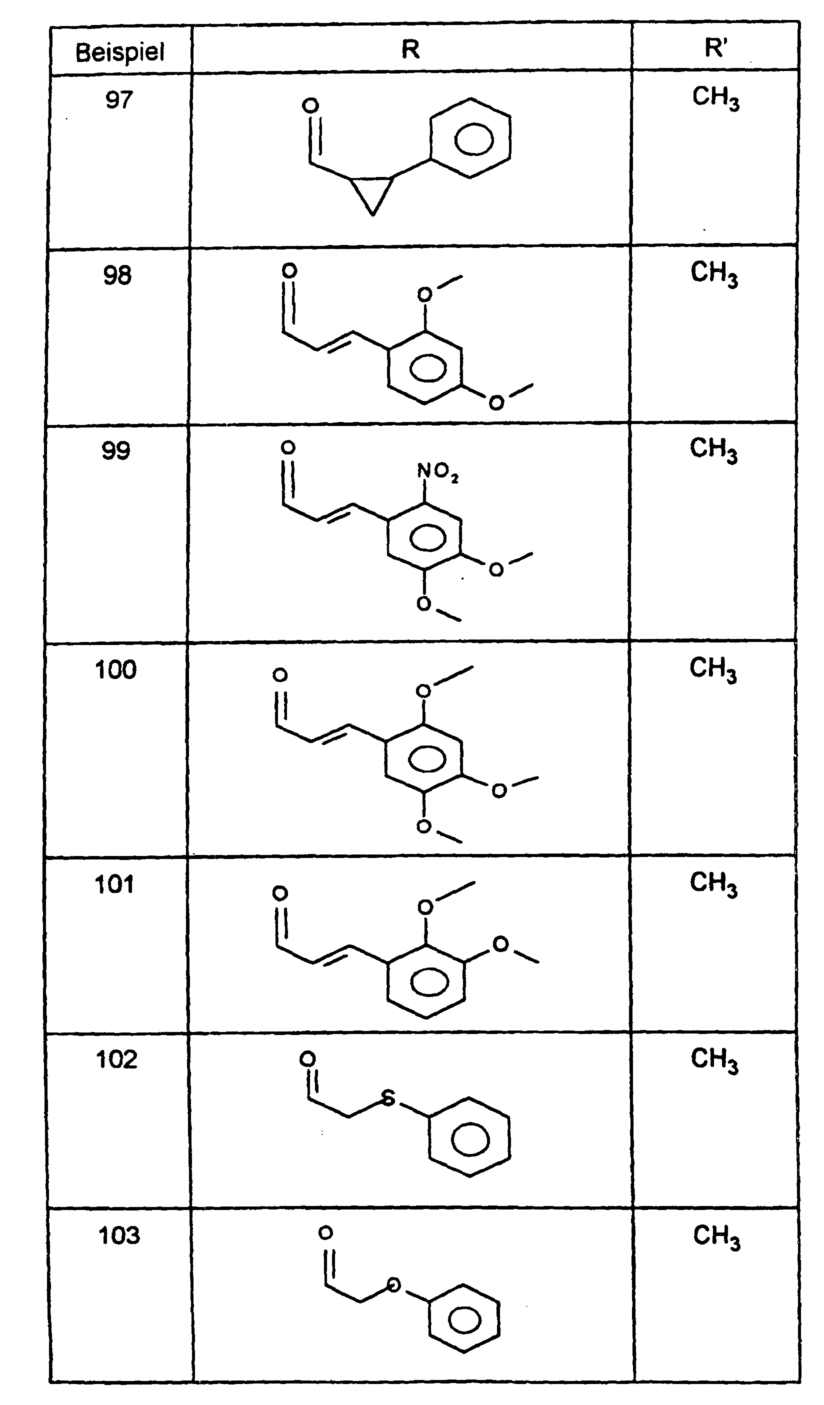

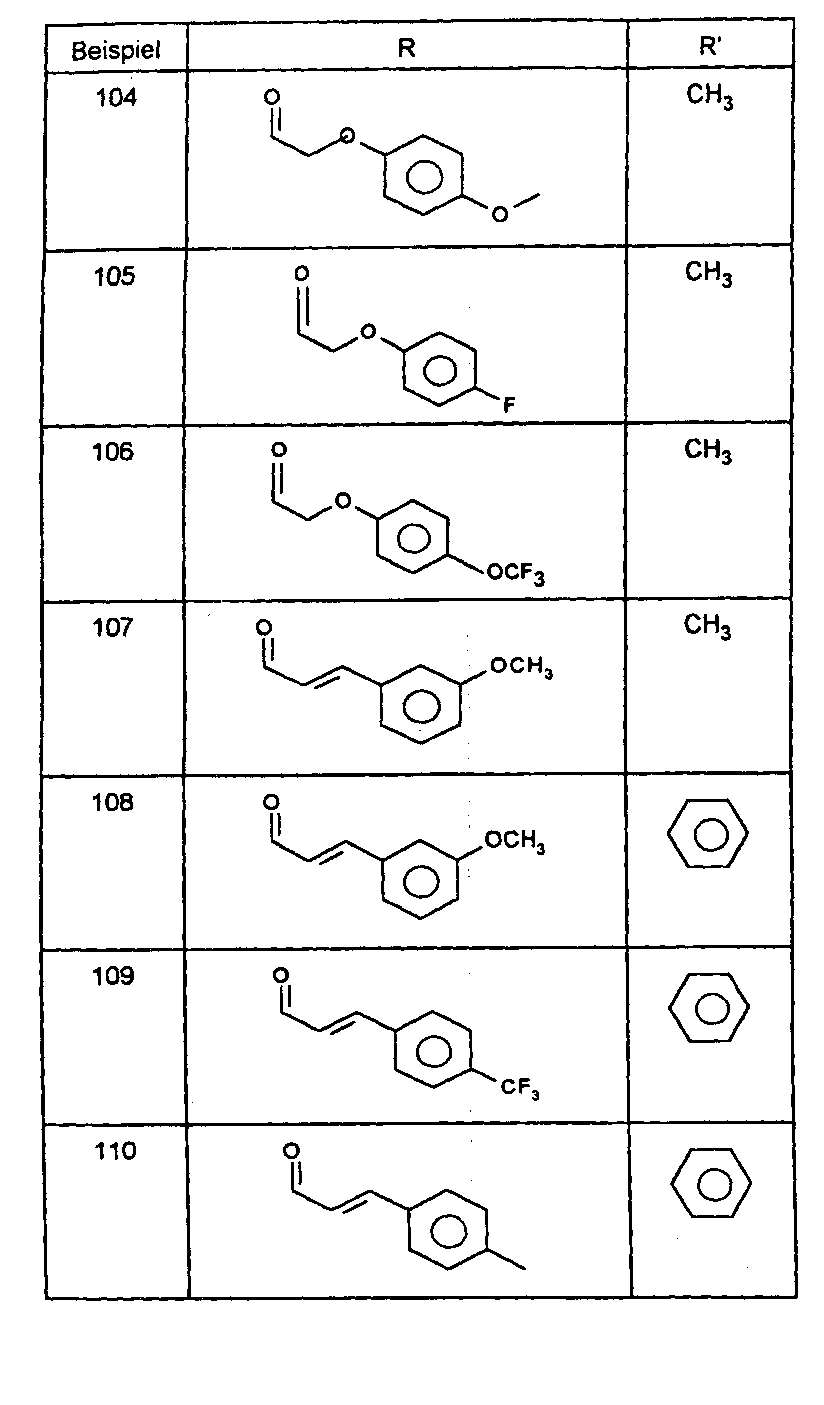

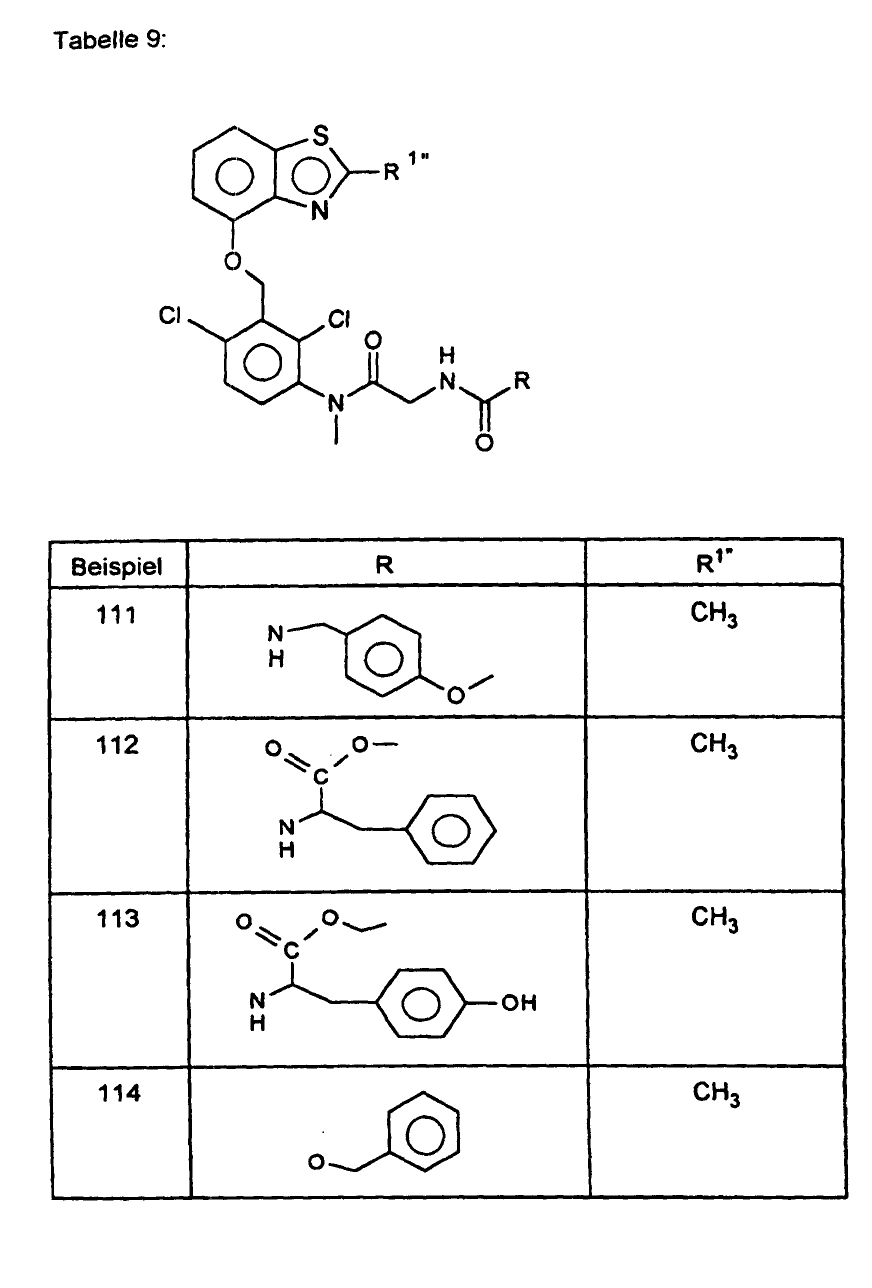

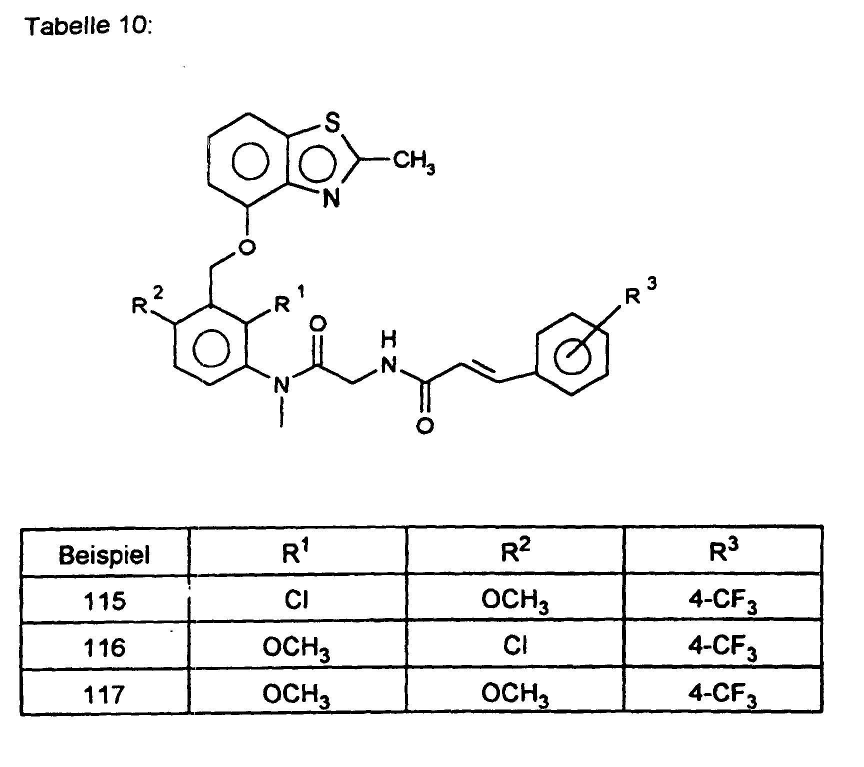

Die Anwendung kann enteral, parenteral - wie z.B. subkutan, i.m. oder i.v. -, nasal, rektal oder per Inhalation erfolgen. Die Dosierung des Wirkstoffs hängt vom Körpergewicht, Alter und von der Applikationsart ab.

Die pharmazeutischen Präparate der vorliegenden Erfindung werden in an sich bekannten Lösungs-, Misch-, Granulier-, Tabletten- oder Dragierverfahren hergestellt.

Zur parenteralen Applikation werden die aktiven Verbindungen oder deren physiologisch verträgliche Salze, gewünschtenfalls mit den pharmazeutisch üblichen Hilfsstoffen, beispielsweise zur Isotonisierung oder pH-Einstellung sowie Lösungsvermittler, Emulgatoren, oder anderen Hilfsstoffen, in Lösung, Suspension oder Emulsion gebracht.

Für die beschriebenen Arzneistoffe ist auch der Einsatz von injizierbaren Retardzubereitungen zur subkutanen oder intramuskulären Anwendung sinnvoll. Als Arzneimittel können z.B. ölige Kristallsuspensionen, Mikrokapseln, Mikropartikel, Nanopartikel oder Implantate verwendet werden, wobei die letzteren aus gewebeverträglichen Polymeren, insbesondere bioabbaubaren Polymeren, z.B. auf der Basis von Polymilchsäure-Polyglykolsäure-Copolymeren aufgebaut sein können. Andere denkbare Polymere sind Polyamide, Polyester, Polyacetate oder Polysaccharide.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige alkoholische oder ölige Suspensionen oder wäßrige alkoholische oder ölige Lösungen. Als inerte Träger können z.B. Gummi arabicum, Magnesia, Magnesiumcarbonat, Kaliumphosphat, Milchzucker, Glucose, Magnesiumstearylflumarat oder Stärke, insbesondere Maisstärke verwendet werden. Dabei kann die Zubereitung von festen Arzneiformen sowohl als Trockenund Feuchtgranulat erfolgen. Als ölige Trägerstoffe oder Lösungsmittel kommen beispielsweise pflanzliche oder tierische Öle in Betracht, wie Sonnenblumenöl und Lebertran.

Auch orale Retardzubereitungen oder Zubereitungen mit magensaftresistenten Überzügen sind denkbar. Retardzubereitungen können auf Basis von Fett-, Wachsoder Polymereinbettungen aufgebaut sein. Möglich sind hierbei auch Mehrschichtoder Manteltabletten oder Pellets.

Für die beschriebenen Arzneistoffe ist auch eine Applikation auf Schleimhäute zur Erzielung systemisch wirksamer Spiegel sinnvoll. Dies betrifft die Möglichkeit der intranasalen, inhalativen und rektalen Anwendung.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie Pulver wäßrige alkoholische oder ölige Suspensionen oder wäßrige alkoholische oder ölige Lösungen. Wäßrigen intranasalen Zubereitungen können Chelatbildner, wie Ethylendiamin-N,N,N',N'-tetraessigsäure und Puffer wie Essigsäure, Phosphorsäure, Citronensäure, Weinsäure und deren Salze zugefügt werden. Mehrfachdosenbehälter enthalten Konservierungsmittel wie Benzalkoniumchlorid, Chlorbutanol, Chlorhexidin, Sorbinsäure, Benzoesäure, PHB-Ester oder Organoquecksilberverbindungen.

Die Applikation der Nasallösungen kann mittels Dosierzerstäuber erfolgen oder als Nasaltropfen mit viskositätserhöhendem Anteil bzw. Nasengels oder Nasencremes. Für die inhalative Anwendung können Vernebler oder Druckgas-Packungen unter Verwendung inerter Trägergase benutzt werden.

Zur Applikation von Pulvern zur nasalen oder pulmonalen Inhalation sind spezielle Applikatoren erforderlich.

In Ausübung der erfindungsgemäßen Methode können die oben beschriebenen nicht-peptidischen Bradykinin-Antagonisten an Säugern wie dem Menschen, Affen, Hunden, Katzen, Ratten etc. angewendet werden.

Die wirksame Dosis der Verbindungen der Formel (I) beträgt mindestens 0,01 mg/kg/Tag, vorzugsweise mindestens 0,1 mg/kg/Tag, höchstens 30 mg/kg/Tag, vorzugsweise 0,3 bis 10 mg/kg/Tag Körpergewicht in Abhängigkeit vom Schweregrad der Symptomatik, bezogen auf einen Erwachsenen von 75 kg Körpergewicht.

### Beispiele:

Wirkung der Verbindungen der Formel (I) auf die durch das Alzheimer-Protein β/A4 stimulierte cGMP-Produktion in Endothelzellkulturen

### Testsysteme:

Bovine Aorten-Endothelzell-Kulturen (BAECs = bovine aortic endothelial cells), intravasculäre koronare Ratten-Endothelzell-Kulturen (RMCECs = rat microvascular coronary endothelial cells) und humane Nabelschnur-Endothelzell-Kulturen (HUVECs = human umbilical vein endothelial cells)

### Methode:

Bestimmung des Einflusses von Bradykinin-Antagonisten der Formel (I) auf die durch Gabe von 0.1 und 1 µmol/l des Alzheimer-Proteins β/A4 stimulierte Produktion von cGMP in Endothelzell-Kulturen verschiedener Spezies und Organen.
cGMP: zyklisches Guanosinmonophosphat

Es ist hinreichend gezeigt worden, daß Endothelzellen ein geeignetes Testsystem zum Nachweis einer Wirkung und Freisetzung von Bradykinin darstellen (G. Wiemer et al. Hypertension 1991; 18: 558-563). An Endothelzellen führt Bradykinin zur Erhöhung der Produktion von cGMP welches mittels eines Radioimmunassays bestimmt wird. Erhöhung der Bildung von cGMP durch Bradykinin ist ein Indikator für eine Freisetzung von NO (Stickstoffmonoxid) aus Endothelzellen.

### Versuch:

Stimulation der cGMP-Produktion durch βA (1-40) und inhibitorischer Effekt der Bradykinin-Antagonisten der Beispiele 3 und 7 (10⁻⁷ mol/l) in 3 verschiedenen Arten von Endothel-Zellen:

| Endothel Zell-Typ: | BAECs | RMCECs | HUVECs |
|---|---|---|---|
| | Konz. cGMP (pmol/mg Protein) | | |
| Basale cGMP-Produktion | 2.2 ± 0.35 | 0.2 ± 0.07 | 4.75 ± 0.4 |
| BK 10 ⁻⁸ mol/l | 8.8 ± 0.34 | 1.13 ± 0.2 | 11.46 ± 2 |
| BK 10 ⁻⁸ mol/l + Beispiel 7 | 1.9 ± 0.19 | 0.30 ± 0.03 | 4.02 ± 0.45 |
| βA (1-40) 10 ⁻⁷ mol/l | 5.9 ± 0.23 | 0.59 ± 0.07 | 14.07 ± 1.6 |
| βA (1-40) 10 ⁻⁷ mol/l + Bsp. 7 | 1.0 ± 0.1 | 0.24 ± 0.01 | 4.26 ± 0.45 |
| βA (1-40) 10 ⁻⁶ mol/l | 4.6 ± 0.13 | 0.74 ± 0.09 | 15.3 ± 1.9 |
| βA (1-40) 10 ⁻⁶ mol/l + Bsp. 7 | 3.0 ± 0.14 | 0.39 ± 0.03 | 5.9 ± 0.55 |
| βA (1-40) 10 ⁻⁶ mol/l + Bsp. 3 | 1.9 ± 0.14 | 0.30 ± 0.04 | 4.2 ± 0.43 |

### Ergebnis:

Die gleichzeitige Inkubation der o.a. Zellkulturen verschiedener Species und Organe mit Verbindungen der Beispiele 3 und 7 als repräsentative Beispiele der Verbindungen der Formel (I) in einer Konzentration von 0.1 µmol/l verhindert die vom β/4A-Protein ausgelöste Stimulation der Produktion von cGMP.

### Bewertung:

Der durchgeführte Versuch zeigt an, daß die Wirkung des Alzheimer-Proteins β/A4 auf die Produktion von cGMP über eine Bindung von Bradykinin an seine Zellrezeptoren vermittelt ist.
Endothelzellkulturen dienen hierbei als Indikator einer Wirkung von β/A4 die über Bradykinin vermittelt ist. Die Endothelzellen sind dabei jedoch nicht nur das Indikatorsystem für eine Wirkung über Bradykinin-Rezeptoren, sondern auch das Effektororgan bei der Alzheimerschen Krankheit. Endothelzellen sind Bestandteile der Blutgefäße und bilden diese aus. Die Blutgefäße selbst sind von Ablagerungen des Alzheimer-Proteins Amyloid (β/A4) bei der Alzheimerschen Krankheit neben neuronalem Gewebe stark betroffen. Endothelzellen sind verantwortlich für eine durch Bradykinin ausgelöste Steigerung der Durchlässigkeit der Bluthirnschranke.

Zunehmend wird die Bedeutung einer lokalisierten Entzündung für die destruktiven Veränderungen im Gehirn von Patienten mit Alzheimerschen Krankheit erkannt. Entzündliche Veränderungen führen zur Chronifizierung und zur fortschreitenden Destruktion des Gehirns und damit schweren Demenz (J. Rogers, Inflammation as a pathogenic mechanism in Alzheimer's disease. Arzneimittelforschung 1995; 45 (3A), 439-442). Es war bisher nicht bekannt, daß Bradykinin, ein stark entzündlicher Mediator in der Peripherie, eine Rolle bei Alzheimerscher Krankheit spielen könnte. Dies ist darauf zurückzuführen, daß es keine Vorstellungen zur Freisetzung von Bradykinin im Gehirn von Patienten mit Alzheimerscher Krankheit gab. Die inaktiven hochmolekularen Vorstufen, aus denen Bradykinin freigesetzt wird, können nämlich wegen der geringen Durchlässigkeit der Bluthirnschranke nicht ohne weiteres ins Gehirn (neuronale Gewebe) gelangen.
Unsere Untersuchungen zeigen, daß das Alzheimer Protein β/A4 aus dem Endothel von Gefäßwänden Bradykinin freisetzen kann. Dem Alzheimer Protein β/A4 werden die wesentlichen pathologischen Veränderungen der Alzheimer Krankheit zugeschrieben (Joachim C.L. Selkoe D.J."The seminal role of beta-amyloid in the pathogenesis of Alzheimer disease" Alzheimer-Dis-ASSOC-Disord. 1992, Spring; 6(1): 7-34). Ist einmal die Freisetzung des entzündlich wirksamen Bradykinins durch einen Mechanismus gezeigt, der spezifisch für die Alzheimersche Krankheit ist, wird Bradykinin zum pathophysiologischen Faktor ersten Ranges, über welchen das Alzheimer Protein seine destruktive Wirkung vermitteln kann. Dies gilt vor allem im Hinblick auf die Entzündung, deren Bedeutung für die destruktiven Veränderungen zunehmend erkannt wird, denn Bradykinin ist eine der stärksten entzündlich wirksamen körpereigenen Substanzen. Neben der entzündlichen Wirkung besitzt Bradykinin noch zwei weitere Eigenschaften, durch welche es zu den destruktiven Veränderungen bei Alzheimersche Krankheit beitragen kann. Bradykinin stimuliert ZNS-Neurone. Bei starker Stimulation führt dies zur Kalziumüberladung der betroffenen Zellen mit nachfolgendem Zelltod. Bei mäßiger Stimulierung wird Bradykinin nur zum falschen Transmitter, welcher Neurone inadäquat stimuliert. Eine solche inadäquate Stimulierung von Neuronen, die eigentlich gar nicht erregt werden sollen, kann den Prozeß der Informationsverarbeitung im Gehirn empfindlich stören und zu den typischen Hirnleistungsstörungen beitragen, wobei der letztere Mechanismus, ausgelöst durch mäßige Stimulierung, reversibel erscheint.
Als vasoaktiver Mediator erhöht Bradykinin bekanntermaßen die Durchlässigkeit der Bluthirnschranke. Dies führt dazu, daß die Vorstufen von Bradykinin erst von den Blutgefäßen ins Gehirn gelangen können, um dort ihre zerstörerische Wirkung zu entfalten.

Somit sind nicht-peptidische Bradykininantagonisten der Formel (I) geeignet für die therapeutische und präventive Behandlung der Alzheimer'schen Krankheit.

## Patentansprüche

1. Verwendung eines nicht-peptidischen Bradykinin-Antagonisten der Formel (I) 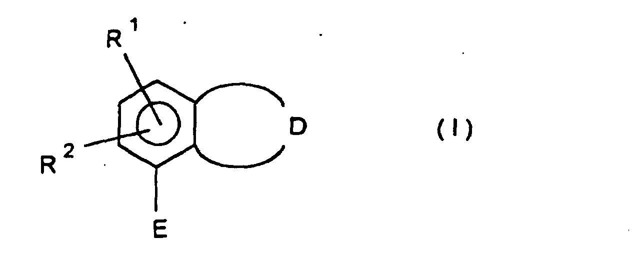 in welcher die Symbole folgende Bedeutung haben:
D
1. ein Rest der Formel (II) oder 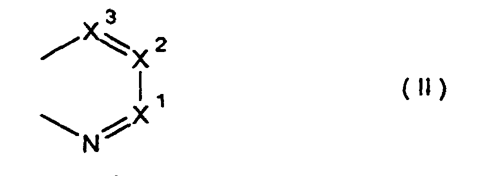
2. ein Rest der Formeln (III) bis (VI): 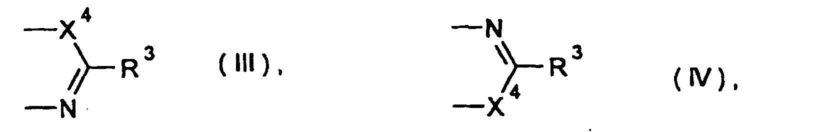 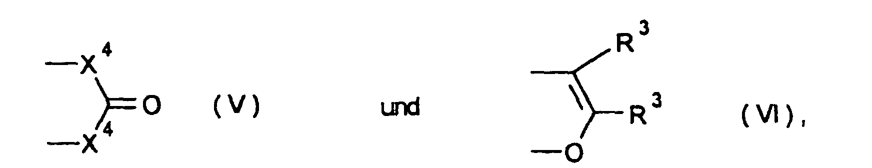
E
1.ein Rest der Formel (VII) oder 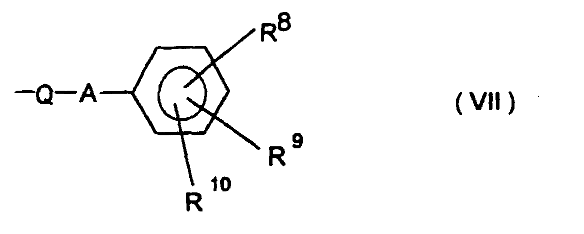
2. Wasserstoff, Halogen, (C₁-C₄)-Alkyl, (C₁-C₄)-Alkylthio oder (C₁-C₄)-Alkoxy, wobei in den letzten 3 Resten 1 oder mehrere Wasserstoffatome durch Fluor ersetzt sein können;
X¹ Stickstoff oder C-R⁴ ;
X² Stickstoff oder C-R⁵ ;
X³ Stickstoff oder C-R⁶ ;
X⁴ unabhängig voneinander Sauerstoff, Schwefel, Stickstoff oder N-R⁷ ;
R¹,R² sind gleich oder verschieden und stehen für Wasserstoff, Halogen, (C₁-C₄)-Alkyl und (C₁-C₄)-Alkoxy;
R³ unabhängig voneinander Wasserstoff, Halogen, (C₁-C₆)-Alkyl, (C₆-C₁₂)-Aryl, (C₁-C₃)-Alkyl-(C₆-C₁₂)-Aryl, (C₃-C₅)-Alkenyl, (C₁-C₄)-Alkoxy oder CO₂R¹¹;
R⁴ Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, wobei der Alkylrest jedes vorgenannten Substituenten gegebenenfalls substituiert ist mit Hydroxy, (C₁-C₄)-Alkoxy, Amino und (C₁-C₄)-Alkylamino, oder (C₆- C₁₂)-Aryl, gegebenenfalls substituiert mit (C₁-C₄)-Alkyl oder CO₂R¹¹;
R⁵
1. Wasserstoff, (C₁-C₄)-Alkyl oder
2. 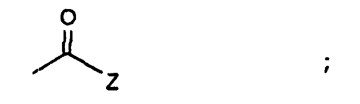
R⁶
1. Wasserstoff, Halogen, (C₁-C₄)-Alkyl, Hydroxy, (C₁-C₄)-Alkylthio, Amino, (C₁-C₄)-Alkylamino, (C₁-C₄)-Dialkylamino, (C₁-C₄)-Alkoxy, wobei der Alkylrest jedes vorgenannten Substituenten gegebenenfalls substituiert ist mit Hydroxy, (C₁-C₄)-Alkoxy, Amino und (C₁-C₄)-Alkylamino, oder (C₆-C₁₂)-Aryl, gegebenenfalls substituiert mit (C₁-C₄)-Alkyl oder CO₂R¹¹; oder
2. ein Rest der Formel (VIII): 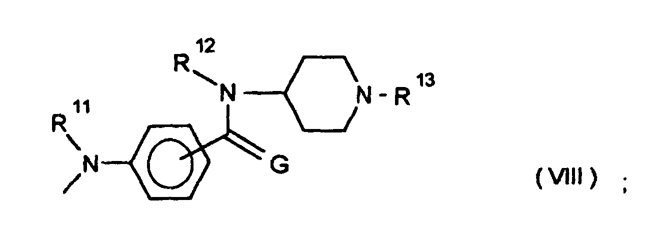
R⁷ ist gleich oder verschieden (C₁-C₄)-Alkyl, (C₆-C₁₂)-Aryl oder (C₁-C₃)-Alkyl-(C₆-C₁₂)-Aryl;
R⁸,R⁹ sind gleich oder verschieden und stehen für Wasserstoff, Halogen, (C₁-C₃)-Alkyl und (C₁-C₃)-Alkoxy;
A (C₁-C₃)-Alkandiyl;
Q O oder NR¹¹;
R¹⁰ ein Rest der Formel (IX) 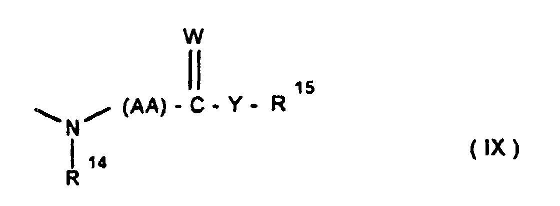
R¹¹,R¹⁴ unabhängig voneinander Wasserstoff oder (C₁-C₄)-Alkyl;
W O oder S;
G O oder zwei Wasserstoffatome;
R¹² steht bei G=O für Wasserstoff und bei G=zwei Wasserstoffatome für Wasserstoff oder R¹⁶CO;
R¹³ ist (C₁-C₄)-Alkyl, (C₃-C₇)-Cycloalkyl, -(CH₂)ₘ-(C₃-C₇)-Cycloalkyl, -(CH₂)ₘ-CON(R¹¹)₂, oder 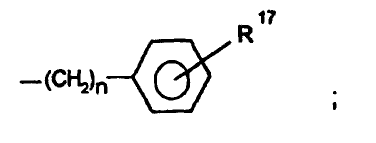
m, n sind gleich oder verschieden eine Zahl 0-6
AA steht für eine Aminosäure wie Methionin, Alanin, Phenylalanin, 2-Chlorphenylalanin, 3-Chlorphenylalanin, 4-Chlarphenylalanin, 2-Fluorphenylalanin, 3-Fluorphenylalanin, 4-Fluorphenylalanin, Tyrosin, o-Methyltyrosin, β-(2-Thienyl)-alanin, Glycin, Cyclohexylalanin, Leucin, Isoleucin, Valin, Norleucin, Phenylglycin, Serin, Cystein, Aminopropionsäure oder Aminobuttersäure;
Y
1. (C₂-C₅)-Alkendiyl,
2. (C₁-C₈)-Alkandiyl,
3. (C₃-C₁₀)-Cycloalkandiyl oder
4. -(CH)ₚ-Tₒ-(CH₂)_{q}-,
wobei 1. bis 4. gegebenenfalls mit einen oder mehreren gleichen oder verschiedenen Resten wie O-R¹⁸, NO₂, CN, CO₂R¹¹, SO₃R¹⁸, NR²⁰R²¹, SO₂NR²⁰R²¹, CONR²⁰R²¹ substituiert sein kann;
T O, NR²¹ oder S;
o ist eine Zahl 0 oder 1;
p,q sind gleich oder verschieden und bedeuten eine Zahl von 0 bis 6;
R¹⁵
1. Wasserstoff,
2. (C₁-C₅)-Alkyl,
3. (C₆-C₁₀)-Aryl oder
4. (C₁-C₉)-Heteroaryl,
wobei 3. und 4. gegebenenfalls mit einer oder mehreren gleichen oder verschiedenen Gruppen substituiert sein können, wie Halogen, CN, NO₂, (C₁-C₆)-Alkyl, (C₆-C₁₀)-Aryl, (C₂-C₅)-Alkenyl, (C₁-C₅)-Alkoxy, wobei die letzten vier Reste gegebenenfalls teilweise oder vollständig mit Halogen substituiert sein können, (C₁-C₅)-Alkylthio, NR²⁰R²¹, CO₂R¹⁹, SO₃R¹⁸, SO₂NR²⁰R²¹, SO₂R¹⁸, O-R¹⁸; NR²⁰CO-R¹⁵;
R¹⁶ ist Wasserstoff, (C₁-C₄)-Alkyl, (C₆-C₁₂)-Aryl, (C₁-C₄)-Alkyl-(C₆-C₁₂)-Aryl oder Perfluoro-(C₁-C₄)-Alkyl;
R¹⁷ ist Wasserstoff, Halogen, (C₁-C₄)-Alkyl, (C₁-C₄)-Alkoxy, Perfluoro-(C₁-C₄)-Alkyl, NO₂, OH, NH₂, CON(R¹⁶)₂ oder NR¹⁶CON(R¹⁶)₂;
R¹⁸,R¹⁹,R²⁰ gleich oder verschieden sind und Wasserstoff, (C₁-C₅)-Alkyl, (C₃-C₅)-Alkenyl, (C₆-C₁₂)-Aryl-(C₁-C₃)-Alkyl, (C₃-C₁₀)-Cycloalkyl, (C₃-C₁₀)-Cycloalkyl-(C₁-C₃)-Alkyl, C(O)-O-(C₁-C₅)-Alkyl oder C(O)-NH-(C₁-C₅)-Alkyl bedeuten;
R²¹ unabhängig voneinanderWasserstoff, C(O)-O-(C₁-C₅)-Alkyl oder C(O)-O-(C₁-C₃)-Alkyl-(C₆-C₁₀)-Aryl;
Z -N(R¹⁴)(R²²);
R²² steht für 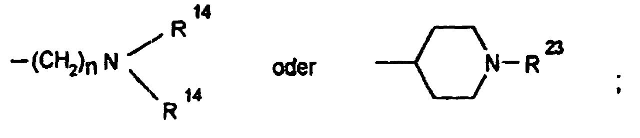
R²³ (C₁-C₄)-Alkyl, 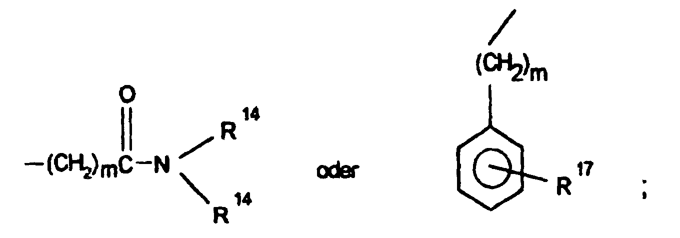
sowie deren physiologisch verträglichen Salze zur Herstellung eines Arzneimittels zur Behandlung und Prävention der Alzheimerschen Krankheit.

2. Verwendung nach Anspruch 1 eines nicht-peptidischen Bradykinin-Antagonisten der Formel (I), in welcher die Symbole folgende Bedeutung haben:
D
1. einen Rest der Formel (Xa) 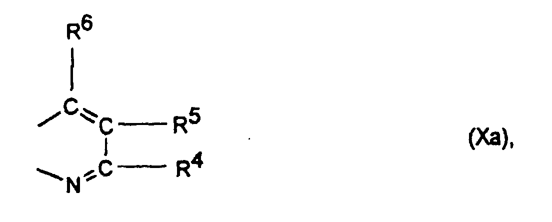
2. einen Rest der Formel (Xb) 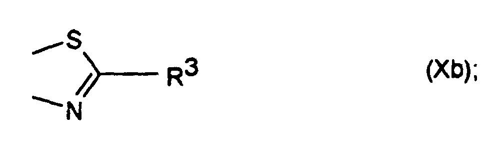
E
1. einen Rest der Formel (XI) 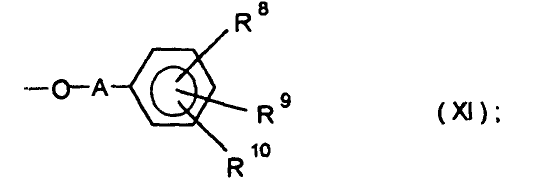
2. Wasserstoff, (C₁-C₄)-Alkyl und (C₁-C₄)-Alkoxy;
R¹,R² sind gleich oder verschieden und stehen für Wasserstoff, Halogen und (C₁-C₄)-Alkyl;
R⁴ ist Wasserstoff, (C₁-C₄)-Alkyl, Phenyl oder Methoxy;
R⁵
1. Wasserstoff, (C₁-C₄)-Alkyl oder
2. 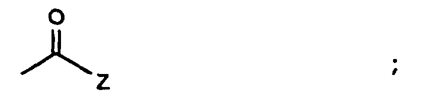
R⁶
1. Wasserstoff, (C₁-C₄)-Alkyl oder
2. ein Rest der Formel (VIII): 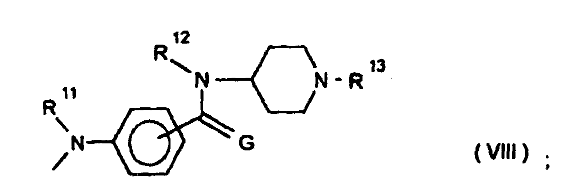
R⁸,R⁹ sind gleich oder verschieden und stehen für Wasserstoff, Halogen, (C₁-C₃)-Alkyl und (C₁-C₃)-Alkoxy;
A ist -CH₂- oder -CH₂-CH₂-;
R¹⁰ steht für einen Rest der Formel (IX): 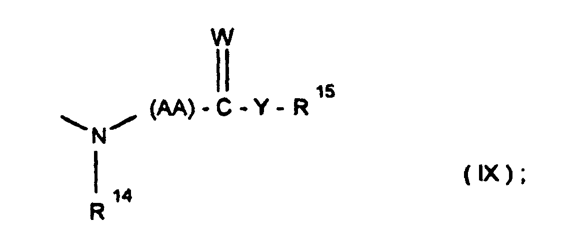
R¹¹,R¹⁴ sind gleich oder verschieden Wasserstoff, Methyl oder Ethyl;
W steht für O oder S;
G steht für O oder zwei Wasserstoffatome;
R¹² steht bei G gleich O für Wasserstoff oder bei G gleich zwei Wasserstoffatome für Wasserstoff oder R¹⁶CO;
R¹³ ist (C₁-C₄)-Alkyl, Cyclopentyl, Cyclohexyl, -(CH₂)ₘCON(R¹¹)₂, 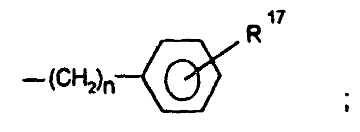
m,n sind gleich oder verschieden eine Zahl 0-2;
AA steht für die Aminocarbonsäure Glycin oder Alanin;
Y
1. (C₂-C₅)-Alkendiyl,
2. (C₂-C₄)-Alkandiyl,
3. (C₃-C₆)-Cycloalkandiyl oder
4. -(CH₂)ₚ-Tₒ-(CH₂)_{q}- ;
T steht für 0 oder S;
o ist eine Zahl 0 oder 1;
p,q sind gleich oder verschieden und bedeuten eine Zahl von 0-2;
R¹⁵
1. Wasserstoff,
2. (C₁-C₅)-Alkyl,
3. Phenyl oder
4. (C₅-C₉)-Heteroaryl,
wobei 3. und 4. gegebenenfalls mit einer, zwei oder drei gleichen oder verschiedenen Gruppen substituiert sein können, wie Halogen, NO₂, (C₁-C₃)-Alkyl, (C₁-C₃)-Alkoxy, in denen gegebenenfalls die H-Atome des Alkylrestes jedes vorgenannten Substituenten teilweise oder vollständig durch Halogen ersetzt sind, (C₁-C₃)-Alkylthio, NR²⁰R²¹, NR²⁰CO-(C₁-C₅)-Alkyl und NR²⁰CO-Pyridyl
R¹⁶ ist Wasserstoff, (C₁-C₄)-Alkyl oder Phenyl;
R¹⁷ ist Wasserstoff, Halogen, (C₁-C₄)-Alkyl, NO₂ oder NH₂;
R²⁰ ist Wasserstoff, (C₁-C₄)-Alkyl oder Benzyl;
R²¹ ist Wasserstoff oder C(O)-O-(C₁-C₅)-Alkyl;
Z ist -N(R¹⁴)(R²²)
R²² 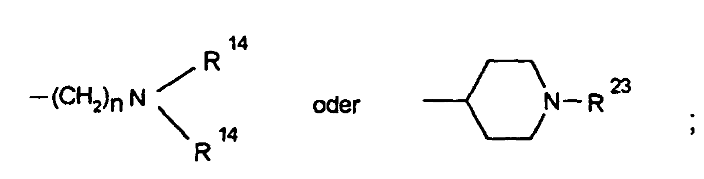
R²³ (C₁-C₄)-Alkyl, 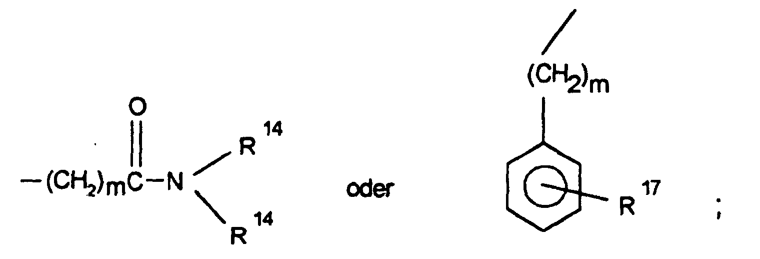
sowie deren physiologisch verträglichen Salze.

3. Verwendung nach Anspruch 2 eines nicht-peptidischen Bradykinin-Antagonisten der Formel (I) in welcher die Symbole folgende Bedeutung haben:
D
1. ein Rest der Formel (Xa) oder 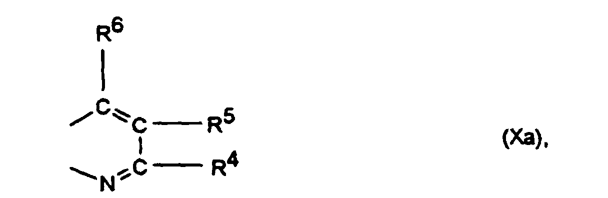
2. ein Rest der Formel (Xb) 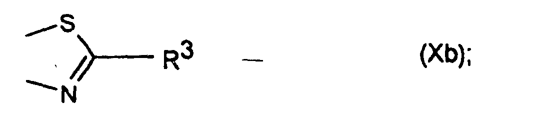
E 1. ein Rest der Formel (XI) 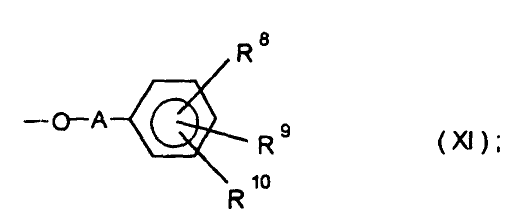
R¹,R² sind gleich oder verschieden und stehen für Wasserstoff, Halogen und (C₁-C₄)-Alkyl;
R⁴ Wasserstoff oder (C₁-C₄)-Alkyl;
R⁵ Wasserstoff;
R⁶ Wasserstoff;
R⁸,R⁹ sind gleich oder verschieden und stehen für Wasserstoff, Chlor, Methyl und Methoxy;
A -CH₂- oder -CH₂-CH₂- ;
R¹⁰ ein Rest der Formel (IX): 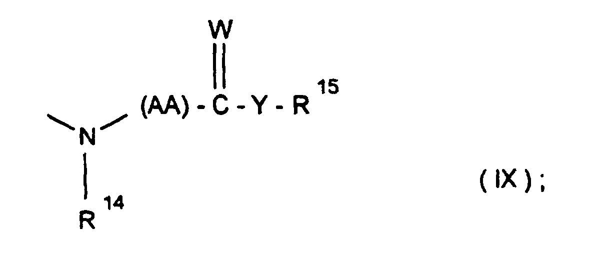
R¹⁴ Wasserstoff, Methyl oder Ethyl;
W ist O;
AA steht für die Aminocarbonsäure Glycin;
Y
1. (C₂-C₅)-Alkendiyl,
2. (C₂-C₄)-Alkandiyl,
3. (C₃-C₆)-Cycloalkandiyl oder
4. -(CH₂)ₚ-Tₒ-(CH₂)_{q}- ;
T steht für 0 oder S;
o ist eine Zahl 0 oder 1;
p,q sind gleich oder verschieden und bedeuten eine Zahl von 0-2;
R¹⁵
1. Wasserstoff
2. (C₁-C₃)-Alkyl,
3. Phenyl oder
4. (C₅-C₉)-Heteroaryl,
wobei 3. und 4. gegebenenfalls mit einer, zwei oder drei gleichen oder verschiedenen Gruppen substituiert sein können, wie Halogen, NO₂, (C₁-C₃)-Alkyl, (C₁-C₃)-Alkoxy in denen gegebenenfalls die H-Atome des Alkylrestes jedes vorgenannten Substituenten teilweise oder vollständig durch Halogen ersetzt sind, NR²⁰R²¹, NR²⁰CO-(C₁-C₃)-Alkyl und NR²⁰CO-Pyridyl;
R²⁰ ist Wasserstoff, (C₁-C₄)-Alkyl oder Benzyl;
R²¹ ist Wasserstoff oder C(O)-O-(C₁-C₅)-Alkyl;
sowie deren physiologisch verträglichen Salze.

## Claims

1. The use of a nonpeptide bradykinin antagonist of the formula (I) 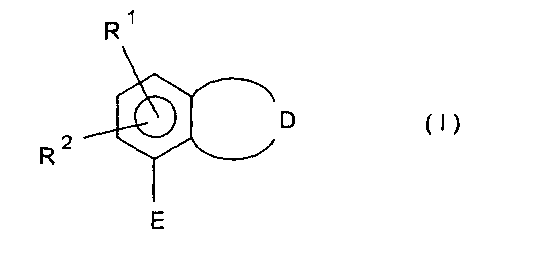 in which the symbols have the following meanings:
D is
1. a radical of the formula (II) 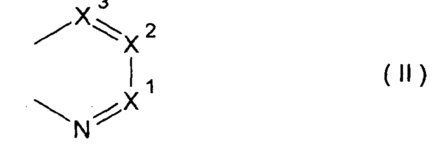 or
2. a radical of the formulae (III) to (VI) 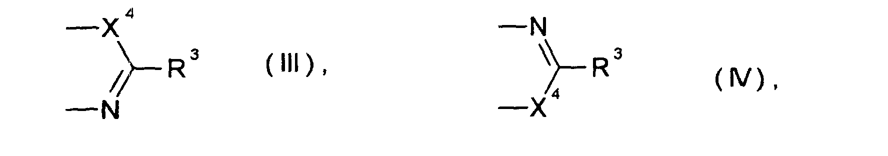 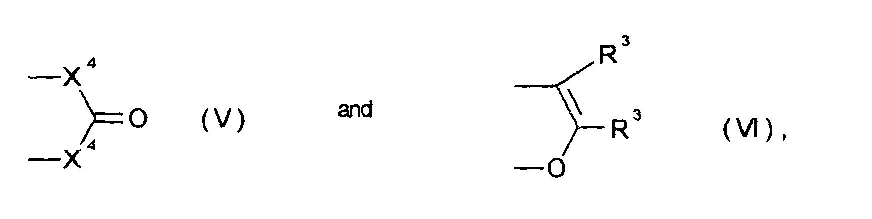
E is
1. a radical of the formula (VII) 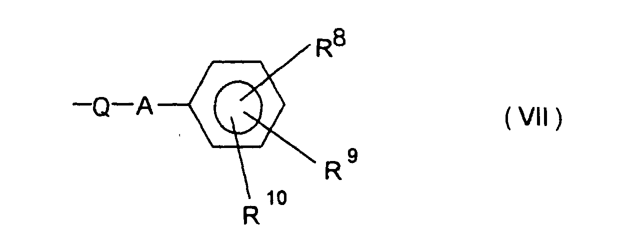 or
2. hydrogen, halogen, (C₁-C₄)-alkyl, (C₁-C₄)-alkylthio or (C₁-C₄)-alkoxy, where in the last 3 radicals 1 or more hydrogen atoms can be replaced by fluorine;
X¹ is nitrogen or C-R⁴;
X² is nitrogen or C-R⁵ ;
X³ is nitrogen or C-R⁶ ;
X⁴ is independently at each occurrence oxygen, sulfur, nitrogen or N-R⁷ ;
R¹, R² are identical or different and represent hydrogen, halogen, (C₁-C₄)-alkyl and (C₁-C₄)-alkoxy;
R³ is independently at each occurrence hydrogen, halogen, (C₁-C₆)-alkyl, (C₆-C₁₂)-aryl, (C₁-C₃)-alkyl-(C₆-C₁₂)-aryl, (C₃-C₅)-alkenyl, (C₁-C₄)-alkoxy or CO₂R¹¹;
R⁴ is hydrogen, halogen, (C₁-C₄)-alkyl, hydroxyl, (C₁-C₄)-alkylthio, amino, (C₁-C₄)-alkylamino, (C₁-C₄)-dialkylamino, (C₁-C₄)-alkoxy, where the alkyl radical of each of the abovementioned substituents is optionally substituted by hydroxyl, (C₁-C₄)-alkoxy, amino and (C₁-C₄)-alkylamino, or (C₆-C₁₂)-aryl, optionally substituted by (C₁-C₄)-alkyl or CO₂R¹¹;
R⁵ is
1. hydrogen, (C₁-C₄)-alkyl or
2. 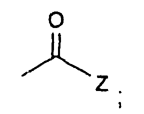
R⁶ is
1. hydrogen, halogen, (C₁-C₄)-alkyl, hydroxyl, (C₁-C₄)-alkylthio, amino, (C₁-C₄)-alkylamino, (C₁-C₄)-dialkylamino, (C₁-C₄)-alkoxy, where the alkyl radical of each of the abovementioned substituents is optionally substituted by hydroxyl, (C₁-C₄)-alkoxy, amino and (C₁-C₄)-alkylamino, or (C₆-C₁₂)-aryl, optionally substituted by (C₁-C₄)-alkyl or CO₂R¹¹; or
2. a radical of the formula (VIII) 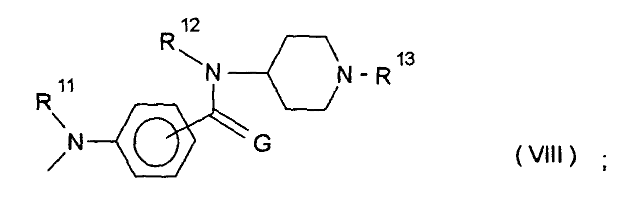
R⁷ is identically or differently at each occurrence (C₁-C₄)-alkyl, (C₆-C₁₂)-aryl or (C₁-C₃)-alkyl-(C₆-C₁₂)-aryl;
R⁸, R⁹ are identical or different and are hydrogen, halogen, (C₁-C₃)-alkyl or (C₁-C₃)-alkoxy;
A is (C₁-C₃)-alkanediyl;
Q is O or NR¹¹;
R¹⁰ is a radical of the formula (IX)
R¹¹, R¹⁴ are independently hydrogen or (C₁-C₄)-alkyl; 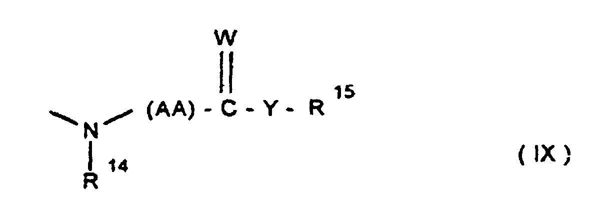
W is O or S;
G is O or two hydrogen atoms_{;}
R¹² is, if G is O, hydrogen and, if G is two hydrogen atoms, hydrogen or R¹⁶CO;
R¹³ is (C₁-C₄)-alkyl, (C₃-C₇)-cycloalkyl, -(CH₂)ₘ-(C₃-C₇)-cycloalkyl, -(CH₂)ₘ-CON(R¹¹)₂, or 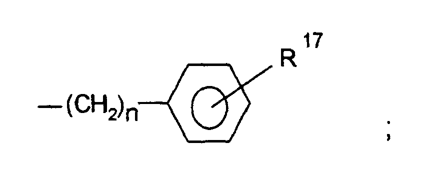
m, n are, identically or differently, a number 0-6
AA is an amino acid such as methionine, alanine, phenylalanine, 2-chlorophenylalanine, 3-chlorophenylalanine, 4-chlorophenyl-alanine, 2-fluorophenylalanine, 3-fluorophenylalanine, 4-fluorophenylalanine, tyrosine, o-methyltyrosine, β-(2-thienyl)-alanine, glycine, cyclohexylalanine, leucine, isoleucine, valine, norleucine, phenylglycine, serine, cysteine, aminopropionic acid or aminobutyric acid;
Y is
1. (C₂-C₅)-alkenediyl,
2. (C₁-C₈)-alkanediyl,
3. (C₃-C₁₀)-cycloalkanediyl or
4. -(CH)ₚ-Tₒ-(CH₂)_{q}-,
where 1. to 4. can optionally be substituted by one or more identical or different radicals such as O-R¹⁸, NO₂, CN, CO₂R¹¹, SO₃R¹⁸, NR²⁰R²¹, SO₂NR²⁰R²¹, CONR²⁰R²¹;
T is O, NR²¹ or S;
o is a number 0 or 1;
p, q are identical or different and denote a number from 0 to 6;
R¹⁵ is
1. hydrogen,
2. (C₁-C₅)-alkyl,
3. (C₆-C₁₀)-aryl or
4. (C₁-C₉)-heteroaryl,
where 3. and 4. can optionally be substituted by one or more identical or different groups, such as halogen, CN, NO₂, (C₁-C₆)-alkyl, (C₆-C₁₀)-aryl, (C₂-C₅)-alkenyl, (C₁-C₅)-alkoxy, where the last four radicals can optionally be partly or completely substituted by halogen, (C₁-C₅)-alkylthio, NR²⁰R²¹, CO₂R¹⁹, SO₃R¹⁸, SO₂NR²⁰R²¹, SO₂R¹⁸, O-R¹⁸, NR²⁰CO-R¹⁵;
R¹⁶ is hydrogen, (C₁-C₄)-alkyl, (C₆-C₁₂)-aryl, (C₁-C₄)-alkyl-(C₆-C₁₂)-aryl or perfluoro-(C₁-C₄)-alkyl;
R¹⁷ is hydrogen, halogen, (C₁-C₄)-alkyl, (C₁-C₄)-alkoxy, perfluoro-(C₁-C₄)-alkyl, NO₂, OH, NH₂, CON(R¹⁶)₂ or NR¹⁶CON(R¹⁶)₂;
R¹⁸,R¹⁹,R²⁰ are identical or different and are hydrogen, (C₁-C₅)-alkyl, (C₃-C₅)-alkenyl, (C₆-C₁₂)-aryl-(C₁-C₃)-alkyl, (C₃-C₁₀)-cycloalkyl, (C₃-C₁₀)-cycloalkyl-(C₁-C₃)-alkyl, C(O)-O-(C₁-C₅)-alkyl or C(O)-NH-(C₁-C₅)-alkyl;
R²¹ is independently at each occurrence hydrogen, C(O)-O-(C₁-C₅)-alkyl or C(O)-O-(C₁-C₃)-alkyl-(C₆-C₁₀)-aryl;
Z is -N(R¹⁴)(R²²);
R²² is 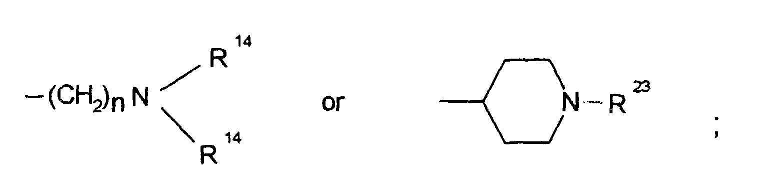
R²³ is (C₁-C₄)-alkyl, 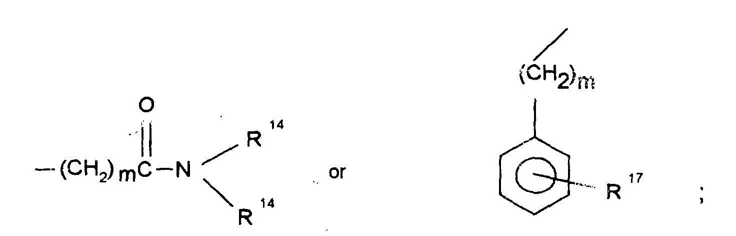
or their physiologically tolerable salts for the production of a pharmaceutical for the treatment and prevention of Alzheimer's disease.

2. The use as claimed in claim 1 of a nonpeptide bradykinin antagonist of the formula (I), in which the symbols have the following meaning:
D is
1. a radical of the formula (Xa) 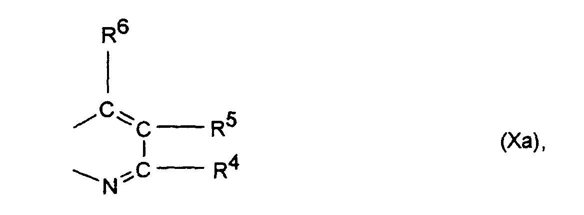
2. a radical of the formula (Xb) 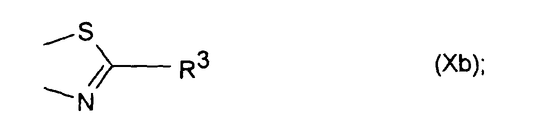
E is
1. a radical of the formula (XI) 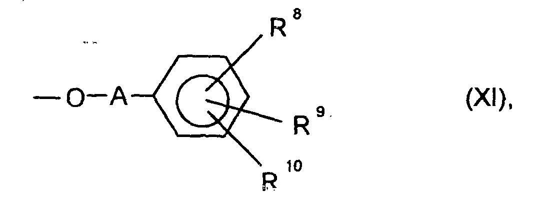
2. hydrogen, (C₁-C₄)-alkyl or (C₁-C₄)-alkoxy;
R¹,R² are identical or different and represent hydrogen, halogen and (C₁-C₄)-alkyl;
R⁴ is hydrogen, (C₁-C₄)-alkyl, phenyl or methoxy;
R⁵ is
1. hydrogen, (C₁-C₄)-alkyl or
2. 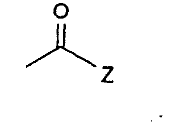
R⁶ is
1. hydrogen, (C₁-C₄)-alkyl or
2. a radical of the formula (VIII) 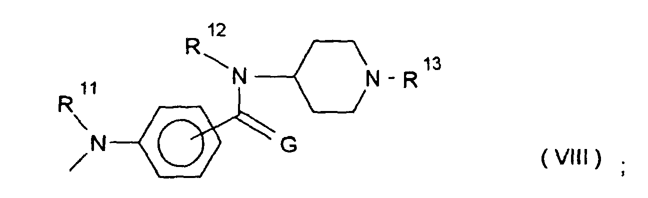
R⁸,R⁹ are identical or different and are hydrogen, halogen, (C₁-C₃)-alkyl or (C₁-C₃)-alkoxy;
A is -CH₂- or -CH₂-CH₂-;
R¹⁰ is a radical of the formula (IX) 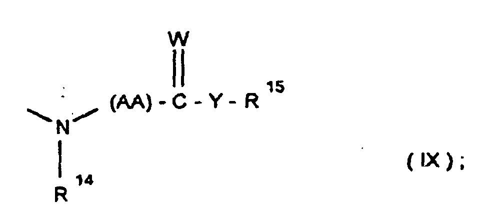
R¹¹, R¹⁴ are identically or differently, hydrogen, methyl or ethyl;
W is O or S;
G is O or two hydrogen atoms;
R¹² is, if G is O, hydrogen or, if G is two hydrogen atoms, hydrogen or R¹⁶CO;
R¹³ is (C₁-C₄)-alkyl, cyclopentyl, cyclohexyl, -(CH₂)ₘCON(R¹¹)₂, 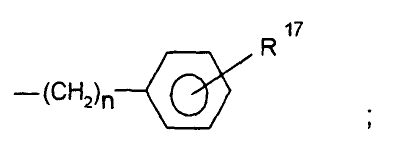
m,n are, identically or differently, a number 0-2;
AA is the aminocarboxylic acid glycine or alanine;
Y is
1. (C₂-C₅)-alkenediyl,
2. (C₂-C₄)-alkanediyl,
3. (C₃-C₆)-cycloalkanediyl or
4. -(CH₂)ₚ-Tₒ-(CH₂)_{q}- ;
T is O or S;
o is a number 0 or 1;
p, qare identical or different and are a number from 0-2;
R¹⁵ is
1. hydrogen,
2. (C₁-C₅)-alkyl,
3. phenyl or
4. (C₅-C₉)-heteroaryl,
where 3. and 4. can optionally be substituted by one, two or three identical or different groups, such as halogen, NO₂, (C₁-C₃)-alkyl, (C₁-C₃)-alkoxy, in which, if appropriate, the hydrogen atoms of the alkyl radical of each of the abovementioned substituents can be partly or completely replaced by halogen, (C₁-C₃)-alkylthio, NR²⁰R²¹, NR²⁰CO-(C₁-C₅)-alkyl and NR²⁰CO-pyridyl
R¹⁶ is hydrogen,(C₁-C₄)-alkyl or phenyl;
R¹⁷ is hydrogen, halogen, (C₁-C₄)-alkyl, NO₂ or NH₂;
R²⁰ is hydrogen, (C₁-C₄)-alkyl or benzyl;
R²¹ is hydrogen or C(O)-O-(C₁-C₅)-alkyl;
Z is -N(R¹⁴)(R²²)
R²² is 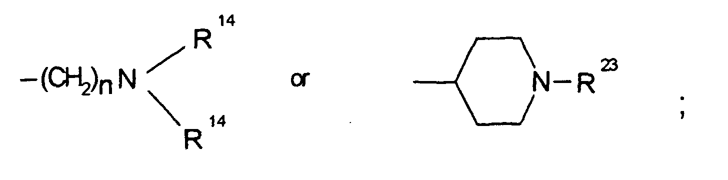
R²³ is (C₁-C₄)alkyl, 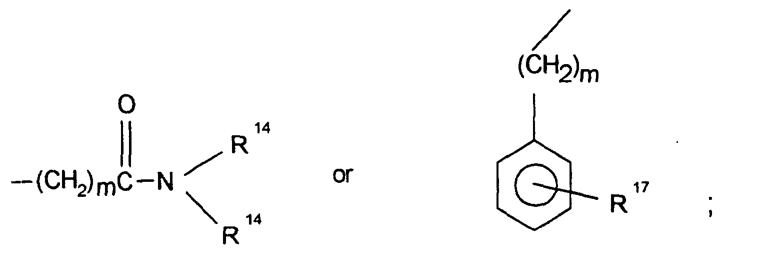
or their physiologically tolerable salts.

3. The use as claimed in claim 2 of a nonpeptide bradykinin antagonist of the formula (I), in which the symbols have the following meanings:
D is
1. a radical of the formula (Xa) 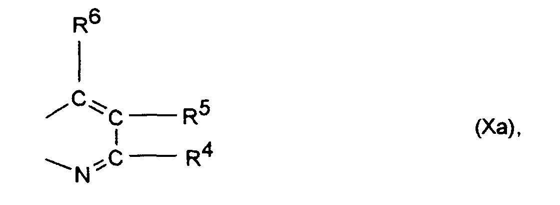
2. a radical of the formula (Xb) 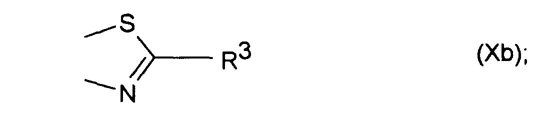
E is 1. a radical of the formula (XI) 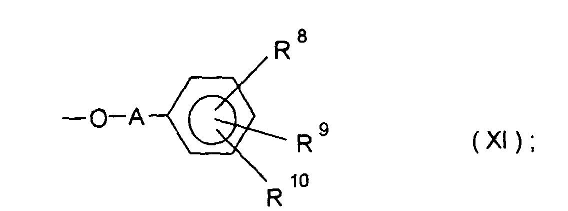
R¹, R² are identical or different and represent hydrogen, halogen and (C₁-C₄)-alkyl;
R⁴ is hydrogen or (C₁-C₄)-alkyl;
R⁵ is hydrogen;
R⁶ is hydrogen;
R⁸, R⁹ are identical or different and are hydrogen, chlorine, methyl or methoxy;
A is -CH₂- or -CH₂-CH₂-;
R¹⁰ is a radical of the formula (IX) 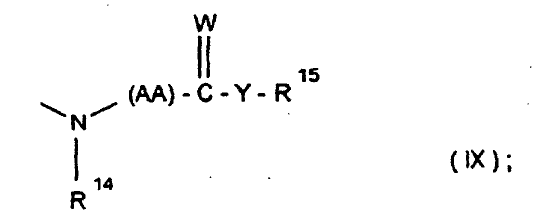
R¹⁴ is hydrogen, methyl or ethyl;
W is O;
AA is the aminocarboxylic acid glycine;
Y is
1. (C₂-C₅)-alkenediyl,
2. (C₂-C₄)-alkanediyl,
3. (C₃-C₆)-cycloalkanediyl or
4. 4. -(CH₂)ₚ-Tₒ-(CH₂)_{q}- ;
T is O or S;
o is a number 0 or 1;
p, qare identical or different and are a number from 0-2;
R¹⁵ is
1. hydrogen
2. (C₁-C₃)-alkyl,
3. phenyl or
4. (C₅-C₉)-heteroaryl,
where 3. and 4. can optionally be substituted by one, two or three identical or different groups, such as halogen, NO₂, (C₁-C₃)-alkyl, (C₁-C₃)-alkoxy, in which, if appropriate, the hydrogen atoms of the alkyl radical of each of the abovementioned substituents are partly or completely replaced by halogen NR²⁰R²¹, NR²⁰CO-(C₁-C₃)-alkyl and NR²⁰CO-pyridyl;
R²⁰ is hydrogen, (C₁-C₄)-alkyl or benzyl;
R²¹ is hydrogen or C(O)-O-(C₁-C₅)-alkyl;
or their physiologically tolerable salts.

## Revendications

1. Utilisation d'un antagoniste non peptidique de bradykinine, de formule (I) 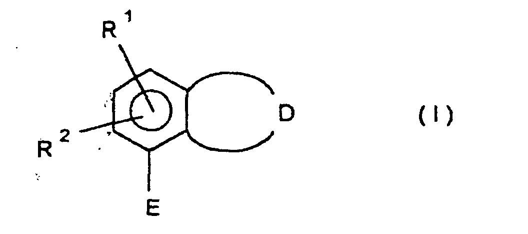 dans laquelle les symboles ont les significations suivantes :
D représente
1. un reste de formule (II) ou 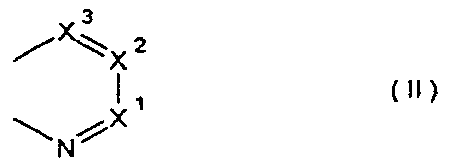
2. un reste de formules (III) à (VI) : 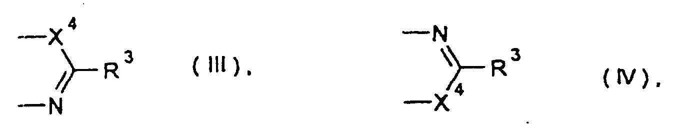 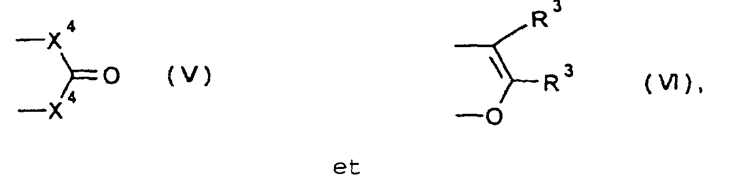
E représente
1. un reste de formule (VII) ou 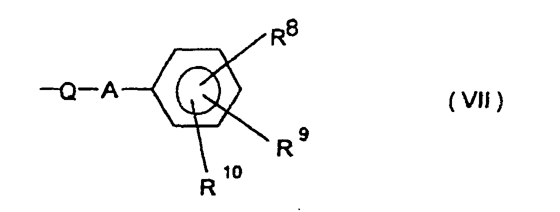
2. un atome d'hydrogène ou d'halogène ou un groupe alkyle en C₁-C₄, alkyl(C₁-C₄)thio ou alcoxy en C₁-C₄, dans les trois derniers restes un ou plusieurs atomes d'hydrogène pouvant être remplacés par le fluor ;
X¹ représente un atome d'azote ou C-R⁴ ;
X² représente un atome d'azote ou C-R⁵ ;
X³ représente un atome d'azote ou C-R⁶ ;
X⁴ représentent, indépendamment les uns des autres, un atome d'oxygène, de soufre ou d'azote ou N-R⁷ ;
R¹, R² sont identiques ou différents et représentent un atome d'hydrogène ou d'halogène ou un groupe alkyle en C₁-C₄ ou alcoxy en C₁-C₄ ;
R³ représentent, indépendamment les uns des autres, un atome d'hydrogène ou d'halogène ou un groupe alkyle en C₁-C₆, aryle en C₆-C₁₂, alkyl(C₁-C₃)aryle(C₆-C₁₂), alcényle en C₃-C₅, alcoxy en C₁-C₄ ou CO₂R¹¹ ;
R⁴ représente un atome d'hydrogène ou d'halogène ou un groupe alkyle en C₁-C₄, hydroxy, alkyl(C₁-C₄)thio, amino, alkyl(C₁-C₄)amino, dialkyl(C₁-C₄)amino, alcoxy en C₁-C₄, le radical alkyle de chaque substituant précité étant éventuellement substitué par un groupe hydroxy, alcoxy en C₁-C₄, amino ou alkyl-(C₁-C₄)amino, ou représente un groupe aryle en C₆-C₁₂ éventuellement substitué par un groupe alkyle en C₁-C₄ ou CO₂R¹¹ ;
R⁵ représente
1. un atome d'hydrogène, un groupe alkyle en C₁-C₄, ou
2. 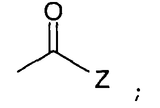
R⁶ représente
1. un atome d'hydrogène ou d'halogène ou un groupe alkyle en C₁-C₄, hydroxy, alkyl (C₁-C₄) thio, amino, alkyl(C₁-C₄)amino, dialkyl (C₁-C₄) amino, alcoxy en C₁-C₄, le radical alkyle de chaque substituant précité étant éventuellement substitué par un groupe hydroxy, alcoxy en C₁-C₄, amino ou alkyl(C₁-C₄)amino, ou représente un groupe aryle en C₆-C₁₂, éventuellement substitué par un groupe alkyle en C₁-C₄ ou CO₂R¹¹ ; ou
2. un reste de formule (VIII) : 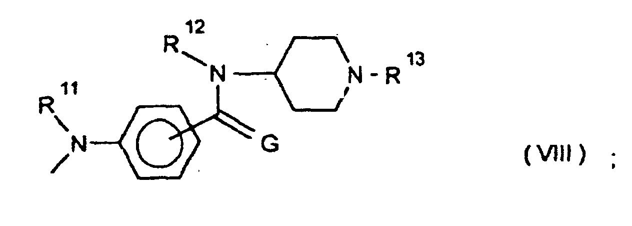
R⁷ sont identiques ou différents et représentent un groupe alkyle en C₁-C₄, aryle en C₆-C₁₂ ou alkyl(C₁-C₃)aryle(C₆-C₁₂) ;
R⁸, R⁹ sont identiques ou différents et représentent un atome d'hydrogène ou d'halogène ou un groupe alkyle en C₁-C₃ ou alcoxy en C₁-C₃ ;
A représente un groupe alcanediyle en C₁-C₃ ;
Q représente O ou NR¹¹ ;
R¹⁰ représente un reste de formule (IX) 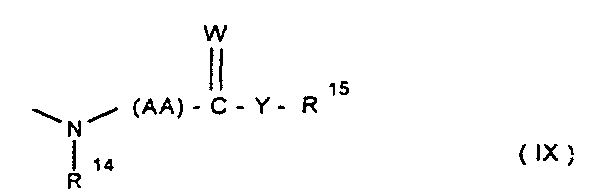
R¹¹, R¹⁴ représentent, indépendamment l'un de l'autre, un atome d'hydrogène ou un groupe alkyle en C₁-C₄ ;
W représente O ou S ;
G représente O ou deux atomes d'hydrogène ;
R¹² représente, lorsque G = O, un atome d'hydrogène et lorsque G = deux atomes d'hydrogène , un atome d'hydrogène ou R¹⁶-CO ;
R¹³ est un groupe alkyle en C₁-C₄, cycloalkyle en C₃-C₇, -(CH₂)ₘ-cycloalkyle(C₃-C₇), -(CH₂)ₘ-CON(R¹¹)₂ ou 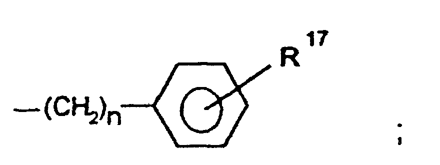
m, n sont identiques ou différents et représentent un nombre allant de 0 à 6 ;
AA représente un aminoacide tel que la méthionine, l'alanine, la phénylalanine, la 2-chlorophénylalanine, la 3-chlorophénylalanine, la 4-chlorophénylalanine, la 2-fluorophénylalanine, la 3-fluorophénylalanine, la 4-fluorophénylalanine, la tyrosine, l'o-méthyltyrosine, la β-(2-thiényl)alanine, la glycine, la cyclohexylalanine, la leucine, l'isoleucine, la valine, la norleucine, la phénylglycine, la sérine, la cystéine, l'acide aminopropionique ou l'acide aminobutyrique ;
Y représente
1. un groupe alcènediyle en C₂-C₅,
2. un groupe alcanediyle en C₁-C₈,
3. un groupe cycloalcanediyle en C₃-C₁₀ ou
4. -(CH)ₚ-Tₒ-(CH₂)_{q}-,
les groupes 1 à 4 pouvant éventuellement être substitués par un ou plusieurs radicaux, identiques ou différents, tels que O-R¹⁸, NO₂, CN, CO₂R¹¹, SO₃R¹⁸, NR²⁰R²¹, SO₂NR²⁰R²¹, CONR²⁰R²¹ ;
T représente O, NR²¹ ou S ;
O est le nombre 0 ou 1 ;
p, q sont identiques ou différents et représentent un nombre allant de 0 à 6 ;
R¹⁵ représente
1. un atome d'hydrogène,
2. un groupe alkyle en C₁-C₅,
3. un groupe aryle en C₆-C₁₀ ou
4. un groupe hétéroaryle en C₁-C₉,
les groupes 3 et 4 pouvant éventuellement porter un ou plusieurs substituants identiques ou différents, tels que des atomes d'halogène ou des radicaux CN, NO₂, alkyle en C₁-C₆, aryle en C₆-C₁₀, alcényle en C₂-C₅, alcoxy en C₁-C₅, les quatre derniers radicaux pouvant éventuellement être totalement ou partiellement substitués par des atomes d'halogène, des radicaux alkyl-(C₁-C₅)thio, NR²⁰R²¹, CO₂R¹⁹, SO₃R¹⁸, SO₂NR²⁰R²¹, SO₂R¹⁸, O-R¹⁸, NR²⁰CO-R¹⁵ ;
R¹⁶ est un atome d'hydrogène ou un groupe alkyle en C₁-C₄, aryle en C₆-C₁₂, alkyl (C₁-C₄)-aryle-(C₆-C₁₂) ou perfluoro-alkyle(C₁-C₄) ;
R¹⁷ est un atome d'hydrogène ou d'halogène ou un groupe alkyle en C₁-C₄, alcoxy en C₁-C₄, perfluoro-alkyle(C₁-C₄), NO₂, OH, NH₂, CON(R¹⁶)₂ ou NR¹⁶CON(R¹⁶)₂ ;
R¹⁸, R¹⁹, R²⁰ sont identiques ou différents et représentent un atome d'hydrogène ou un groupe alkyle en C₁-C₅, alcényle en C₃-C₅, aryl(C₆-C₁₂)-alkyle(C₁-C₃), cycloalkyle en C₃-C₁₀, cycloalkyl(C₃-C₁₀)-alkyle (C₁-C₃), C(O)-O-alkyle(C₁-C₅) ou C(O)-NH-alkyle(C₁-C₅) ;
R²¹ représentent, indépendamment les uns des autres, un atome d'hydrogène, un groupe C (O) -O-alkyle (C₁-C₅) ou C (O) -O-alkyl(C₁-C₃)-aryle(C₆-C₁₀) ;
Z représente un groupe -N(R¹⁴)(R²²) ;
R²² représente 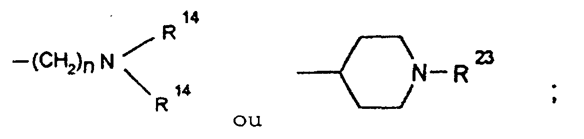
R²³ représente un groupe alkyle en C₁-C₄, 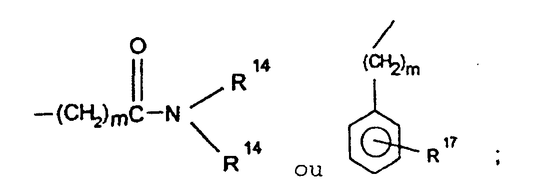
ainsi que leurs sels physiologiquement acceptables, pour la fabrication d'un médicament destinés au traitement et à la prévention de la maladie d'Alzheimer.

2. Utilisation selon la revendication 1 d'un antagoniste non peptidique de bradykinine de formule (I), dans laquelle les symboles ont les significations suivantes :
D représente
1. un reste de formule (Xa) ou 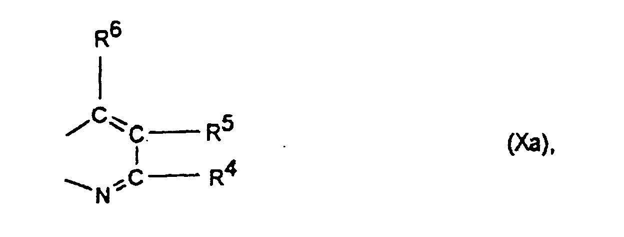
2. un reste de formule (Xb) 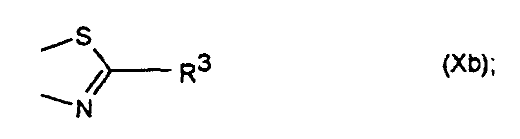
E représente
1. un reste de formule (XI) 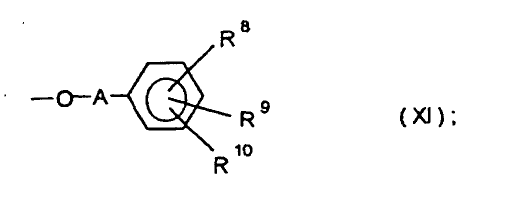
2. un atome d'hydrogène ou un groupe alkyle en C₁-C₄ ou alcoxy en C₁-C₄ ;
R¹, R² sont identiques ou différents et représentent un atome d'hydrogène ou d'halogène, ou un groupe alkyle en C₁-C₄ ;
R⁴ est un atome d'hydrogène ou un groupe alkyle en C₁-C₄, phényle ou méthoxy ;
R⁵ représente
1. un atome d'hydrogène, un groupe alkyle en C₁-C₄ ou
2. 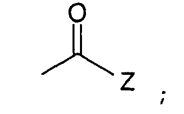
R⁶ représente
1. un atome d'hydrogène, un groupe alkyle en C₁-C₄ ou
2. un reste de formule (VIII) : 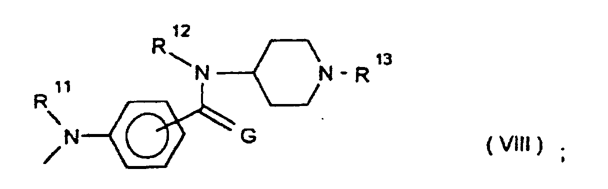
R⁸, R⁹ sont identiques ou différents et représentent un atome d'hydrogène ou d'halogène ou un groupe alkyle en C₁-C₃ ou alcoxy en C₁-C₃ ;
A est -CH₂- ou -CH₂-CH₂- ;
R¹⁰ représente un reste de formule (IX) : 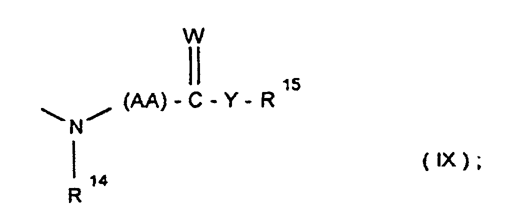
R¹¹, R¹⁴ sont identiques ou différents et représentent un atome d'hydrogène, le groupe méthyle ou éthyle ;
W représente O ou S ;
G représente O ou deux atomes d'hydrogène;
R¹² représente, lorsque G est O, un atome d'hydrogène ou, lorsque G est deux atomes d'hydrogène, un atome d'hydrogène ou R¹⁶CO ;
R¹³ est un groupe alkyle en C₁-C₄, cyclopentyle, cyclohexyle, -(CH₂)ₘCON(R¹¹)₂, 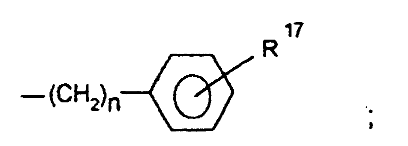
m, n sont identiques ou différents et représentent un nombre allant de 0 à 2 ;
AA représente l'acide aminocarboxylique glycine ou alanine ;
Y représente
1. un groupe alcènediyle en C₂-C₅,
2. un groupe alcanediyle en C₂-C₄,
3. un groupe cycloalcanediyle en C₃-C₆ ou
4. -(CH₂)ₚ-Tₒ-(CH₂)_{q}- ;
T représente O ou S ;
o représente le nombre 0 ou 1 ;
p, q sont identiques ou différents et représentent un nombre allant de 0 à 2 ;
R¹⁵ représente
1. un atome d'hydrogène,
2. un groupe alkyle en C₁-C₅,
3. le groupe phényle ou
4. un groupe hétéroaryle en C₅-C₉,
les groupes 3 et 4 pouvant éventuellement porter un, deux ou trois substituants identiques ou différents, tels que des atomes d'halogène, des groupes NO₂, alkyle en C₁-C₃, alcoxy en C₁-C₃, dans lesquels les atomes d'hydrogène du radical alkyle de chaque substituant précité sont éventuellement remplacés partiellement ou totalement par des atomes d'halogène, des groupes alkyl (C₁-C₃)thio, NR²⁰R²¹, NR²⁰CO-alkyle(C₁-C₅) et NR²⁰CO-pyridyle ;
R¹⁶ est un atome d'hydrogène ou un groupe alkyle en C₁-C₄ ou phényle ;
R¹⁷ est un atome d'hydrogène ou d'halogène ou un groupe alkyle en C₁-C₄, NO₂ ou NH₂ ;
R²⁰ est un atome d'hydrogène ou un groupe alkyle en C₁-C₄ ou benzyle ;
R²¹ est un atome d'hydrogène ou un groupe C(O)-O-alkyle(C₁-C₅) ;
Z est -N(R¹⁴)(R²²) ;
R²² est 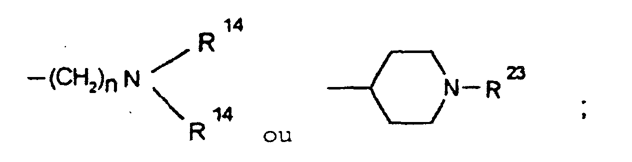
R²³ représente un groupe alkyle en C₁-C₄ 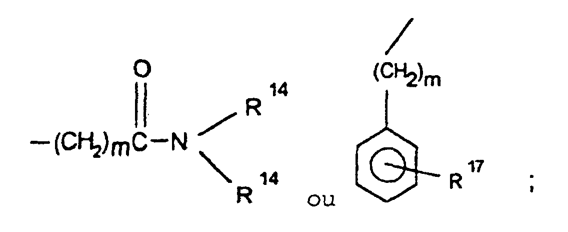
ainsi que de leurs sels physiologiquement acceptables.

3. Utilisation selon la revendication 2 d'un antagoniste non peptidique de bradykinine, de formule (I), dans laquelle les symboles ont les significations suivantes :
D représente
1. un reste de formule (Xa) ou 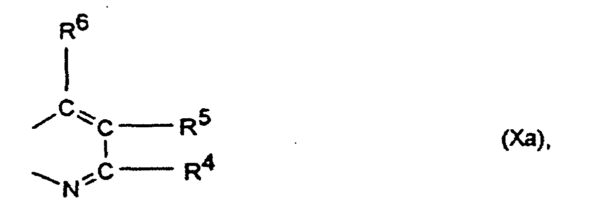
2. un reste de formule (Xb) 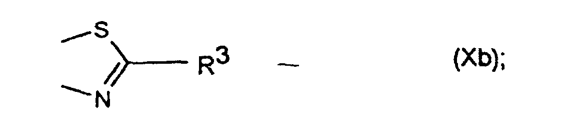
E représente 1. un reste de formule (XI) 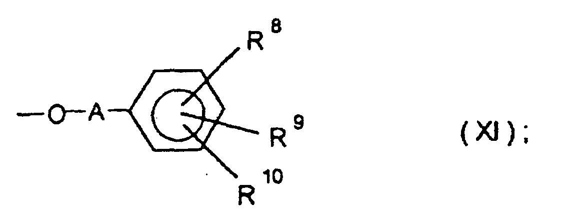
R¹, R² sont identiques ou différents et représentent un atome d'hydrogène ou d'halogène, ou un groupe alkyle en C₁-C₄ ;
R⁴ est un atome d'hydrogène ou un groupe alkyle en C₁-C₄ ;
R⁵ représente un atome d'hydrogène ;
R⁶ représente un atome d'hydrogène ;
R⁸, R⁹ sont identiques ou différents et représentent un atome d'hydrogène ou de chlore ou le groupe méthyle ou méthoxy ;
A représente -CH₂- ou -CH₂-CH₂- ;
R¹⁰ représente un reste de formule (IX) : 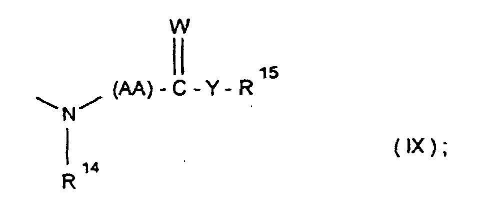
R¹⁴ représente un atome d'hydrogène ou le groupe méthyleou éthyle ;
W est O ;
AA représente l'acide aminocarboxylique glycine ;
Y représente
1. un groupe alcènediyle en C₂-C₅,
2. un groupe alcanediyle en C₂-C₄,
3. un groupe cycloalcanediyle en C₃-C₆ ou
4. -(CH₂)ₚ-Tₒ-(CH₂)_{q}- ;
T représente O ou S ;
o est le nombre 0 ou 1 ;
p, q sont identiques ou différents et représentent un nombre allant de 0 à 2 ;
R¹⁵ représente
1. un atome d'hydrogène,
2. un groupe alkyle en C₁-C₃,
3. le groupe phényle ou
4. un groupe hétéroaryle en C₅-C₉,
les groupes 3 et 4 pouvant éventuellement porter un, deux ou trois substituants identiques ou différents, tels que des atomes d'halogène et des groupes NO₂, alkyle en C₁-C₃, alcoxy en C₁-C₃, dans lesquels les atomes d'hydrogène du radical alkyle de chaque substituant précité sont éventuellement remplacés partiellement ou totalement par des atomes d'halogène, ou des groupes NR²⁰R²¹, NR²⁰CO-alkyle(C₁-C₃) et NR²⁰CO-pyridyle ;
R²⁰ est un atome d'hydrogène ou un groupe alkyle en C₁-C₄ ou benzyle ;
R²¹ est un atome d'hydrogène ou un groupe C(O)-O-alkyle(C₁-C₅) ;
ainsi que de leurs sels physiologiquement acceptables.
